# EUROPEAN PATENT APPLICATION

(11) **EP 2 143 794 A1**
(43) Date of publication of application: **13.01.2010**
(21) Application number: 08752166.2
(22) Date of filing: 25.04.2008
(51) Int. Cl.: C12N 15/09, A61K 35/76, A61K 38/00, A61K 48/00, A61P 35/00

(54) **VIRAL VECTOR FOR GENE THERAPY**

(30) Priority: 27.04.2007 JP 2007119130
(71) Applicant: Kyushu University, National University Corporation, Higashi-ku Fukuoka-shi Fukuoka 812-8581 (JP); Dnavec Corporation, Tsukuba-shi, Ibaraki 305-0856 (JP)
(72) Inventor: TANI, Kenzaburo, Fukuoka-shi Fukuoka 812-8581 (JP); INOUE, Hiroyuki, Fukuoka-shi Fukuoka 812-8581 (JP); INOUE, Makoto, Tsukuba-shi Ibaraki 305-0856 (JP); KINOH, Hiroaki, Chiba 260-0854 (JP); HASEGAWA, Mamoru, Tsukuba-shi Ibaraki 305-0856 (JP)
(74) Representative: Vossius & Partner
(86) International application number: PCT/JP2008/058132
(87) International publication number: WO 2008/136438

(57) **Abstract**

An objective of the present invention is to provide safe viral vectors for gene therapy that can be introduced by a simple technique and sufficiently express genes of interest *in vivo.* The present inventors demonstrated that anti-tumor effect can be produced when a heparin-binding cytokine such as granulocyte macrophage colony stimulating factor (GM-CSF) and a chemokine such as TARC or RANTES are expressed *in vivo* using a viral vector based on a negative-strand RNA virus. The present inventors also demonstrated that the protective effect of the vector is superior to that of conventional adenovirus vectors. Thus, the present invention relates to negative-strand RNA viral vectors comprising a cytokine gene and a chemokine gene. The viral vectors are suitable for treatment of cancers, in particular, metastatic cancers. The present invention also provides compositions comprising such viral vectors, and gene therapy methods using them.

## Description

### Technical Field

The present invention relates to viral vectors for gene therapy. More specifically, the present invention relates to viral vectors based on negative-strand RNA viruses, which comprise a gene encoding a cytokine such as granulocyte macrophage colony stimulating factor (GM-CSF), and are appropriate for treatment of cancers, in particular, metastatic cancers. The present invention also relates to compositions comprising such viral vectors, and gene therapy using them.

### Background Art

Currently available therapeutic methods for cancer include surgical therapy, radiotherapy, chemotherapy, and methods using interferon α, interleukin (for example, IL-2), or such. However, when treated with such therapeutic methods, metastatic cancer patients have a very poor prognosis and the five-year survival rate is about 10%. Furthermore, a problem with these therapeutic methods is high cost. In recent years, methods using molecular targeted agents have also become available against kidney cancer. However, such agents show a survival advantage for only a few months. Moreover, daily administration of the agents is essential, and frequently adverse effects of the agents have been reported.

To date, immunogene therapy is expected to be an effective therapeutic method for cancers, in particular, metastatic cancers. It is conceivable that immunogene therapy has fewer side effects and persistent therapeutic effects, and is cost-beneficial for patients. For example, recent studies have reported that an autologous tumor vaccine for introducing a granulocyte macrophage colony stimulating factor (GM-CSF) gene has a substantial anti-tumor activity in preclinical cancer models and some clinical trials. Serotype 5 E1-deleted adenovirus (Ad5)/GM-CSF is one of the most promising vectors. However, adenovirus vector-mediated gene transfer into various tumor cells is limited due to the lack of expression of integrins αvβ3 and αvβ5, and the Ad5 receptor, which is a coxsackie-adenovirus receptor (CAR).

Besides adenovirus vectors, poxvirus vectors, which are also double-stranded DNA vectors, and retroviral vectors such as single-stranded RNA (RT) viral vectors are commonly used. However, the clinical application of these vectors is limited by not only the complicated and time-consuming procedure for vector introduction, but also the insufficient GM-CSF production *in vivo.* The major obstacle for successful application of gene therapy strategy is the inability of recombinant viral vectors to spread to the whole tumor mass, and low efficiency of *in vivo* introduction (Non-patent Document 1). To promote gene therapy, it is necessary to develop a novel vector system that is capable of safely and efficiently introducing genes into target cells.

Sendai virus (SeV) vectors are cytoplasmic viral vectors with all steps of their expression carried out within the cytoplasm. Thus, even when the vectors are used *in vivo,* there is no worry that a carried gene will become integrated into the host chromosome and cause genetic toxicity. Furthermore, these vectors have a number of excellent characteristics, such as high efficiency of gene transfer and expression both *in vitro* and *in vivo,* and long-term sustained expression *in vitro.* Thus, SeV is expected to have many applications and uses as a gene transfer vector in gene therapy, gene vaccination, antibody production, and functional analysis, and such (Non-patent Documents 2 and 3). In recent years, the advanced engineering of the negative-strand RNA viral genome has enabled additional insertion of non-viral genes into the genome, and thus the development of a new class of viral vectors for gene transfer approaches has become possible (Non-patent Document 2).
[Patent Document 1] International Publication WO2003/25570
[Non-Patent Document 1] Nat. Med., 3: 1354-1361, 1997
[Non-Patent Document 2] J. Gene Med., 5(7): 543-553, 2003
[Non-Patent Document 3] Curr. Opin. Mol. Ther., 7(4): 346-352, 2005
[Non-Patent Document 4] Abe J, et al. Journal of cancer research and clinical oncology. 1995; 121: 587-92
[Non-Patent Document 5] Kojima T, Yamazaki K, Tamura Y, et al. Granulocyte-macrophage colony-stimulating factor gene-transduced tumor cells combined with tumor-derived gp96 inhibit tumor growth in mice. Human gene therapy. 2003; 14: 715-28.
[Non-Patent Document 6] Shibata S, Okano S, Yonemitsu Y, et al. Induction of efficient antitumor immunity using dendritic cells activated by recombinant Sendai virus and its modulation by exogenous IFN-beta gene. J Immunol. 2006; 177: 3564-76.
[Non-Patent Document 7] Vertuani S, De Geer A, Levitsky V, Kogner P, Kiessling R, Levitskaya J. Retinoids act as multistep modulators of the major histocompatibility class I presentation pathway and sensitize neuroblastomas to cytotoxic lymphocytes. Cancer research. 2003; 63: 8006-13.
[Non-Patent Document 8] Soiffer R, Lynch T, Mihm M, et al. Vaccination with irradiated autologous melanoma cells engineered to secrete human granulocyte-macrophage colony-stimulating factor generates potent antitumor immunity in patients with metastatic melanoma. Proc Natl Acad Sci U S A. 1998; 95: 13141-6.
[Non-Patent Document 9] Tani K, Azuma M, Nakazaki Y, et al. Phase I study of autologous tumor vaccines transduced with the GM-CSF gene in four patients with stage IV renal cell cancer in Japan: clinical and immunological findings. Mol Ther. 2004; 10: 799-816.
[Non-Patent Document 10] Chu Y, Xia M, Lin Y, et al. Th2-dominated antitumor immunity induced by DNA immunization with the genes coding for a basal core peptide PDTRP and GM-CSF. Cancer gene therapy. 2006; 13: 510-9.
[Non-Patent Document 11] Ellyard JI, Simson L, Parish CR. Th2-mediated anti-tumour immunity: friend or foe? Tissue antigens. 2007; 70: 1-11.
[Non-Patent Document 12] Inoue M, et al. J. Virology. 2003; 77: 3238-46
[Non-Patent Document 13] Abe J ,et al. Journal of cancer research and clinical oncology. 1995; 121:587-92

### Disclosure of the Invention

### [Problems to be Solved by the Invention]

As described above, immunogene therapy is a promising gene therapy method for cancers, in particular, metastatic cancers. For example, recent studies have reported that an autologous tumor vaccine for introducing a granulocyte macrophage colony stimulating factor (GM-CSF) gene has a substantial anti-tumor activity in preclinical cancer models and some clinical trials. Thus, serotype 5 E1-deieted adenovirus (Ad5)/GM-CSF is expected to be one of the most promising vectors. However, adenovirus vector-mediated gene transfer into various tumor cells is limited due to the lack of expression of integrins αvβ3 and αvβ5, and the Ad5 receptor, which is a coxsackie-adenovirus receptor (CAR). Besides adenovirus vectors, poxvirus vectors, which are also double-stranded DNA vectors, and retroviral vectors such as single-stranded RNA (RT) viral vectors are commonly used. However, the clinical application of these vectors is limited by not only the complicated and time-consuming procedure for vector introduction, but also the insufficient GM-CSF production *in vivo.* Thus, to promote gene therapy, it is necessary to develop a new vector system that is capable of safely and efficiently introducing genes into target cells.

### [Means for Solving the Problems]

An important advantage of the negative-strand RNA viral vector-mediated gene delivery is that high efficiency gene delivery is achieved by a simple technique. In general, gene delivery mediated by a retroviral vector or such has low efficiency, and therefore requires the vector be concentrated by centrifugation for optimal gene delivery. However, centrifugation often reduces the viral titer. Furthermore, a toxic agent, polybrene, is sometimes needed for high efficiency infection (Bunnell, B.A. et al., Proc. Natl. Acad. Sci. U S A, 1995, 92: 7739-7743; Chuck, A.S., Hum. Gene Ther., 1996, 7: 743-750; Chinnasamy, D. et al., Blood 2000, 96: 1309-1316; Fehse, B. et al., Br. J. Haematol., 1998, 102: 566-574). Furthermore, *ex-vivo* administration sometimes requires the step of selecting cells retaining the introduced gene after cell infection. In contrast, a negative-strand RNA viral vector can achieve a more superior gene delivery by merely contacting cells with the virus without a special agent. In addition, the infection efficiency is very high, and it is usually unnecessary to select cells that have been introduced with the gene with agents or such after vector infection. Furthermore, in the negative-strand RNA viral vector-mediated gene delivery, the optimal efficiency can be achieved in very short cell exposure time (30 minutes or shorter). In view of clinical situations, these characteristics enable to simplify the procedure for administration *ex vivo, in vivo,* and such, and to minimize procedure-dependent adverse effects such as cell damage.

Negative-strand RNA viruses are excellent gene transfer vectors. Since the viruses do not have a DNA phase, and their transcription and replication occur only in the host cytoplasm, the viruses are not integrated into the chromosome (Lamb, R.A. and Kolakofsky, D., Paramyxoviridae: The viruses and their replication. In: Fields BN, Knipe DM, Howley PM, (eds). Fields of virology. Vol. 2. Lippincott-Raven Publishers: Philadelphia, pp. 1177-1204, 1996). Therefore, safety issues such as transformation and immortalization due to chromosomal aberration do not occur. This characteristic of negative-strand RNA viruses greatly contributes to safety when they are used as vectors. For example, results on foreign gene expression show that few nucleotide mutations are observed even after multiple consecutive passages of Sendai virus (SeV). This indicates that the viral genome is highly stable and the inserted foreign genes are stably expressed over a long period of time (Yu, D. et al., Genes Cells 2, 457-466 (1997)). Furthermore, there are qualitative advantages such as flexibility in the size and packaging of inserted genes due to the absence of a capsid structural protein. Thus, it is suggested that negative-strand RNA viral vectors are a novel class of highly efficient vectors for human anti-tumor gene therapy. Transmissible SeV vectors are capable of carrying an inserted foreign gene of up to at least 4 kb, and simultaneously expressing two or more types of genes by adding transcriptional units.

The Sendai virus (SeV) vector, which is a cytoplasmic viral vector, has come to be recognized as a new promising means of gene transfer, since it can infect almost all mammalian cells including tumor cells and propagate in the cells. Furthermore, SeV poses no risk of fusion caused by host DNA transfer that results in tumor formation. The present inventors compared the *in vivo* anti-tumor activity against mouse renal cell cancer (RENCA (RC)) between RC vaccine cells, RENCA/SeV18+mGM-CSF/TSΔF and RENCA/AdV+mGM-CSF, into which the SeV18/TSΔF (F gene-deficient) vector carrying GM-CSF (SeV18+mGM-CSF/TSΔF) and the Ad5 vector carrying GM-CSF (AdV+mGM-CSF) have been transduced, respectively.

The result showed that the *in vivo* anti-tumor effect was comparable between RENCA/Sev18+mGM-CSF/TSΔF and RENCA/AdV+mGM-CSF. In a vaccination model, RENCA/SeV18+mGM-CSF/TSΔF produced a superior protective effect as compared to RENCA/AdV+mGM-CSF (p<0.02). In both CTL and cytokine analyses, as compared to the respective control, tumor-specific response and significantly high production of IL-2, IL-4, and IFN-γ were observed in mice treated with RENCA/SeV18+mGM-CSF/TSΔF and RENCA/AdV+mGM-CSF, which showed suppressed tumor growth. In particular, IFN-γ production was much higher in RENCA/SeV18+mGM-CSF/TSΔF-treated mice than in RENCA/AdV+mGM-CSF-treated mice (p<0.05). The result obtained shows that RENCA/SeV18+mGM-CSF/TSΔF has *in vivo* anti-tumor effect comparable to that of RENCA/AdV+mGM-CSF, although the level of GM-CSF production is low. This suggests that SeV carrying GM-CSF may have a greater application for the production of autologous GM-CSF vaccines.

Specifically, the present invention relates to the following:
[1] a negative-strand RNA viral vector that carries a cytokine gene;
[2] the vector of [1], which is a paramyxovirus, vector;
[3] the vector of [2], which is a Sendai virus vector;
[4] the vector of any one of [1] to [3], wherein the cytokine gene encodes a cytokine having heparin-binding activity;
[5] the vector of [4], wherein the cytokine having heparin-binding activity is granulocyte macrophage colony stimulating factor (GM-CSF);
[6] the vector of any one of [1] to [3], wherein the cytokine gene encodes a chemokine;
[7] the viral vector of [6], wherein the chemokine is TARC or RANTES;
[8] the vector of any one of [1] to [7], which is intended for treatment of cancer;
[9] the vector of [8], wherein the cancer is metastatic cancer;
[10] the vector of [9], wherein the cancer is kidney cancer;
[11] the vector of [9], wherein the cancer is lung cancer;
[12] an anti-tumor composition that comprises the vector of any one of [1] to [11];
[13] the anti-tumor composition of [12], which comprises a dendritic cell; and
[14] the anti-tumor composition of [12] or [13], which comprises two types of vectors, wherein one of the vectors is a vector comprising a GM-CSF-encoding gene, and the other is a vector comprising a chemokine-encoding gene.

### [Effects of the Invention]

It was demonstrated that the gene transfer method could be simplified, and a superior protective effect could be produced *in vivo* by using negative-strand RNA virus vectors, in particular, Sendai virus-based vectors, to express cytokine genes. The vector system of the present invention could be, for example, a powerful tool for producing autologous tumor vaccines.

### Brief Description of the Drawings

The abbreviations are specifically defined as follows (they are also used in the Examples and drawings):
(1) AdV+GFP: the Ad5 vector carrying GFP (RDB:1727
   (http://www2.brc.riken.jp/cache/dna/1727))
(2) AdV+mGM-CSF: the Ad5 vector carrying mouse GM-CSF (mGM-CSF)
   (RDB:1419 (http://www2.brc.riken.jp/cache/dna/1419))
(3) SeV18+GFP/TSΔF: the F gene-deficient Sendai virus vector having temperature-sensitive mutations and carrying GFP
(4) SeV18+mGM-CSF/TSΔF: the F gene-deficient Sendai virus vector having temperature-sensitive mutations and carrying mGM-CSF
(5) SeV18+hGM-CSF/TSΔF: the F gene-deficient Sendai virus vector having temperature-sensitive mutations and carrying human GM-CSF (hGM-CSF)
(6) SeV18+GFP/ΔF: the F gene-deficient Sendai virus vector carrying GFP
(7) SeV18+mGM-CSF/ΔF: the F gene-deficient Sendai virus vector carrying mGM-CSF
(8) SeV 18+mTARC/ΔF: the F gene-deficient Sendai virus vector carrying mouse TARC (mTARC)
(9) SeV18+mRANTES/ΔF: the F gene-deficient Sendai virus vector carrying mouse RANTES (mRANTES)
(10) IrRENCA: RC vaccine cells (vaccine cells)
(11) IrRENCA/AdV+GFP: RC vaccine cells transduced with the vector of (1) (vaccine cells)
(12) IrRENCA/AdV+mGM-CSF: RC vaccine cells transduced with the vector of (2) (vaccine cells)
(13) IrRENCA/SeV 18+GFP/TSΔF: RC vaccine cells transduced with the vector of (3) (vaccine cells)
(14) IrRENCA/SeV18+mGM-CSF/TSΔF: RC vaccine cells transduced with the vector of (4) (vaccine cells)
(15) IrLLC: LLC vaccine cells (vaccine cells)
(16) IrLLC/SeV18+GFP/TSΔF: LLC vaccine cells transduced with the vector of (3) (vaccine cells)
(17) IrLLC/SeV I 8+mGM-CSF/TSΔF: LLC vaccine cells transduced with the vector of (4) (vaccine cells)
(18) LLC/SeV18+GFP/ΔF: LLC cells transduced with the vector of (6)
(19) LLC/SeV18+mGM-CSF/ΔF: LLC cells transduced with the vector of (7)
(20) LLC/SeV18+mTARC/ΔF: LLC cells transduced with the vector of (8)
(21) LLC/SeV18+mRANTES/ΔF: LLC cells transduced with the vector of (9)
(22) DC: dendritic cells
(23) DC/LPS: LPS-treated DC
(24) DC/SeV 18+GFP/TSΔF: dendritic cells transduced with the vector of (3)
(25) DC/SeV18+mGM-CSF/TSΔF: dendritic cells transduced with the vector of (4)

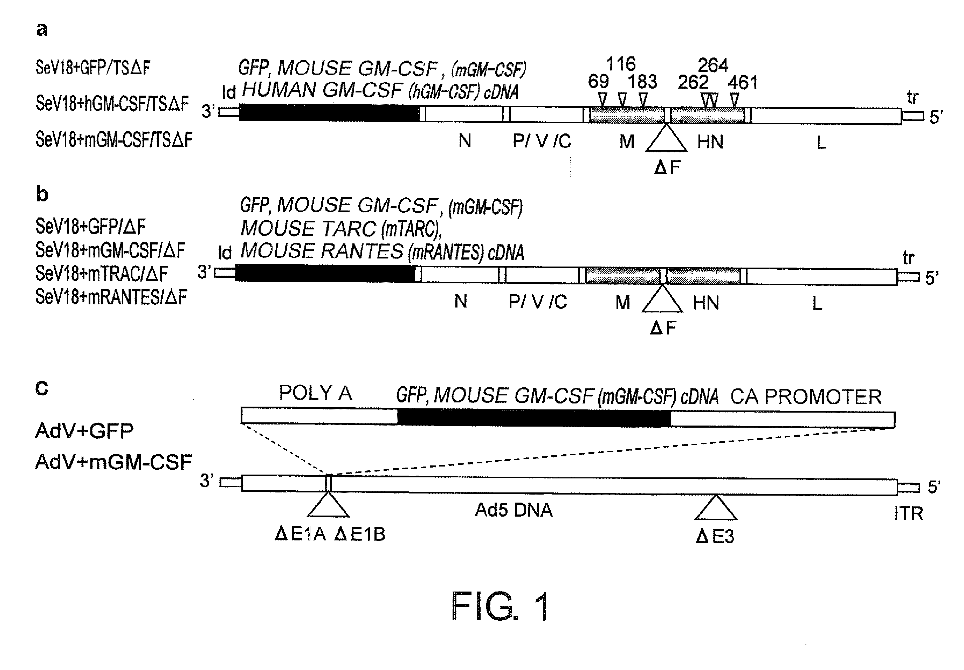

Fig. 1 shows the viral vectors used in the present invention. (a) SeV18+GFP/TSΔF, SeV18+mGM-CSF/TSΔF, and SeV18+hGM-CSF/TSΔF are shown. The SeV genome is delimited by the following two promoter regions: the leader (ld) and trailer (tr) regions. The respective exogenous genes (GFP, mGM-CSF, and hGM-CSF) were inserted between the leader sequence and the open reading frame of the N gene. The SeV genes encode the envelope-related proteins, M, F, and HN, and the negative-strand genomic ribonucleotide-protein complex (RNP) proteins, N, P/N/C, and L. Sendai virus vectors having temperature-sensitive mutations do not express the envelope-related M and HN genes, and have ribonucleotide substitutions in the M, HN, and L genes, as indicated by the arrowheads (Inoue M, Tokusumi Y, Ban H, et al. Nontransmissible virus-like particle formation by F-deficient sendai virus is temperature sensitive and reduced by mutations in M and HN proteins. Journal of virology. 2003; 77: 3238-46). (b) SeV18+GFP/ΔF, SeV18+mGM-CSF/ΔF, and SeV18+mTARC/ΔF, and SeV 18+mRANTES/ΔF are shown. The SeV genome is delimited by the following two promoter regions: the leader (ld) and trailer (tr) regions. The respective exogenous genes (GFP, mGM-CSF, and hGM-CSF) were inserted between the leader sequence and the open reading frame of the N gene. The SeV genes encode the envelope-related proteins, M, F, and HN, and the negative-strand genomic ribonucleotide-protein complex (RNP) proteins, N, P/N/C, and L. (c) The recombinant AdV vectors containing the GFP and mouse GM-CSF cDNA expression cassettes (AdV/GFP and AdV/mGM-CSF) were constructed by homologous recombination between the expression cosmid cassette and the parental virus genome (Abe J, Wakimoto H, Yoshida Y, Aoyagi M, Hirakawa K, Hamada H. Antitumor effect induced by granulocyte/macrophage-colony-stimulating factor gene-modified tumor vaccination: comparison of adenovirus- and retrovirus-mediated genetic transduction. Journal of cancer research and clinical oncology, 1995; 121: 587-92). The expression of these genes was driven by a CAG promoter. These replication-defective adenovirus serotype 5 (Ad5)-based vectors have deletions in the E1A, E1B, and E3 regions.

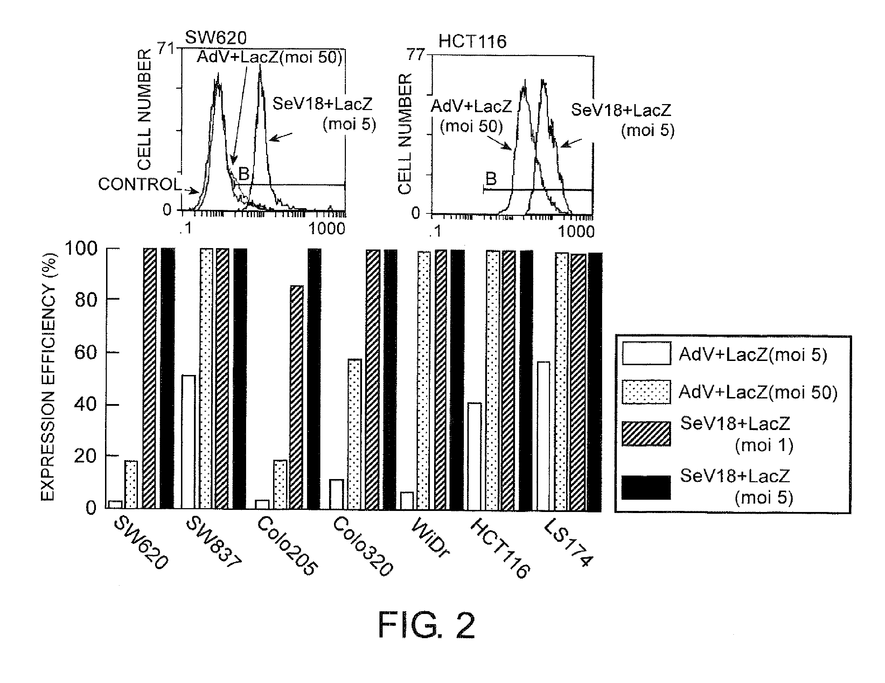

Fig. 2 shows the result of comparing the efficiency of gene transfer and expression between SeV and an adenovirus vector (AdV) in a colon cancer cell line.

The abbreviations in this figure are specifically defined as follows.
(1) AdV+LacZ (moi 5): a LacZ-carrying Ad5 vector was transduced into cells at an MOI of 5.
(2) AdV+LacZ (moi 50): a LacZ-carrying Ad5 vector was transduced into cells at an MOI of 50.
(3) Sev18+LacZ/TSΔF (moi 1): the F gene-deficient Sendai virus vector having temperature-sensitive mutations and carrying LacZ was transduced into cells at an MOI of 1.
(4) SeV18+LacZ/TSΔF (moi 5): the F gene-deficient Sendai virus vector having temperature-sensitive mutations and carrying LacZ was transduced into cells at an MOI of 5.

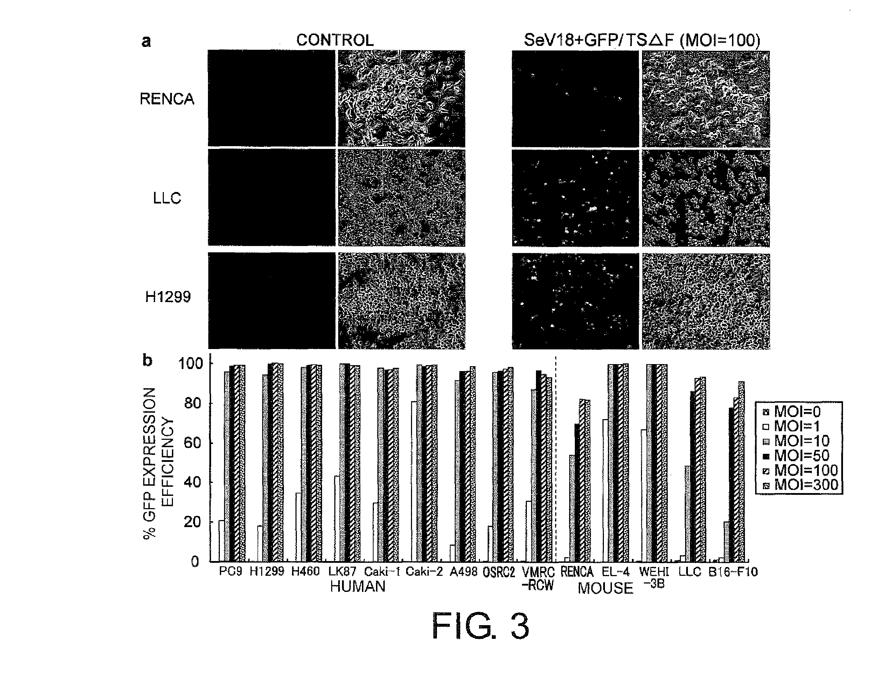

Fig. 3 presents photographs and a graph showing the transduction efficiency of various mouse and human cell lines. (a) SeV18+GFP/TSΔF was transduced into RENCA (upper row), LLC (middle row), and H1299 cells (lower row) at an MOI of 100 (indicated as "SeV18+GFP/TSΔF" on the panel). The "CONTROL" shows RENCA (upper row), LLC (middle row), and H1299 cells (lower row) into which no vector (*e.g.,* Sev18+GFP/TSΔF) was transduced. After 48 hours, fluorescence microscopy of transduced cells was performed (the GFP phase is displayed in the third column). The background fluorescence of transduced cells (control; first column) was determined in the "CONTROL". Phase contrast pictograms are displayed in the second and fourth columns. (b) Sev18+GFP/TSΔF was transduced into nine mouse and five human cell lines at an MOI of 0, 1, 10, 50, 100, or 300. The percentage of GFP expressing cells was determined by flow cytometry analysis. The bar graph depicts the percentage of GFP-positive cells at 48 hours post transduction in the presence (MOI-1, 10, 50, or 100) or absence (MOI=0) of SeV18+GFP/TSΔF.

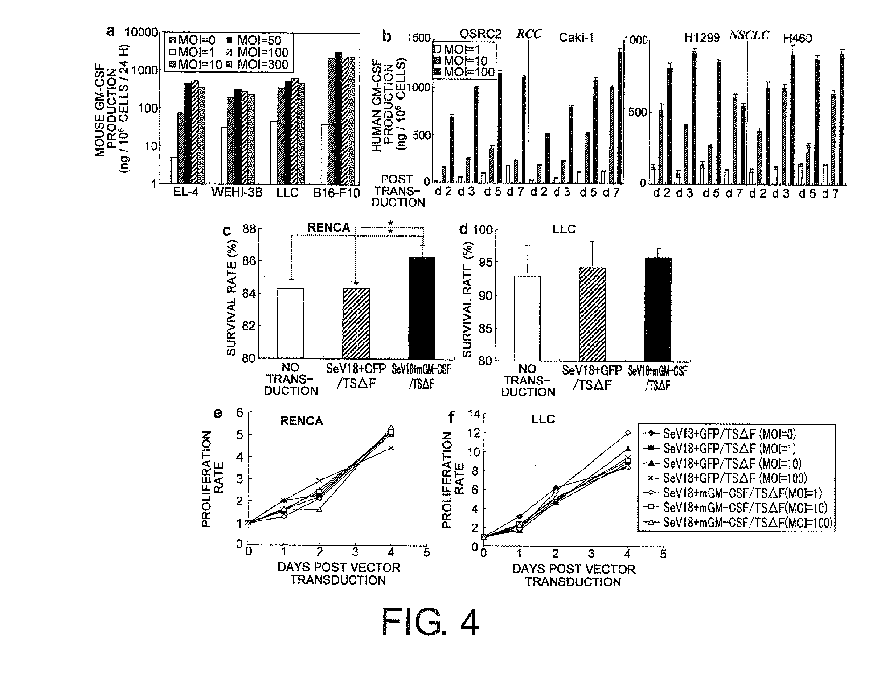

Fig. 4 shows the transduction of SeV18+mGM-CSF/TSΔF and SeV18+hGM-CSF/TSΔF into mouse and human tumor cell lines. (a) One million cells of four mouse tumor cell lines were transduced with SeV/ΔF/mGM at an MOI of 0, 1, 10, 50, 100, or 300 for 90 minutes in serum-free RPMI, and incubated in 10% FBS/RPMI in 6-well plates for 24 hours. The level of mouse GM-CSF produced in the supernatant was measured by ELISA. (b) Similar to (a), the human GM-CSF level in four human cell lines (two NSCLC and two RCC lines) transduced with SeV/ΔF/hGM at an MOI of 1, 10, or 100 on days two, three, five, and seven post infection was measured by ELISA. (c and d) Cell viability after SeV infection was evaluated by trypan blue exclusion. Two million parental RENCA or LLC cells were infected with SeV/ΔF/GFP (MOI=100) or SeV/ΔF/mGM (MOI=100) for 90 minutes, and cultured for 48 hours. The number of trypan blue-positive and -negative cells was counted under a light microscope, and the percentage of cells that had excluded trypan blue was represented as an index of cell viability. (e and f) *in vitro* proliferation assay. RENCA and LLC cells were cultured separately in 96-well microplates at 10⁴ cells/well. These cells were infected with SeV18+GFP/TSΔF (MOI=1, 10, or 100) or SeV18+mGM-CSF/TSΔF (MOI=1, 10, or 100) for 90 minutes in serum-free medium, and cultured for one, two, and four days in 10% FBS/RPMI or 10% FBS/DMEM, respectively. At each time point (day zero, one, two, and four post SeV infection), the number of viable cells was estimated spectrophotometrically by the incorporation of tetrazolium dye using Cell Count Reagent SF. Representative data from three independent experiments are shown.

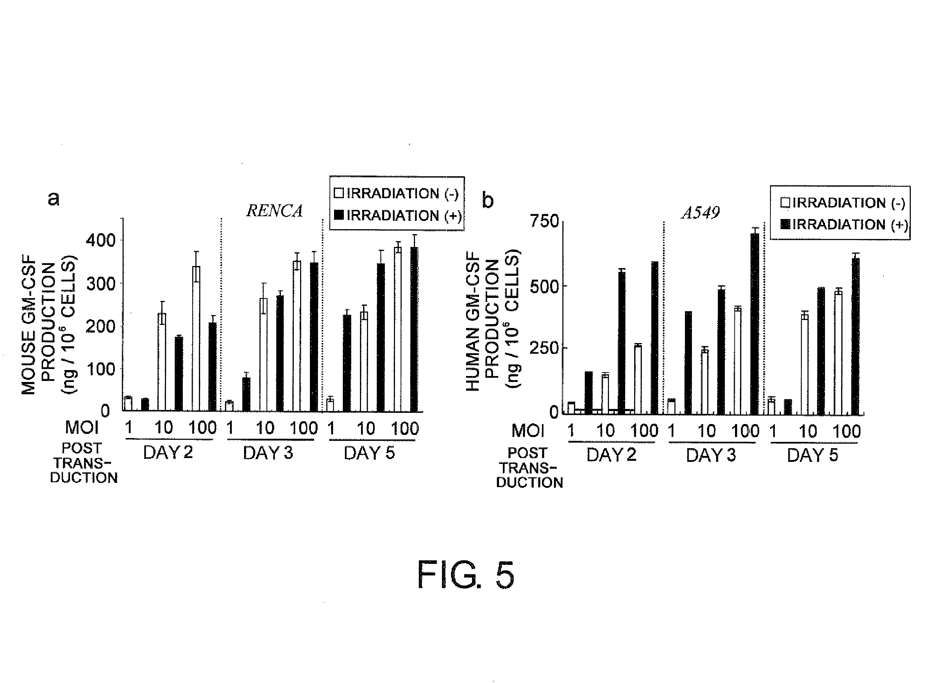

Fig. 5(a) shows the amount of mGM-CSF produced in mouse RENCA cells transduced with SeV18+mGM-CSF/TSΔF. The amount was determined by ELISA. Fig. 5(b) shows the amount of hGM-CSF produced in human H1299 cells transduced with SeV18+hGM-CSF/TSΔF. The amount was determined by ELISA.

In both (a) and (b) of this figure, the bars show mean ± SEM, and the abbreviations are defined below.
Irradiation (+): samples irradiated one day after transduction of SeV 18+mGM-CSF/TSΔF or SeV18+hGM-CSF/TSΔF into cells.
Irradiation (-): samples without irradiation.
Day 2: samples collected two days after transduction of SeV 18+mGM-CSF/TSAF or SeV18+hGM-CSF/TSΔF into cells.
Day 3: samples collected three days after transduction of SeV 18+mGM-CSF/TSΔF or SeV 18+hGM-CSF/TSΔF into cells.
Day 5: samples collected five days after transduction of Sev18+mGM-CSF/TSΔF or SeV 18+hGM-CSF/TSΔF into cells.
MOI 1: samples obtained by transducing SeV18+mGM-CSF/TSΔF or SeV18+hGM-CSF/TSΔF into cells at an MOI of 1.
MOI 10: samples obtained by transducing SeV18+mGM-CSF/TSΔF or SeV18+hGM-CSF/TSΔF into cells at an MOI of 10.
MOI 100: samples obtained by transducing SeV18+mGM-CSF/TSΔF or SeV18+hGM-CSF/TSΔF into cells at an MOI of 100.

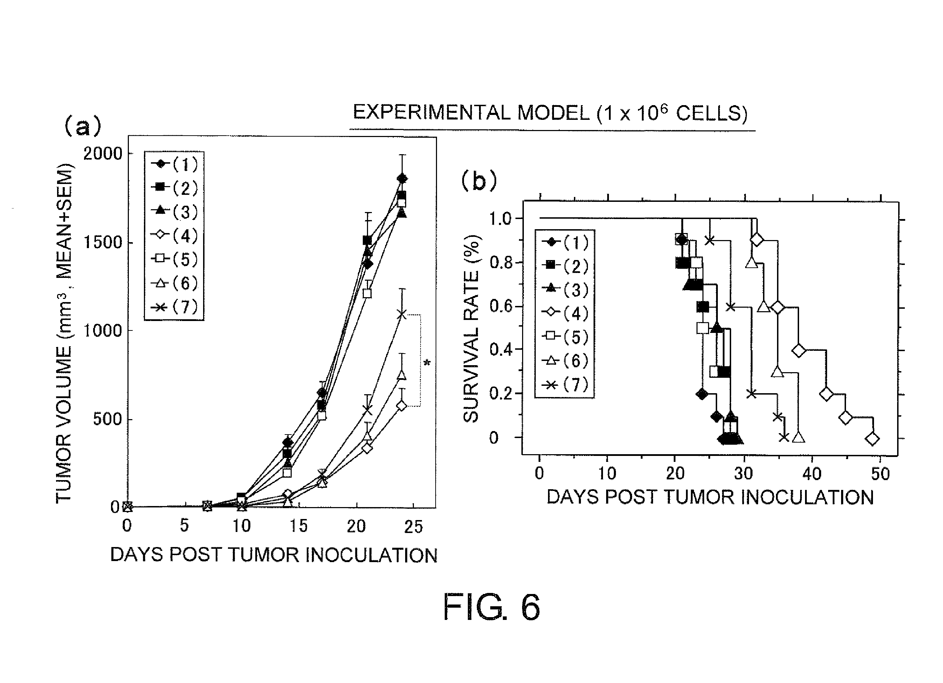

Fig. 6 shows the effect of RENCA/SeV 18+mGM-CSF/TSΔF on the tumor (kidney cancer) model mice (RENCA model mice). The tumor volume (left panel, a) and mouse survival rate (right panel, b) were monitored over time. The significant difference is indicated with an asterisk (*p<0.05). The abbreviations in this figure are defined below:
(1) the group administered with HBSS (Hanks' balanced solution salt),
(2) the group administered with IrRENCA irradiated at 50 Gy,
(3) RC vaccine cells transduced with SeV18+GFP/TSΔF (MOI=100) irradiated at 50 Gy (IrRENCA/SeV18+GFP/TSΔF (MOI=100)),
(4) RC vaccine transduced with SeV18+mGM-CSF/TSΔF (MOI=100) irradiated at 50 Gy (IrRENCA/SeV18+mGM-CSF/TSΔF (MOI=100)),
(5) RC vaccine transduced with AdV+GFP (MOI=300) irradiated at 50 Gy (IrRENCA/AdV+mGM-CSF (MOI=300)),
(6) RC vaccine transduced with AdV+mGM-CSF (MOI=300) irradiated at 50 Gy (IrRENCA/AdV+mGM-CSF (MOI=300)),
(7) RC vaccine transduced with AdV+mGM-CSF (MOI=5) irradiated at 50 Gy (IrRENCA/AdV+mGM-CSF (MOI=5)).

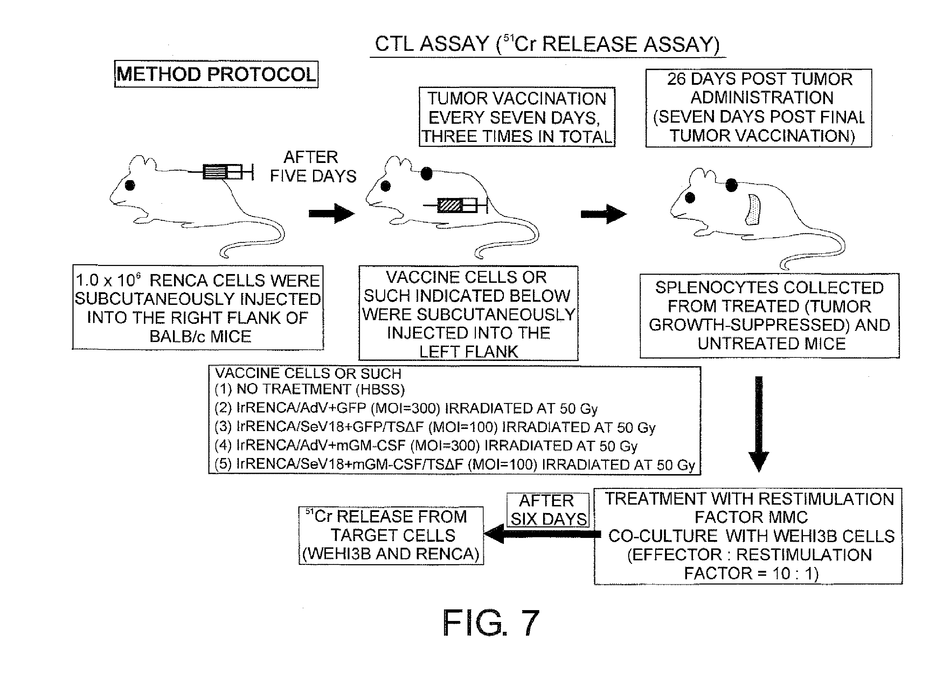

Fig. 7 shows the outline of the experimental procedure of CTL assay using IrRENCA/SeV18+mGM-CSF/TSΔF.

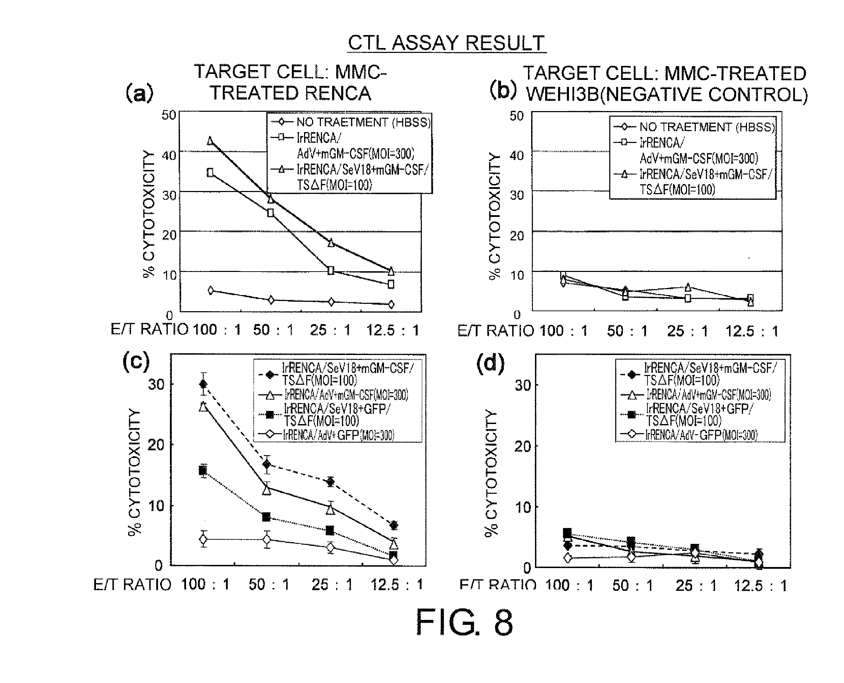

Fig. 8 presents the result of CTL assay using IrRENCA/SeV18+mGM-CSF/TSΔF. The tumor-specific response in comparison to the negative control is shown.

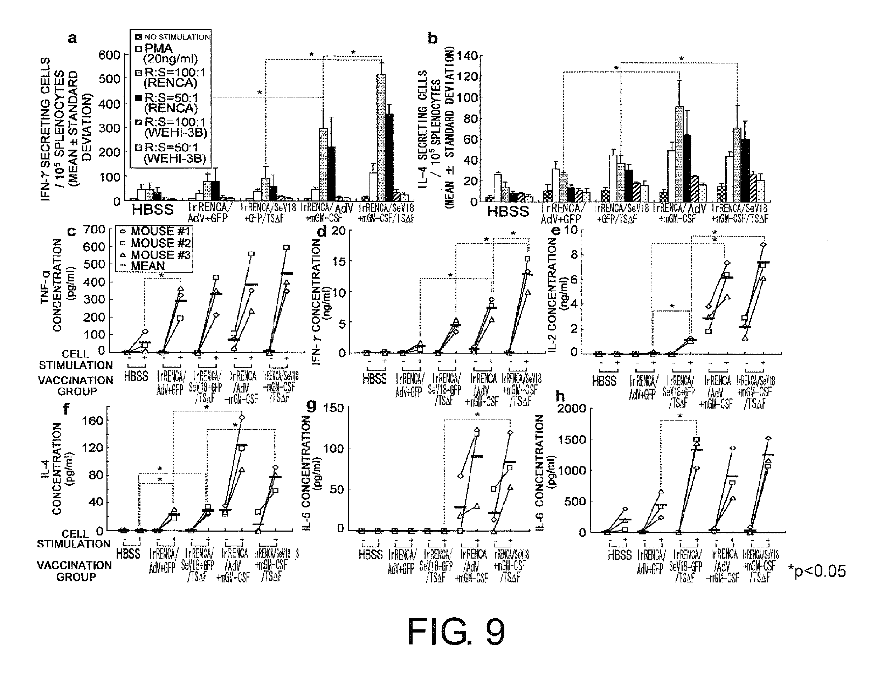

Fig. 9 shows the IFN-γ and IL-4 production by splenocytes from mice immunized with IrRENCA/AdV+mGM-CSF or IrRENCA/SeV18+mGM-CSF evaluated using mouse IFN-γ (a) and IL-4 (b) ELISPOT assays. Ten thousand splenocytes from RENCA-bearing mice vaccinated with the above-mentioned tumor vaccines were incubated for 20 hours with stimulator cells at the above-mentioned ratios. Bound cytokines were visualized by incubation with biotinylated anti-IFN-γ and anti-IL-4 monoclonal antibodies, and then with streptavidin-HRP and premixed peroxidase substrate AEC. The result is expressed as the mean number of spot-forming cells + SD from quadruplicate determinations per 1 x 10⁵ splenocytes. Figs. 9(c) to (h) present the result of mixed cultures of RENCA (cancer) cells and the above-mentioned splenocytes. Specifically, the cytokine production in the mixed-culture cells was determined. Tumor-specific cytokine production by splenocytes stimulated with GM-CSF-transduced tumor vaccination is shown. The concentration of mouse TNF-α (c), IFN-γ (d), IL-2 (e), IL-4 (f), IL-5 (g), or IL-6 (h) in the culture supernatant was measured by CBA (c to g) or ELISA (h). Significant differences are denoted with asterisks (*p<0.05). The abbreviations are defined below.
Cell stimulation (-): non-irradiated RENCA (cancer) cells.
Cell stimulation (+): irradiated RENCA (cancer) cells.

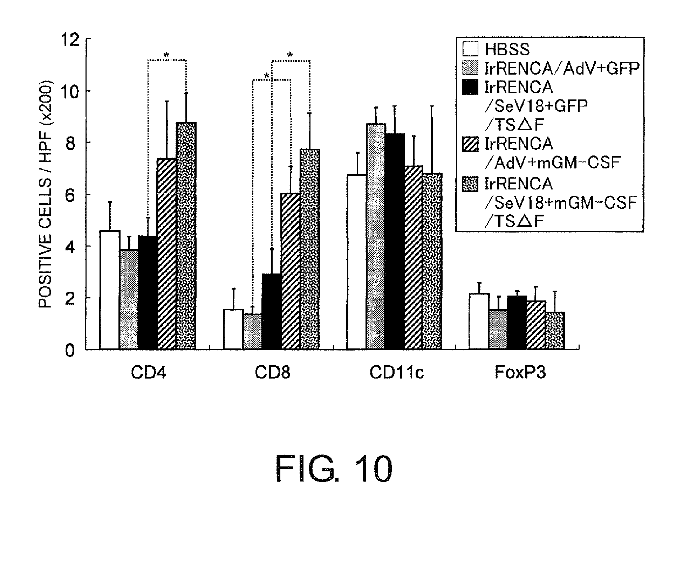

Fig. 10 shows the immunophenotypic analysis of tumor-infiltrating cells in a tumor mass. RENCA-bearing mice were either left untreated (HBSS) or treated with the above-mentioned tumor vaccine cells (IrRENCA/AdV+GFP, IrRENCA/AdV+mGM-CSF, IrRENCA/SeV18+GFP/TSΔF, or IrRENCA/SeV 18+mGM-CSF/TSΔF), as described in "Materials and methods". Resected RENCA tumors were then subjected to immunohistological evaluation. To evaluate the distribution of CD4⁺ T, CD8⁺ T, CD11c⁺ and FoxP3⁺ T cells in tumors, positively stained cells were enumerated microscopically at 200X magnification in 30 to 70 HPFs. The bars depict the mean ± SEM. Significant differences are denoted with asterisks (*p<0.05).

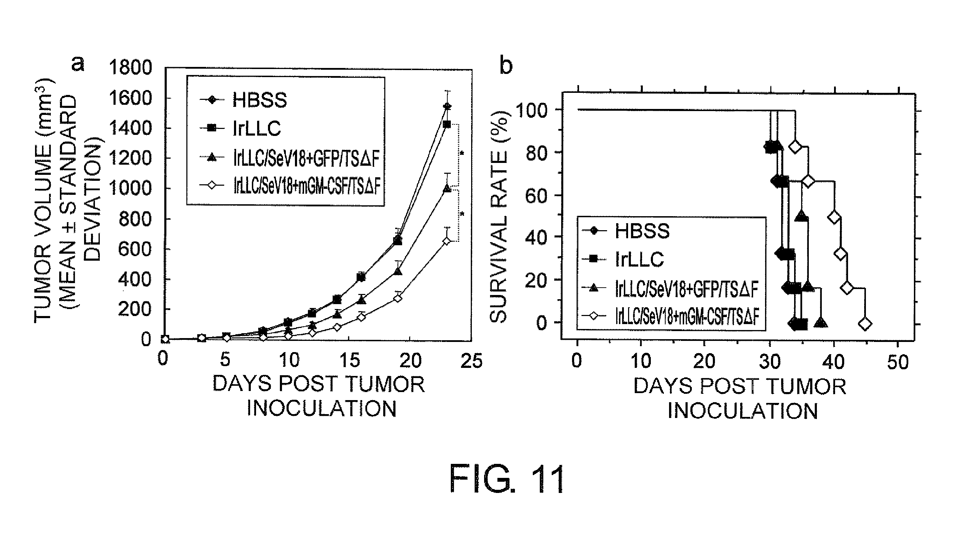

Fig. 11 shows the antitumor effect of IrLLC/SeV 18+mGM-CSF/TSΔF in tumor (lung cancer) model mice (LLC model mice). 2 x 10⁵ mouse LLC tumor cells were inoculated subcutaneously into the right flank of C57BL/6 mice (day zero). From day three post inoculation, IrLLC irradiated at 50 Gy (1.0 x 10⁶ cells), IrLLC/SeV18+GFP/TSΔF irradiated at 50 Gy (MOI=100, 1.0 x 10⁶ cells), or IrLLC/SeV18+mGM-CSF/TSΔF irradiated at 50 Gy (MOI=100, 1.0 x 10⁶ cells) were subcutaneously inoculated into the left flank every four days for three times as tumor vaccination. The tumor volume and survival rate were evaluated. Significant differences are denoted with asterisks (*p<0.05).

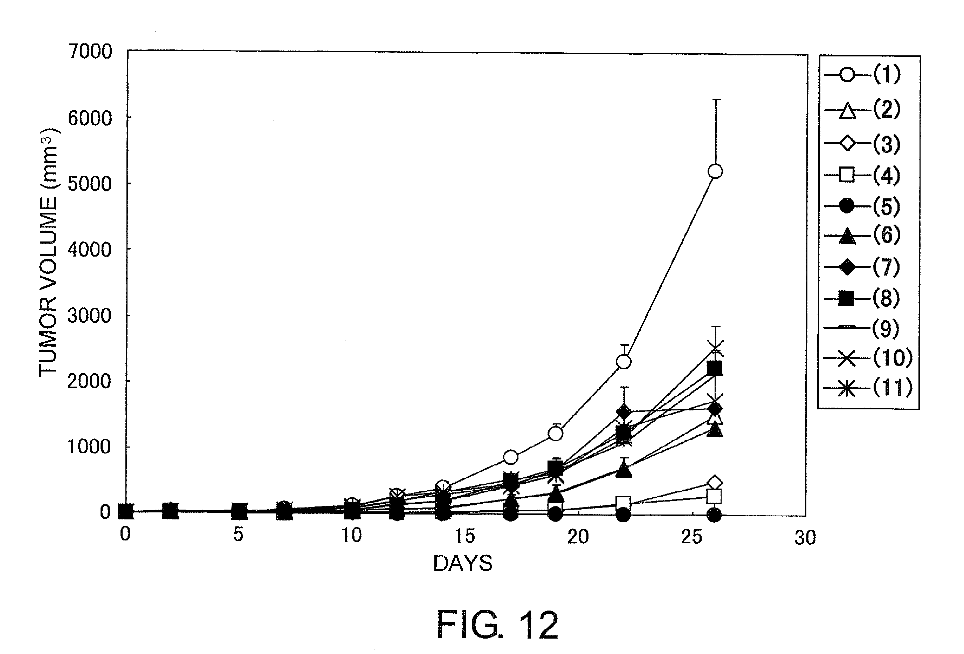

Fig. 12 presents a graph showing the tumor formation-suppressing effect of SeV18+mGM-CSF/ΔF, etc. The cells listed below were subcutaneously inoculated into the right flank region of Balb/c mice (n = 6) (referred to as administration groups (1) to (11)). The groups (1) to (11) are as follows:
(1) the group administered with 5.0 x 10⁵ LLC cells,
(2) the group administered with 5.0 x 10⁵ LLC cells transduced with SeV18+GFP/ΔF (MOI=10)
   (LLC/SeV18+GFP/ΔF (MOI=10)),
(3) the group administered with 5.0 x 10⁵ LLC cells transduced with SeV18+GFP/ΔF (MOI=100) (LLC/SeV18+GFP/ΔF (MOI=100)),
(4) the group administered with 5.0 x 10⁵ LLC cells transduced with SeV18+mGM-CSF/ΔF (MOI=10) (LLC/SeV18+mGM-CSF/ΔF (MOI=10)),
(5) the group administered with 5.0 x 10⁵ LLC cells transduced with SeV18-mGM-CSF/ΔF (MOI=100) (LLC/SeV18+mGM-CSF/ΔF (MOI=100)),
(6) the group administered with 5.0 x 10⁵ LLC cells transduced with SeV18+mTARC/ΔF (MOI=10) (LLC/SeV18+mTARC/ΔF (MOI=10)),
(7) the group administered with 2.5 x 10⁵ LLC/SeV18+mTARC/ΔF (MOI=10) cells and 2.5 x 10⁵ LLC cells,
(8) the group administered with 5.0 x 10⁴ LLC/SeV18+mTARC/ΔF (MOI=10) cells and 4.5 x 10⁵ LLC cells,
(9) the group administered with 5.0 x 10⁵ LLC cells transduced with SeV18+mRANTES/ΔF (MOI=10) (LLC/SeV18+ mRANTES/ΔF (MOI=10)),
(10) the group administered with 2.5 x 10⁵ LLC/SeV18+mRANTES/ΔF (MOI=10) cells and 2.5 x 10⁵ LLC cells,
(11) the group administered with 5.0 x 10⁴ LLC/SeV 18+mRANTES/ΔF (MOI=10) cells and 4.5 x 10⁵ LLC cells.

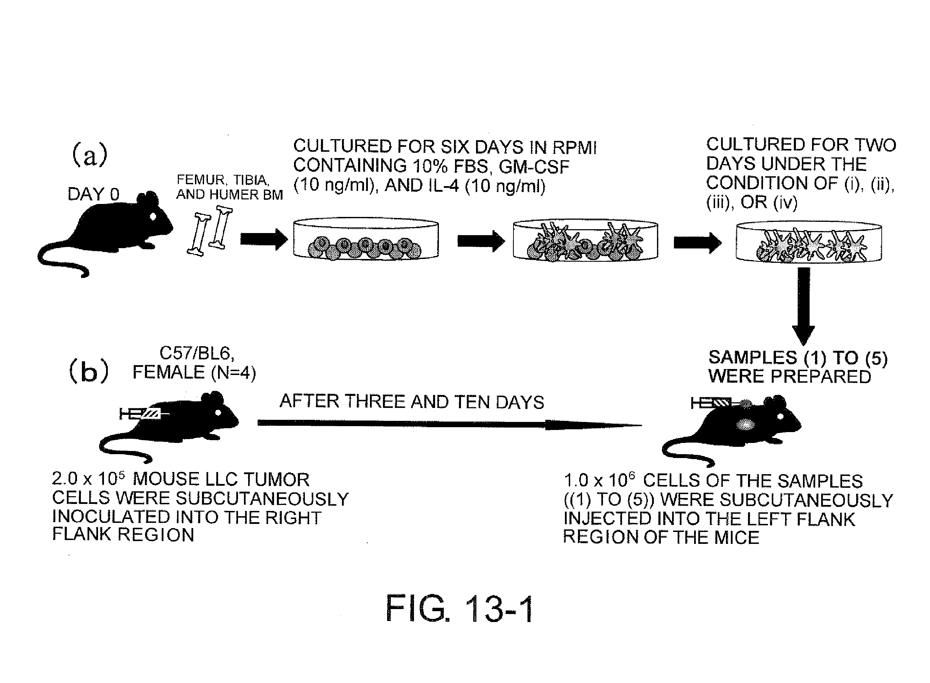

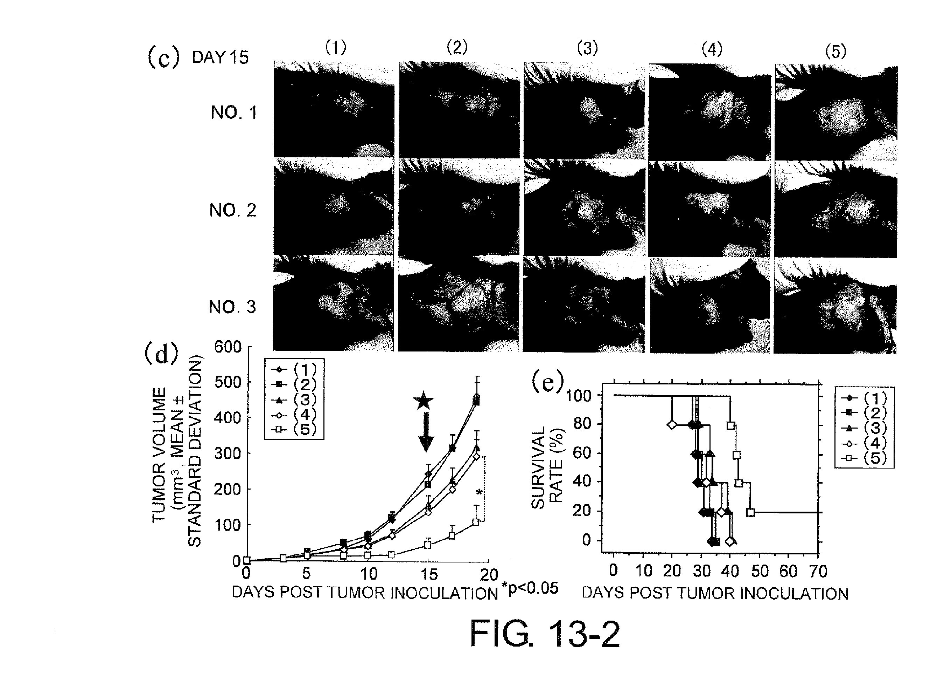

Figs. 13-1 and 13-2 show the anti-tumor effect of DC/SeV18+mGM-CSF/TSΔF on a mouse tumor (lung cancer) model (LLC model).
(a) shows the method for culturing dendritic cells used in this experiment. Bone marrow (tibia, femur, and humerus) was collected from C57/BL6 mice. After removing erythrocytes, the cells were cultured for six days in RPMI containing 10% FBS, GM-CSF (10 ng/ml), and IL-4 (10 ng/ml). Then, the cells cultured for six days were further cultured for two days in RPMI containing 10% FBS. Dendritic cells were prepared by this procedure.
   The dendritic precursor cells were cultured for another two days under the four culture conditions ((i) to (iv)) described below to prepare the four types of dendritic cells used in this experiment.
   (i) RPMI containing 10% FBS
   (ii) RPMI containing 10% FBS and 1.0 µg/ml LPS (referred to as "LPS-treated")
   (iii) RPMI containing 10% FBS and SeV18+GFP/TSΔF (MOI=100)
   (iv) RPMI containing 10% FBS and SeV18+mGM-CSF/TSΔF (MOI=100)
(b) shows a schematic diagram illustrating the method for administering each of the following (1) to (5) to female C57BL/6 mice (n = 4). The abbreviations (1) to (5) below are used in (a) to (e) of this figure.
   (1) HBSS (Hanks' balanced solution salt)
   (2) HBSS containing DC (prepared according to the method described in (a) above and the condition described in (i) above)
   (3) HBSS containing DC/LPS (prepared according to the method described in (a) above and the condition described in (ii) above)
   (4) HBSS containing DC/SeV18+GFP/TSΔF (prepared according to the method described in (a) above and the condition described in (iii) above)
   (5) HBSS containing DC/SeV18+mGM-CSF/TSΔF (prepared according to the method described in (a) above and the condition described above in (iv) above)

   Lung cancer model mice were prepared by subcutaneously inoculating 2.0 x 10⁵ mouse LLC tumor cells into the right flank region of C57BL/6 mice (n = 4). Three days after inoculation (when the tumor diameter was 3 to 5 mm), samples of 1.0 x 10⁶ cells ((1) to (5) above) were subcutaneously inoculated into the left flank region of the above-described model mice. Furthermore, ten days after inoculation of the tumor cells described above, samples of 1.0 x 14⁶ cells ((1) to (5) above) were subcutaneously inoculated into the left flank region of the above-described model mice.
(c) presents photographs showing tumors formed on the right flank region of the above-mentioned mice at the time point marked with a star in graph d. Three mice were selected from each of the administration groups (1) to (5) described above, and the tumors formed in the right flank region of the mice were photographed.
(d) presents a graph showing the tumor volume after administration of each of (1) to (5) above to the mice described above. The significant difference is indicated with an asterisk (* p<0.05).
(e) presents a graph showing the mouse survival rate after administration of each of (1) to (5) above to the mice described above.

### Mode for Carrying Out the Invention

The present invention provides negative-strand RNA virus vectors comprising cytokine genes.

### (1) Negative-strand RNA virus

A negative-strand RNA virus is an envelope virus that includes a minus-strand (an antisense strand against a sense strand that encodes viral proteins) RNA (also referred to as negative-strand RNA) as the genome. The virus exhibits high infectivity and is capable of overexpressing the genes carried by the virus in the cytoplasm. Negative-strand RNA viruses used in the present invention particularly include negative single-stranded RNA viruses (also referred to as "non-segmented negative-strand RNA viruses"). The "negative single-stranded RNA viruses" refers to viruses that include a negative single-stranded (i.e., minus-strand) RNA as the genome. Such viruses include those belonging to *Paramyxoviridae* (including the genera *Paramyxovirus, Morbillivirus, Rubulavirus,* and *Pneumovirus,* etc.), *Rhabdoviridae* (including the genera *Vesiculovirus, Lyssavirus,* and *Ephemerovirus,* etc.), *Filoviridae, Orthomyxoviridae,* (including Influenza viruses A, B, and C, and Thogoto-like viruses, etc.), *Bunyaviridae* (including the genera *Bunyavirus, Hantavirus, Nairovirus,* and *Phlebovirus,* etc.), *Arenaviridae,* and such.

The advantages of negative-strand RNA viruses include the following: (i) there is no risk of integration into the genomic DNA, since the replication cycle occurs exclusively in the cytoplasm; (ii) the introduction efficiency does not depend on the cell cycle of target cells; (iii) homologous recombination does not occur between the virus and a different viral genome or a wild type virus; (iv) virus incorporation into cells requires only a very short contact time; (v) genes encoded by the virus can be strongly and controllably expressed in a broad range of host cells. The viruses are expected to be suitable for use in immunogene therapy that utilizes various cytokines due to the above-mentioned advantages and the induction of superior protective effect demonstrated herein.

Negative-strand RNA viruses that are preferably used in the present invention include viruses of *Paramyxoviridae, Orthomyxoviridae,* and *Rhabdoviridae.* Among them, viruses of *Paramyxoviridae* and derivatives thereof are preferred as the vectors of the present invention. Herein, "paramyxovirus" refers to a virus of *Paramyxoviridae,* or a derivative thereof. *Paramyxoviridae* is a group of viruses that have a non-segmented negative-strand RNA as the genome, and includes *Paramyxovirinae* (including the genera *Respirovirus* (also referred to as *Paramyxovirus*), *Rubulavirus,* and *Morbillivirus*) and *Pneumovirinae* (including the genera *pneumovirus* and *metapneumovirus*). The viruses that are used in the present invention are more preferably viruses belonging to *Paramyxoviridae* (including the genera *Respirovirus, Rubulavirus,* and *Morbillivirus*) or derivatives thereof, and still more preferably viruses belonging to the genus *Respirovirus* (also referred to as the genus *Paramyxovirus*) or derivatives thereof.

Specifically, the viruses belonging to *Paramyxoviridae* include the following: Sendai virus (SeV, also referred to as mouse parainfluenza virus-1), Newcastle disease virus (NDV), mumps virus, measles virus, respiratory syncytial virus (RS virus), rinderpest virus, distemper virus, simian parainfluenza virus 5 (SV5), SPIV-10), human parainfluenza viruses 1, 2, 3, 4a, and 4b (HPIV-1, 2, 3, 4a, and 4b), bovine parainfluenza virus-3 (BPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), etc. More preferably, the paramyxovirus of the present invention is Sendai virus.

Sendai virus is known to be pathogenic in rodents causing pneumonia, but is not pathogenic for human. This is also supported by a previous report that nasal administration of wild type Sendai virus does not have severely harmful effects on non-human primates (Hurwitz, J.L. et al., Vaccine 15: 533-540, 1997; Bitzer, M. et al., J. Gene Med. 5: 543-553, 2003). These characteristics of Sendai virus suggest that when a vector is constructed based on Sendai virus, the vector can be applied to human treatment, and can be a promising choice in gene therapy for human cancer or such.

Herein, "derivatives" of a virus refers to viruses that have been genetically modified or chemically modified in a manner not to impair the gene-transferring ability of the viral vectors. The viral vectors of the present invention may be derived from natural strains, wild type strains, mutant strains, laboratory-passaged strains, artificially-constructed strains, or such.

### (2) Cytokine genes

In the present invention, "gene" refers to a genetic substance, i.e., a nucleic acid encoding a transcriptional unit. The genes include RNAs and DNAs. In the present invention, a nucleic acid encoding a protein is referred to as a gene for the protein. In general, a gene may have a naturally-occurring or artificially-designed sequence. In the present invention, "DNAs" include both single-stranded and double-stranded DNAs. "Encoding a protein" means that a polynucleotide comprises an ORF that encodes an amino acid sequence of the protein in the sense or antisense direction, so that the protein can be expressed under appropriate conditions.

Cytokines to be carried by the vectors of the present invention may be cytokines that induce immune cell differentiation and/or growth, and have anti-tumor activity. Such cytokines include cytokine that are produced by T cells, NK cells, monocytes, macrophages, or such, and induce T cell differentiation and/or growth. An immunostimulatory cytokine gene can be isolated, for example, from T-cell-derived cDNAs or such by PCR amplification using primers designed based on the sequence of the gene. Cytokines having anti-tumor activity are well known to those skilled in the art. Such cytokine genes can be preferably used in the present invention. For example, the effectiveness of IL-2, IL-4, and GM-CSF has been demonstrated in brain tumor animal models (IL-2: Iwadate, Y. et al., Cancer Res., 61: 8769-8774 (2001); IL-2, IL-4, GM-CSF: Sampson, J.H. et al., Proc. Natl. Acad. Sci. USA 93, 10399-10404 (1996); GM-CSF: Herrlinger, U. et al., Cancer Gene Ther. 4, 345-352 (1997); IL-4: Seleh, M. et al., J. Natl. Cancer Inst. 91, 438-445, (1999); IL-4: Giezeman-Smits, K.M. et al., Cancer Res. 60, 2449-2457 (2000)). IL-23 has been demonstrated to be closely associated with the migration of immune cells in brain autoimmune diseases (Becher B. et al., J Clin Invest. 112(8), 1186-91 (2000)). Meanwhile, Fas-L has been shown to be effective as a chemoattractant (Silvestris F et al., Br J Haematol. 122(1) 39-52. (2003)). For activation of the immune system against tumors by IL-2 or such, see the following documents: Iwadate, Y et al., Cancer Gene Ther. 7, 1263-1269 (2000); Iwadate, Y et al. Cancer Res. 61, 8769-8774 (2001); Iwadate, Y. et al. Int. J. Mol. Med. 10, 741-747 (2002); Iwadate, Y. et al. Oncology (Basel) 54, 329-334 (1997); Iwadate, Y. et al. Int. J. Oncol. 23, 483-488 (2003).

Cytokine genes that are particularly useful in the present invention include those encoding cytokines that have heparin-binding activity, such as GM-CSF. Furthermore, cytokine genes of the present invention also include genes encoding chemokines such as Thymus and activation-regulated chemokine (TARC) and RANTES. The sequence of GM-CSF is known, and one can obtain it, for example, using the Accession numbers M11220 (protein ID AAA52578), A14305 (protein ID CAA01150), etc. It is also possible to use known sequence information for other cytokines.

Cytokine genes that are used in the present invention may be derived from human or other mammals, for example, mouse, rat, rabbit, pig, and primates, such as monkey. In the present invention, cytokines include variants of naturally occurring cytokines as long as they retain biological activity. Such variants include, for example, polypeptides with a deletion or addition of one to several amino acid residues (for example, two, three, four, five, or six residues) at the N- or C-terminus, and polypeptides with a substitution of one to several amino acid residues (for example, two, three, four, five, or six residues). The biological activity of a cytokine can be determined by known methods for assaying cytokine activity. Alternatively, the activity can be determined by the method for assaying tumor suppression described herein. Genes encoding variants with a biological activity equivalent to that of a naturally occurring cytokine are expected to exhibit an anti-tumor growth effect equivalent to that of the naturally occurring cytokine. Variants of a naturally occurring cytokine include fragments, analogues, and derivatives of a naturally occurring cytokine, and fusion proteins with other polypeptides (for example, a cytokine having a heterologous signal peptide and a polypeptide fused with an antibody fragment). Specifically, cytokines that are used in the present invention include polypeptides that comprise a sequence with a substitution, deletion, and/or addition of one or more amino acids in the amino acid sequence of a naturally occurring cytokine or fragment thereof, and have a biological activity equivalent to that of the naturally occurring cytokine. The fragment refers to a polypeptide comprising a portion of a naturally occurring cytokine polypeptide, which includes, for example, N- or C-terminal truncated forms. Cytokine fragments with the biological activity typically comprise a continuous region of 70% or more, preferably 80% or more, more preferably 90% or more of a naturally occurring polypeptide (in its mature form after secretion).

Amino acid sequence variants can be prepared, for example, by introducing mutations into DNAs that encode naturally occurring polypeptides (Walker and Gaastra, eds. Techniques in Molecular Biology (MacMillan Publishing Company, New York, 1983); Kunkel Proc. Natl. Acad. Sci. USA 82:488-492, 1985; Kunkel et al. Methods Enzymol. 154:367-382, 1987; Sambrook et al. Molecular Cloning: A Laboratory Manual (Cold Spring Harbor Laboratory Press, Plainview, N.Y, 1989); U.S. Pat. No. 4,873,192). Guidance for substituting amino acids without affecting the polypeptide's biological activity includes, for example, Dayhoff et al. in Atlas of Protein Sequence and Structure (Natl. Biomed. Res. Found., Washington, D.C.) (1978)).

The number of amino acids altered is not specifically limited, but is, for example, 30% or less of the total amino acids in the mature form of a naturally occurring polypeptide, preferably 25% or less, more preferably 20% or less, even more preferably 15% or less, still more preferably 10% or less. It is, for example, 15 amino acids or less, preferably ten amino acids or less, even more preferably eight amino acids or less, still more preferably five amino acids or less, yet more preferably three amino acids or less. In amino acid substitution, substituting amino acids with those that have side chains with similar properties is expected to maintain a protein's original activity. This substitution is referred to as "conservative substitution" in the present invention. Conservative substitutions include substitutions between amino acids within the same group, such as basic amino acids (for example, lysine, arginine, and histidine), acidic amino acids (for example, aspartic acid, and glutamic acid), non-charged polar amino acids (for example, glycine, asparagine, glutamine, serine, threonine, tyrosine, and cysteine), nonpolar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). Conservative substitutions also include, for example, substitutions between amino acids that give a positive score in the BLOSUM62 substitution matrix (S. Henikoff and J.G. Henikoff, Proc. Acad. Natl. Sci. USA 89: 10915-10919, 1992).

Cytokine variants also include polypeptides comprising an amino acid sequence with high homology to the amino acid sequence of a naturally occurring polypeptide. High homology amino acid sequences include those with an identity of, for example, 70% or higher, more preferably 75% or higher, even more preferably 80% or higher, still more preferably 85% or higher, yet more preferably 90% or higher, even still more preferably 93% or higher, yet still more preferably 95% or higher, yet still even more preferably 96% or higher. The amino acid sequence identity can be determined, for example, using the BLASTP program (Altschul, S. F. et al., J. Mol. Biol. 215: 403-410, 1990). For example, search is carried out on the BLAST web page of NCBI (National Center for Biotechnology Information) using default parameters, with all the filters including Low complexity turned off (Altschul, S. F. et al. Nature Genet. 3:266-272, 1993; Madden, T. L. et al. Meth. Enzymol. 266:131-141, 1996; Altschul, S. F. et al. Nucleic Acids Res. 25:3389-3402, 1997; Zhang, J. & Madden, T. L. Genome Res. 7:649-656, 1997). Sequence identity can be determined, for example, by comparing two sequences using the Blast 2 sequences program to prepare an alignment of the two sequences (Tatiana A et al. (1999) FEMS Microbiol Lett. 174:247-250). Gaps are treated in the same way as mismatches. For example, an identity score is calculated in view of the entire amino acid sequence of a naturally occurring cytokine (its mature form after secretion). Specifically, the ratio of the number of identical amino acids to the total number of amino acids in a naturally occurring cytokine (mature form) is calculated.

Preferred variants include polypeptides encoded by nucleic acids that hybridize under stringent conditions with the entire or a portion of the coding region of a naturally occurring cytokine gene, and have a biological activity equivalent to that of the naturally occurring cytokine. When hybridization is used, such a variant can be identified, for example, by preparing a probe either from a nucleic acid that comprises the sequence of the coding region of a naturally occurring cytokine gene or the complementary sequence thereof, or from a nucleic acid to be hybridized, and then detecting whether the probe hybridizes to the other nucleic acid. Stringent hybridization conditions are, for example, hybridization at 60°C, preferably at 65°C, more preferably at 68°C in a solution containing 5x SSC, 7%(W/V) SDS, 100 µg/ml denatured salmon sperm DNA, and 5x Denhardt's solution (1x Denhardt's solution contains 0.2% polyvinylpyrrolidone, 0.2% bovine serum albumin, and 0.2% Ficoll); and washing for two hours at the same temperature as the hybridization while shaking in 2x SSC, preferably 1x SSC, more preferably 0.5x SSC, still more preferably 0.1x SSC.

### (3) Virus vectors

"Negative-strand RNA virus vector" refers to a vehicle that is prepared based on the above-described negative-strand RNA virus and intended for introduction of the above-described cytokine gene into cells. Herein, "infectivity" refers to the capability of a negative-strand RNA viral vector to maintain the cell-adhesion ability toward a cell, and introduce a gene carried by the vector into the inside of the cell to which the vector has adhered. Negative-strand RNA, viral vectors of the present invention may be transmissible vectors or defective non-transmissible vectors. "Transmissible" means that when a viral vector infects a host cell, the virus can replicate within the cell to produce infectious virions.

Herein, "based on a virus" means that the components (proteins, RNAs, and such) of the virus are used in a vector in a non-modified or partially modified manner. For example, a protein and RNA prepared by modifying a paramyxovirus protein and RNA is a protein and RNA "derived from a paramyxovirus", respectively.

In general, a paramyxovirus contains within its envelope a complex (ribonucleoprotein (RNP)) consisting of RNA and proteins. The RNA comprised in the RNP is the genome of paramyxovirus, which is a minus-strand (negative-strand) single-stranded RNA. This single-stranded RNA binds to the NP, P, and L proteins to form an RNP The RNA comprised in the RNP serves as the template for transcription and replication of the viral genome (Lamb, R. A., and D. Kolakofsky, 1996, "Paramyxoviridae: The viruses and their replication" in Fields Virology, 3rd edn. Fields, B. N., D. M. Knipe, and P. M. Howley et al. (ed.), Raven Press, New York.N.Y.pp. 1177-1204, 1996).

"NP, P, M, F, HN, and L genes" of Paramyxovirus refers to genes encoding the nucleocapsid, phospho, matrix, fusion, hemagglutinin-neuraminidase, and large proteins, respectively. The nucleocapsid (NP) protein binds to the genomic RNA and is essential for the template activity of the genomic RNA. In general, the NP gene is occasionally referred to as the "N gene". The phospho (P) protein is a phosphorylated protein, which is a small subunit of RNA polymerase. The matrix (M) protein functions to support the viral particle structure from the inside. The fusion (F) protein is a membrane fusion protein involved in invasion into host cells. The hemagglutinin-neuraminidase (HN) protein is involved in adhesion to host cells. The large (L) protein is a large subunit of RNA polymerase. Each of the genes described above has an independent transcription regulatory unit. Thus, each gene is transcribed into an individual mRNA, which in turn is translated into a protein. The P gene is translated into the P protein, as well as a non-structural protein (C) by using an ORB other than that of the P protein, and another protein (V) produced via RNA editing of the P protein mRNA during translation. In general, respective genes of viruses belonging to the *Paramyxovirinae* family are shown below (starting from the 3' end).
The genus *Respirovirus*: N P/C/V M F HN - L
The genus *Rubulavirus*: N P/V M F HN (SH) L
The genus *Morbillivirus*: N P/C/V M F H - L

The negative-strand RNA virus vectors of the present invention may lack some of the wild-type viral genes. Such viruses can be reconstituted, for example, by exogenously supplying the gene products that are deficient in the viruses. Similar to wild-type viruses, the viruses thus prepared adhere to host cells and cause cell fusion, but cannot form daughter virions that retain the infectivity of the original vectors, since the viral genome introduced into cells is deficient in viral genes. Therefore, such vectors are useful as safe viral vectors that can introduce genes only once. For the preparation of viral gene-deficient vectors, for example, two or more types of vectors which differ in the deficient viral gene on the viral genome carried by the vector are transduced into same cells. The respective deficient viral proteins are supplied through the expression from the other vector(s). Therefore, the vectors complement each other to form infectious viral particles, resulting in a complete replication cycle and amplification of the viral vectors. Specifically, when two or more types of viral vectors of the present invention are inoculated in combination that allows complementation of viral proteins, a mixture of the viral gene-deficient vectors can be produced on a large scale at low cost. Since such viruses lack viral genes, their genome sizes are smaller than those of viral gene-nondeficient viruses and thus can advantageously carry larger foreign genes. Furthermore, these viruses that are non-proliferative due to the lack of viral genes become diluted outside cells, which makes it difficult to maintain their coinfection. The viruses become sterile and thus are advantageous from the viewpoint of controlling their environmental release.

Examples of genes in which the genome may be deficient are the F gene, HN gene, M gene, or any combination thereof. For example, recombinant viruses can be reconstituted by transfecting host cells with a plasmid expressing a recombinant negative-strand RNA viral genome deficient in the F gene, along with an F protein expression vector and expression vectors for the NP, P, and L proteins (WO00/70055, WO00/70070, and WO03/025570; Li, H.-O. et al., J. Virol. 74(14) 6564-6569 (2000)). Viruses can also be produced, for example, using host cells that have incorporated the F gene into their chromosomes. These proteins, which are expressed in virus-producing cells, do not need to have the same amino acid sequences as the viral sequences, and a mutant or homologous gene from another virus may be used as a substitute, as long as the activity in nucleic acid introduction is the same as, or greater than, that of the natural type.

In addition, the viruses of the present invention may be deficient in wild-type virus accessory genes. For example, by knocking out the V gene, which is one of the accessory genes of SeV, the pathogenicity of SeV toward hosts such as mice is markedly reduced without hindering gene expression and replication in cultured cells (Kato, A. et al., J. Virol. 71:7266-7272, 1997; Kato, A. et al., EMBO J. 16:578-587, 1997; Curran, J. et al., WO 01/04272, EP 1067179). Such attenuated vectors are particularly useful as viral vectors for *in vivo* or *ex vivo* gene transfer with lower toxicity.

The NP, P, and L proteins of a *Paramyxoviridae* virus bind to a negative single-stranded RNA, and play an essential role in genomic RNA replication and protein expression (hereinafter, the NP, P, and L proteins are occasionally referred to as "genomic RNA-binding proteins"). The NP protein binds very tightly to the genomic RNA, and thereby confers the genomic RNA with template activity. The genomic RNA has template activity for RNA synthesis only when it binds to the NP protein. The RNA has no template activity when it is free from the NP protein. The P and L proteins bind to the genomic RNA as the small and large subunits of RNA polymerase, respectively. Therefore, in *Paramyxoviridae* viruses, genomic RNA replication cannot occur if any one of the NP, P, and L proteins is defective.

For example, when Sendai virus vectors are used to express foreign genes, these vectors express the viral structural proteins (in particular, the NP, P, and L proteins, which are RNP-constituting proteins essential for transcription and replication) as well as the foreign genes that they carry. Thus, in *in vivo* applications, there is a possibility that immunogenic reaction against these viral structural proteins may be induced. In recent years, various types of gene-deficient SeV vectors have been developed, considering the immunogenicity of the viral structural proteins. For example, there are known vectors that are deficient in one or more of the M, F, and HN proteins, which are not viral RNP-constituting proteins (WO 00/70070; WO 2003/025570). For example, an attenuated negative-strand RNA virus in which the M protein-encoding gene has been deleted or inactivated has in addition to the advantage of attenuation, the advantage of no infection with daughter viruses, since no virion is released due to the loss of production of the M protein, which is essential for the particle formation after viral infection. Furthermore, when the F protein-encoding gene has been deleted or inactivated, a Sendai virus vector becomes a non-transmissible virus, thus securing safety. Moreover, when the HN protein-encoding gene has been deleted or inactivated, a Sendai virus vector has the advantage of not releasing newly formed viral particles from infected cells, since the activity to cleave cell surface sugar chains at sialic acid residues is inhibited. Such gene-deficient viral vectors are also included in the present invention.

In addition, there are also known viral vectors in which temperature-sensitive mutations are introduced into the envelope protein genes (the M, F, and HN genes) encoded by the viral genomic RNA (WO 2003/025570). Such vectors with introduced temperature-sensitive mutations can also be used as a viral vector of the present invention.

NP, P, and L, which are proteins constituting SeV vector RNP, are necessary for transcription and replication of the Sendai virus genome, and thus are normally essential for SeV vectors. However, even when the genome is deficient in these protein genes, theoretically, viral vector particles can be reconstituted and amplified by externally supplying these proteins from expression plasmids or such. Furthermore, when target cells are infected with reconstituted SeV vectors deficient in NP, P, and L, a foreign gene in the genome can be expressed by transcribing mRNAs of the gene using the NP, P, and L proteins carried in the particles. The SeV vectors deficient in NP, P, and L cannot replicate RNP due to lack of the NP, P, and L genes in the genome, and thus are turned into non-replicating SeV vectors. It was previously reported that vectors deficient in either of the NP or P protein gene were constructed to produce non-replicating SeV vectors (Molecular Therapy, 13(Suppl.1), S185, May 2006; NSV2006 (13th International Conference Negative Strand Viruses 2006, Salamance, Spain, June 17-22nd, 2006), Abstract). Furthermore, considering that the L protein itself has RNA polymerase activity, the characteristics of a non-replicating vector are expected to be further assured by deleting the L gene or multiple genes including the L gene, rather than by deleting only the P or NP gene from the genome. In addition, the influence of virus-derived immunogenic proteins is expected to be minimized by deleting multiple genes including the L gene. Thus, deletion of multiple genes including the L gene, for example, all of the NP, P, and L genes, is preferred from the viewpoint of immunogenicity reduction. Furthermore, deletion of the L gene, which occupies about half of the genome length, is expected to enable the genome to carry a larger gene of interest. Moreover, higher transcription efficiency and expression level of a gene of interest can be expected with a small genome size. Thus, L gene-deficient SeV vectors are considered to be very useful. Based on the above concept, the applicants have constructed non-replicative *Paramyxoviridae* viral vectors, and filed a patent application related to the vectors (Japanese Patent Application No. 2006-193433). The vectors of the present invention may be vectors in which the NP, P, and L genes have been completely or partially deleted.

If the NP, P, and L genes have been completely or partially deleted from a vector, the NP, P, and L proteins can be incorporated into the vector by expressing these proteins in cells at the time of intracellular vector reconstitution. In this manner, viral vectors that maintain infectivity can be prepared. Once cells are infected with such vectors, proteins can be expressed from the genomic RNA by intracellular RNPs. However, since the vectors themselves do not have the L gene or such, they cannot reproduce viruses that have the replication ability of the original viruses. Such vectors are very useful for gene therapy and such, in particular, for treatment of target diseases that requires the influence of vectors to be minimized, and for other applications.

The nucleotide sequences of the genes of various viruses including Sendai virus are known. Such known information can be used to produce the vectors of the present invention.

The database accession numbers for the nucleotide sequences of the Sendai virus genes are shown below: M29343, M30202, M30203, M30204, M51331, M55565, M69046, and X17218 (the N gene); M30202, M30203, M30204, M55565, M69046, X00583, X17007, and X17008 (the P gene); D11446, K02742, M30202, M30203, M30204, M69046, U31956, X00584, and X53056 (the M gene); D00152, D11446, D17334, D17335, M30202, M30203, M30204, M69046, X00152, and X02131 (the F gene); D26475, M12397, M30202, M30203, M30204, M69046, X00586, X02808, andX56131 (the HN gene); and D00053, M30202, M30203, M30204, M69040, X00587, and X58886 (the L gene).

Examples of viral genes encoded by other viruses are CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for the N gene; CDV, X51869; DMV, Z47758; SPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for the P gene; CDV, AF014953; DMV, Z47758; HPIV-1. M74081; HPIV-3, D00047; MV, ABO16162 RPV X68311; SeV, AB005796; and Tupaia, AF079780 for the C gene; CDV, M12669; DMV Z30087; HPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for the M gene; CDV, M21849; DMV, AJ224704; HPN-1, M22347; HPIV-2, M60182; HPIV-3, X05303, HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for the F gene; CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, X74443; PDV, Z36979; RPV, AF132934; SeV, U06433; and SV-5, S76876 for the HN (H or G) gene; and CDV, AF014953; DMV, AJ608288; HPIV-1, AF117818; HPIV-2, X57559; HPIV-3, AB012132; Mumps, AB040874; MV, K01711; NDV, AY049766; PDPR, AJ849636; PDV, Y09630; RPV, Z30698; and SV-5, D13868 for the L gene.

Multiple strains are known for each virus, and depending on the strain, there are genes comprised of sequences other than the examples listed above.

Genes encoded by the viral genome RNA to be used as a vector of the present invention may have the original viral gene sequences, or may be deleted or inactivated as described above. Alternatively, a mutation(s) may be introduced into the genes. For example, those skilled in the art can use known methods to introduce into genes in the genomic RNA, mild mutations that do not impair the function of the proteins. For example, site-specific mutations can be introduced by PCR, cassette mutagenesis, or such. Alternatively, random mutations can be introduced by using chemical reagents, random nucleotides, or such.

For example, It is known that the cytotoxicity of a paramyxovirus vector can be reduced by introducing mutations into the P gene. When the P gene is encoded by genomic RNA of the vector of the present invention, such mutations may be introduced into the RNA gene of the genome. Specifically, the mutations include, for example, substitution of a different amino acid for Leu at position 511 (L511) of the SeV P protein, or substitution at the homologous position of the P protein in other negative-strand RNA viruses. The amino acid mutations may be desirable substitutions by a different amino acid, and preferably by amino acids whose side chains have different chemical properties. Amino acids can be categorized, for example, into groups such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). The amino acid substitutions include substitution of an amino acid by another amino acid different those in the group to which the original amino acid belongs. Specifically, the substitutions include substitution of a basic amino acid by an acidic or neutral amino acid; substitution of a polar amino acid by an non-polar amino acid; substitution of an amino acid whose molecular weight is higher than the average molecular weight of the 20 types of natural amino acids by an amino acid whose molecular weight is lower than the average molecular weight; and substitution of an amino acid whose molecular weight is lower than the average molecular weight by an amino acid whose molecular weight is higher than the average molecular weight; however, the substitutions are not limited to those described above. Specifically, the substitutions include, for examples, the substitution of L511 by Phe (L511F).

Genes carried by a negative-strand RNA viral vector are encoded antisense in the viral genomic RNA. "Viral genome RNA" refers to an RNA that has the function to form a ribonucleoprotein (RNP) with the viral proteins of a negative-strand RNA virus. The genes in the genome are expressed by the ribonucleoprotein, and the genomic RNA is replicated to form daughter RNPs. In general, the genome of a negative-strand RNA virus is constituted so that the viral genes are situated in the antisense orientation between the 3'-leader region and 5'-trailer region. A transcription termination sequence (E sequence) - intervening sequence (I sequence) - transcription initiation sequence (S sequence) exists between the ORFs of individual genes, and allows the RNA encoding the ORF of each gene to be transcribed as a separate cistron.

ORFs encoding the viral proteins contained in a vector, and ORFs of other foreign genes are arranged in the antisense direction in the genomic RNA via the above-described E-I-S sequence. The ORF clOsest to the 3'-end of the genomic RNA requires only an S sequence between the 3'-leader region and the ORF, and does not require an E or I sequence. Furthermore, the ORF closest to the 5'-end of the genomic RNA requires only an E sequence between the 5'-trailer region and the ORF, and does not require an I or S sequence. Furthermore, two ORFs can be transcribed as a single cistron, for example, by using an internal ribosome entry site (IRES) sequence. In such a case, an E-I-S sequence is not required between these two ORFs. For example, in wild type paramyxoviruses, a typical RNA genome includes a 3'-leader region, six ORFs encoding the N, P, M, F, HN, and L proteins in the antisense direction in this order, and a 5'-trailer region on the other end. The orientation of the viral gene in the genomic RNAs of the present invention is not restricted. For example, similarly to the wild-type viruses, ORFs encoding the N, P, M, F, HN, and L proteins can be arranged after the 3'-leader region and before the 5'-trailer region. Certain types of viruses have different viral genes, but even in such cases, it is possible to arrange each gene as in the wild type, as described above. In general, vectors maintaining the N, P, and L genes can autonomously express genes from the RNA genome in cells and the genomic RNA is replicated, Furthermore, by the action of genes such as the F and HN genes which encode envelope proteins and the M gene, infectious virions are formed and released to the outside of the cells. Thus, such vectors become transmissible viral vectors. A cytokine gene to be carried by the vectors may be inserted into a non-protein-coding region in this genome, as described below.

Viral vectors of the present invention encode cytokine genes in their genomic RNA. A recombinant viral vector harboring a cytokine gene is obtained by inserting a cytokine gene into an above-described viral vector genome. The cytokine gene can be inserted at any desired position in a non-protein-coding region of the virus genome, for example. The above nucleic acid can be inserted, for example, between the 3'-leader region and the viral protein ORF closest to the 3'-end; between each of the viral protein ORFs; and/or between the viral protein ORF closest to the 5'-end and the 5'-trailer region in genomic DNA. Furthermore, in genomes deficient in the F or HN gene or such, nucleic acids encoding the cytokine genes can be inserted into those deficient regions. When introducing a foreign gene into a paramyxovirus, it is desirable to insert the gene such that the chain length of the polynucleotide to be inserted into the genome will be a multiple of six (Journal of Virology, Vol. 67, No. 8, 4822-4830, 1993). An E-I-S sequence should be arranged between the inserted cytokine gene and the viral ORF. Two or more foreign genes can be inserted in tandem via E-I-S sequences.

Expression levels of a foreign gene carried in a vector can be controlled using the type of transcriptional initiation sequence added upstream (to the 3'-side of the minus strand (negative strand)) of the gene (WO01/18223). The expression levels can also be controlled by the position at which the foreign gene is inserted in the genome: the nearer to the 3'-end of the minus strand the insertion position is, the higher the expression level; while the nearer to the 5'-end the insertion position is, the lower the expression level. Thus, to obtain a desired gene expression level, the insertion position of a foreign gene can be appropriately controlled such that the combination with genes encoding the viral proteins before and after the foreign gene is most suitable. In general, since a high foreign gene expression level is thought to be advantageous, it is preferable to link the foreign gene to a highly efficient transcriptional initiation sequence, and to insert it near the 3'-end of the minus strand genome. Specifically, a foreign gene is inserted between the 3'-leader region and the viral protein ORF closest to the 3'-end. Alternatively, a foreign gene may be inserted between the ORFs of the viral protein gene closest to the 3'-end and the second closest viral protein gene, or between the ORFs of the second and third closest viral protein genes. In wild type paramyxoviruses, the viral protein gene closest to the 3'-end of the genome is the N gene, the second closest gene is the P gene, and the third closest gene is M gene. Alternatively, when a high level of expression of the introduced gene is undesirable, the gene expression level from the viral vector can be suppressed to obtain an appropriate effect, for example, by inserting the foreign gene at a site as close as possible to the 5'-side of the minus strand genome, or by selecting an inefficient transcriptional initiation sequence.

For example, a desired S sequence of a negative-strand RNA virus may be used as the S sequence to be attached when inserting a foreign gene-encoding nucleic acid into the genome. The sequence 3'-UCCCMVUUMC-5' (M= A or C; V= A, C, or G)(SEQ ID NO: 1) can be preferably used for Sendai viruses. Particularly preferred sequences are 3'-UCCCAGUUUC-5' (SEQ ID NO: 2), 3'-UCCCACUUAC-5' (SEQ ID NO: 3), and 3'-UCCCACUUUC-5' (SEQ ID NO: 4). When shown as plus strand-encoding DNA sequences, these sequences are 5'-AGGGTCAAAG-3' (SEQ ID NO: 5), 5'-AGGGTGAATG-3' (SEQ ID NO: 6), and 5'-AGGGTGAAAG-3' (SEQ ID NO: 7). A preferred E sequence of a Sendai viral vector is, for example, 3'-AUUCUUUUU-5' (SEQ ID NO: 8) or 5'-TAAGAAAAA-3' (SEQ ID NO: 9) for the plus strand-encoding DNA. An I sequence may be, for example, any three nucleotides, specifically 3'-GAA-5' (5'-CTT-3' in the plus strand DNA).

Negative-strand RNA viruses of the present invention may be, for example, complexes of negative-strand RNA viral genomic RNAs and viral proteins, that is, ribonucleoproteins (RNPs). RNPs can be introduced into cells, for example, in combination with desired transfection reagents. Specifically, such RNPs are complexes comprising a negative-strand RNA viral genomic RNA, N protein, P protein, and L protein. On introducing an RNP into cells, cistrons encoding the viral proteins are transcribed from the genomic RNA by the action of viral proteins, and, at the same time, the genome itself is replicated to form daughter RNPs. Replication of a genomic RNA can be confirmed by using RT-PCR, Northern blot hybridization, or such to detect an increase in the copy number of the RNA.

The vectors of the present invention may be of any type as long as they have the above-mentioned characteristics, and include a viral vector that has a viral particle structure, an RNP vector which is itself RNP, and such.

Furthermore, recombinant viruses that include an envelope protein other than that of the virus from which the viral genome was derived, may be prepared as viral vectors used in the present invention. For example, when reconstituting a virus, a recombinant virus including a desired envelope protein can be generated by expressing in a cell an envelope protein other than the envelope protein originally encoded by the basic viral genome. Such proteins are not particularly limited. A desired protein that confers an ability to infect cells may be used. Examples of such proteins include the envelope proteins of other viruses, for example, the G protein of vesicular stomatitis virus (VSV-G). The VSV-G protein may be derived from an arbitrary VSV strain. For example, VSV-G proteins derived from Indiana serotype strains (J. Virology 39: 519-528 (1981)) may be used, but the present invention is not limited thereto. Furthermore, the present vector may include any arbitrary combination of envelope proteins derived from other viruses. Preferred examples of such proteins are envelope proteins derived from viruses that infect human cells. Such proteins are not particularly limited, and include retroviral amphotropic envelope proteins and such. For example, the envelope proteins derived from mouse leukemia virus (MuLV) 4070A strain can be used as the retroviral amphotropic envelope proteins. In addition, envelope proteins derived from MuMLV 10A1 strain may also be used (for example, pCL-10A1 (Imgenex) (Naviaux, R. K. et al., J. Virol. 70:5701-5705 (1996)). The proteins of *Herpesviridae* include, for example, gB, gD, gH, and gp85 proteins of herpes simplex viruses, and gp350 and gp220 proteins of EB virus. The proteins of *Hepadnaviridae* include the S protein of hepatitis B virus. These proteins may be used as fusion proteins in which the extracellular domain is linked to the intracellular domain of the F or HN protein. As described above, the viral vectors of the present invention include pseudotype viral vectors comprising envelope proteins such as VSV-G, which are derived from viruses other than the virus from which the genome was derived. If the viral genomic RNAs are designed in a way that these envelope proteins are not encoded by the genome, the proteins will not be expressed from the viral vectors after virions infect the cells.

Furthermore, the viral vectors used in the present invention may be, for example, vectors that comprise on their envelope surface, proteins such as adhesion factors capable of adhering to specific cells, ligands, receptors, antibodies or fragments thereof, or chimeric proteins that have the above proteins in the extracellular domain and polypeptides derived from the viral envelope in the intracellular domain. This enables to control the specificity of viral vector infection. These proteins may be encoded in the viral genome, or supplied by expression of genes other than those in the viral genome (for example, genes in other expression vectors, the host chromosome, or such) at the time of viral reconstitution.

To facilitate the insertion of a foreign gene, a cloning site may be designed at the position of insertion. The cloning site can be made into, for example, a recognition sequence for restriction enzyme. Foreign gene fragments can be inserted into the restriction enzyme site in the vector DNA encoding the genome. Cloning site may be arranged to be a so-called multi-cloning site comprising a plurality of restriction enzyme recognition sequences. Vectors of the present invention may thus harbor additional foreign genes. As necessary, the vectors of the present invention may carry a foreign gene different from the above-mentioned cytokine and other genes.

### (4) Method of vector preparation

To prepare a negative-strand RNA viral vector, a cDNA encoding a genomic RNA of a negative-strand RNA virus is transcribed in mammalian cells, in the presence of viral proteins (i.e., the N, P, and L proteins) essential for reconstitution of an RNP including the genomic RNA of the negative-strand RNA virus. Viral RNP can be reconstituted by producing either the negative-strand genome (the same antisense strand as the viral genome) or the plus strand (antigenome, the complementary strand of the genomic RNA). For increasing the efficiency of vector reconstitution, it is more preferable to produce the plus strand. The RNA terminals preferably reflect the terminals of the 3'-leader sequence and 5'-trailer sequence as accurately as possible, as in the natural viral genome. To accurately regulate the 5'-end of the transcript, for example, the recognition sequence of T7 RNA polymerase may be used as a transcription initiation site and the RNA polymerase may be expressed within a cell. To regulate the 3'-end of the transcript, for example, a self-cleaving ribozyme can be encoded at the 3'-end of the transcript, allowing accurate cleavage of the 3'-end with this ribozyme (Hasan, M. K. et al., J. Gen. Virol. 78: 2813-2820,1997; Kato, A. et al., EMBO J. 16: 578-587, 1997; and Yu, D. et al., Genes Cells 2: 457-466, 1997). An auto-cleaving ribozyme derived from the antigenomic strand of delta hepatitis virus can be used.

Sendai virus-based viral vectors of the present invention that carry a cytokine gene can be constructed, for example, by the following procedure according to the method described in documents such as Kato, A. et al., EMBO J. 16: 578-587, 1997; and Yu, D. et al., Genes Cells 2: 457-466, 1997.

First, a DNA sample comprising the cDNA nucleotide sequence of a desired cytokine gene is prepared. It is preferable that the DNA sample is at a concentration of 25 ng/µl or more and can be electrophoretically identified as a single plasmid. Hereinbelow, a case where a cytokine gene is inserted to a DNA encoding a viral genome utilizing the *Nat*I site will be described as an example. When the *Not*I recognition site is included in the cDNA nucleotide sequence of interest, it is preferable to delete the *Not*I site beforehand by modifying the nucleotide sequence using site-specific mutagenesis and such, so as not to alter the amino acid sequence encoded by the cDNA. From this DNA sample, the desired gene fragment is amplified and recovered by PCR. To have *Not*I sites on both ends of the amplified DNA fragment, and further add a copy of the transcription termination sequence (E), intervening sequence (I) and transcription initiation sequence (S) (EIS sequence) of Sendai virus to one end, a forward synthetic DNA sequence (sense strand) and reverse synthetic DNA sequence (antisense strand) are prepared as a pair of primers containing the *Not*I restriction enzyme cleavage site sequence, the transcription termination sequence (E), intervening sequence (I), and transcription initiation sequence (S), and a partial sequence of the gene of interest.

For example, to secure cleavage by *Not*I*,* the forward synthetic DNA sequence is arranged in a form, in which any two or more nucleotides (preferably four nucleotides excluding GCG and GCC, which are sequences originating in the *Not*I recognition site, more preferably ACTT) are selected on the 5'-side of the synthetic DNA, the *Not*I recognition site "gcggccgc" is added to its 3'-side, and to the 3'-side thereof, any nine nucleotides or nucleotides of nine plus a multiple of six nucleotides are added as the spacer sequence, and to the 3'-side thereof, a sequence of about 25 nucleotides from the ORF including the initiation codon ATG of the desired cDNA is added. It is preferable to select about 25 nucleotides from the desired cDNA as the forward side synthetic DNA sequence so that it has G or C as the final nucleotide on its 3'-end.

In the reverse synthetic DNA sequence, any two or more nucleotides (preferably four nucleotides excluding GCG and GCC, which are sequences originating in the *Not*I recognition site, more preferably ACTT) are selected from the 5'-side of the synthetic DNA, and the *Not*I recognition site "gcggccgc" is added to its 3'-side. Further to its 3'-side, an oligo DNA is added as an insertion fragment to adjust the length. The number of nucleotides in the oligo DNA is determined so that the total nucleotide number of the Sendai virus genome becomes a multiple of six (so-called "rule of six"; Kolakofski, D. et al., J. Virol. 72: 891-899, 1998). Further to the 3'-side of inserted fragment, sequences complementary to the S sequence of Sendai virus, preferably 5'-CTTTCACCCT-3' (SEQ ID NO: 10), the I sequence, preferably 5'-AAG-3', and the E sequence, preferably 5'-TTTTTCTTACTACGG-3' (SEQ ID NO: 11), are added. Further to the 3'-side, a complementary sequence of about 25 nucleotides counted in the reverse direction from the termination codon of the desired cDNA sequenceis selected and added as the 3'-end of the reverse synthetic DNA, wherein the length of the complementary sequence is adjusted so that the final nucleotide is G or C.

PCR can be performed according to conventional methods with, for example, ExTaq polymerase (Takara Shuzo). Preferably, PCR is performed using Vent polymerase (NEB), and the fragments of interest thus amplified are digested with *Not*I*,* then inserted to the *Nat*I site of the plasmid vector pBluescript. Nucleotide sequences of the PCR products thus obtained are confirmed with a sequencer to select a plasmid having the correct sequence. The inserted fragment is excised from the plasmid using *Not*I*,* and cloned to the *Not*I site of a plasmid carrying the genomic cDNA deficient in envelope genes. Alternatively, it is also possible to obtain a recombinant Sendai virus cDNA by directly inserting the fragment to the *Not*I site without mediation of the plasmid vector pBluescript.

The viral vector cDNA of the present invention is transcribed *in vitro* or in cells to produce RNA, which is as necessary allowed to bind to separately expressed L, P, and NP proteins to reconstitute an RNP. Thus, viral vectors comprising the RNP can be produced. According to known methods (WO 97/16539; WO 97/16538; Durbin, A.P. et al., Virology 235: 323-332, 1997; Whelan, S. P. et al., Proc. Natl. Acad. Sci. USA 92: 8388-8392, 1995; Schnell. M. J. et al., EMBO J. 13: 4195-4203, 1994; Radecke, F. et al., EMBO J. 14: 5773-5784, 1995; Lawson, N. D. et al., Proc. Natl. Acad. Sci. USA 92: 4477-4481, 1995; Garcin, D. et al., EMBO J. 14: 6087-6094, 1995; Kato, A. et al., Genes Cells 1: 569-579, 1996; Baron, M.D. and Barrett, T., J. Virol. 71: 1265-1271, 1997; Bridgen, A. and Elliott, R.M., Proc. Natl. Acad. Sci. USA 93: 15400-15404, 1996), viral particles can be reconstituted from a viral vector cDNA encoding a negative single-stranded RNA, which has been modified so as not to express some of the virus-derived proteins, or the complementary strand thereof. In particular, improved methods are effective (WO 2005/071092).

Genes that have been deleted from the viral vector cDNA of the present invention can be expressed by introducing them into host cells separately. When viral particles are reconstituted by forming RNPs and expressing envelope proteins using genes encoded by the viral vector cDNA of the present invention or separate expression vectors, the viral particles can be infectious. Alternatively, each gene may be integrated into the host cell chromosome to complement the gene deficiency. Genes expressed to form RNPs, such as the NP, P, and L genes, are not necessarily completely identical to those encoded in the virus genome on which the vector is based. The amino acid sequences of the proteins encoded by these genes are not necessarily identical to those of the proteins encoded by the RNP genome. Mutations may be introduced into the proteins, or homologous genes of other viruses may be used as substitutes, as long as the proteins bind to the genomic RNA and have the activity of transcribing and replicating the genome in cells.

When obtained in the form of RNP or virion, the viral vectors of the present invention can be amplified by introducing the vectors into cells that allow viral propagation (in the case of viral vectors in which the genes essential for viral expression and propagation have been deleted, cells that express the genes deficient in the viral vectors); culturing the cells; and collecting virions from the culture supernatant. As necessary, the cells into which the vectors are introduced may express proteins that have the function to promote formation and release of virions, for example, the C protein. High viral vector productivity can be achieved by expressing the C protein. The Sendai virus C protein includes four types of proteins, C', C, Y1, and Y2, which are translated using different initiation codons in the same reading frame. Any one, or two or more, or all of the four types of C proteins described above may be expressed in helper cells at the time of viral vector reconstitution. Deletion of the P gene from the genomic RNA of the viral vectors of the present invention results in deletion of the C gene which is in a reading frame different from that of the P gene. When the genomic RNA lacks the C gene, the C gene can be expressed in cells by introducing an expression vector that encodes the C gene into the cells.

As long as a virus can be reconstituted, the host cells used in the reconstitution are not particularly limited. For example, in the reconstitution of Sendai virus vectors and such, it is possible to use cultured cells such as LLC-MK2 cells and CV-1 cells derived from monkey kidney, BHK cells derived from hamster kidney, and cells derived from humans. By expressing suitable envelope proteins in these cells, infectious virions including the proteins in the envelope can also be obtained. Further, to obtain a large quantity of a Sendai virus vector, a viral vector obtained from an above-described host can be used to infect embrionated hen eggs to amplify the vector. Methods for manufacturing viral vectors using hen eggs have already been developed (Nakanishi, et al., ed. (1993), "State-of-the-Art Technology Protocol in Neuroscience Research III, Molecular Neuron Physiology", Koseisha, Osaka, pp. 153-172). Specifically, for example, a fertilized egg is placed in an incubator, and cultured for nine to twelve days at 37 to 38°C to grow an embryo. After the viral vector is inoculated into the allantoic cavity, the egg is cultured for several days (for example, three days) to proliferate the viral vector. Conditions such as the period of culture may vary depending upon the recombinant Sendai virus being used. Then, allantoic fluids including the vector are recovered. Separation and purification of a Sendai virus vector from allantoic fluids can be performed according to a usual method (Tashiro, M., "Virus Experiment Protocol," Nagai, Ishihama, ed., Medical View Co., Ltd., pp. 68-73, (1995)).

RNP may be introduced into cells as a complex formed together with, for example, lipofectamine or polycationic liposomes. Specifically, it is possible to use a variety of transfection reagents. Such transfection reagents include, for example, DOTMA (Boehringer), Superfect (QIAGEN #301305), DOTAP, DOPE, DOSPER (Boehringer #1811169), and Lipofectamine 2000 (Invitrogen). Chloroquine may be added to prevent decomposition in endosomes (Calos, M.P., 1983, Proc. Natl. Acad. Sci. USA 80: 3015).

Major methods for introducing viral vector cDNAs into cells include those described below. (i) methods of preparing DNA precipitates that can be internalized into the cells of interest; (ii) methods of preparing positively-charged complexes comprising DNAs that are suitable for internalization into the cells of interest, and have low cytotoxicity; and (iii) methods of momentarily creating holes with a size sufficient for DNA molecules to pass through on the membrane of cells of interest using electric pulses.

The methods of (i) include, for example, transfection methods using calcium phosphate. It is known that while DNA incorporated into cells by these methods is internalized into phagosomes, a sufficient amount of the DNA also enter the nucleus (Graham, F. L. and Van Der Eb, J., Virology 52: 456, 1973; Wigler, M. and Silverstein, S., Cell 11: 223, 1977). Chen and Okayama studied the optimization of transfer techniques, and reported that (1) incubation conditions for cells and co-precipitates are: 2 to 4% CO₂ at 35°C for 15 to 24 hours; (2) the activity of circular DNAs is higher than that of linear DNAs; and (3) optimal precipitate can be obtained when the DNA concentration of precipitation mixture is 20 to 30 µg/ml (Chen, C. and Okayama, H., Mol. Cell. Biol. 7: 2745, 1987).

Various transfection reagents can be used in the methods of (ii). Such reagents include, for example, DOTMA (Boehringer), Superfect (QIAGEN #301305), DOTAP, DOPE, DOSPER (Boehringer #1811169), and Lipofectamine 2000 (Invitrogen). The methods of (ii) are suitable for transient transfection. Methods for performing transfection by preparing a DEAE-dextran (Sigma #D-9885 M.W 5 x 10⁵) mixture with a desired DNA concentration ratio have been known for a long time. Since most complexes are degraded in endosomes, chloroquine may be added to enhance the effect (Calos, M.P., Proc. Natl. Acad. Sci. USA 80: 3015, 1983).

The methods of (iii) are referred to as electroporation methods, which are not cell-selective, and thus have high versatility compared to the methods of (i) and (ii). The efficiency of these methods is assumed to be high under optimal conditions for the duration of pulse electric current, shape of the pulse, potency of electric field (gap between electrodes, voltage), buffer conductivity, DNA concentration, and cell density.

Of the above three categories, the methods of (ii) are simpler to perform and can be used to examine many samples with a large number of cells. Transfection reagents that can be suitably used in the present invention include Lipofectamine 2000 (Invitrogen), Superfect Transfection Reagent (QIAGEN, Cat No. 301305), and DOSPER Liposomal Transfection Reagent (Boehringer Mannheim, Cat No. 1811169).

Specifically, virus reconstitution from cDNA can be carried out, for example, as follows: In a plastic plate of about 6 to 24 wells, or a 100-mm Petri dish or such, simian kidney-derived LLC-MK2 cells (ATCC CCL-7) are cultured up to about 100% confluency, using minimum essential medium (MEM) containing 10% fetal calf serum (FCS) and antibiotics (100 units/ml penicillin G and 100 µg/ml streptomycin). Then they are infected with, for example, two plaque forming units (PFU)/cell of the recombinant vaccinia virus vTF7-3, which expresses T7 RNA polymerase and has been inactivated by 20-minutes of UV irradiation in the presence of 1 µg/ml psoralen (Fuerst, T. R. et al., Proc. Natl. Acad. Sci. USA 83: 8122-8126,1986; Kato, A. et al., Genes Cells 1: 569-579, 1996). The amount of psoralen added and the UV irradiation time can be appropriately adjusted. One hour after infection, 2 to 60 µg, and more preferably 3 to 20 µg, of DNA encoding the genomic RNA of a recombinant Sendai virus is transfected along with the plasmids expressing trans-acting viral proteins essential for viral RNP generation (0.5 to 24 µg of pGEM-N, 0.25 to 12 µg of pGEM-P, and 0.5 to 24 µg of pGEM-L) (Kato, A. et al., Genes Cells 1: 569-579, 1996), using the lipofection method or such with Superfect (QIAGEN). For example, the ratio of the amounts of expression vectors encoding the N, P, and L proteins is preferably 2:1:2, and the plasmid amounts are appropriately adjusted in the range of 1 to 4 µg of pGEM-N, 0.5 to 2 µg of pGEM-P, and 1 to 4 µg of pGEM-L.

The transfected cells are cultured, as desired, in serum-free MEM supplemented with 100 µg/ml of rifampicin (Sigma) and cytosine arabinoside (AraC), more preferably only 40 µg/ml of cytosine arabinoside (AraC) (Sigma). Optimal drug concentrations are set so as to minimize cytotoxicity due to the vaccinia virus, and to maximize virus recovery rate (Kato, A. et al., 1996, Genes Cells 1: 569-579). After culturing for about 48 to 72 hours after transfection, cells are harvested, and then disrupted by repeating freeze-thawing three times. LLC-MK2 cells are re-transfected with the disrupted materials containing RNP, and cultured. Alternatively, the culture supernatant is recovered, added to a culture medium of LLC-MK2 cells to infect them, and the cells are then cultured. Transfection can be conducted by, for example, forming a complex with lipofectamine, polycationic liposome, or such, and transducing the complex into cells. Specifically, various transfection reagents can be used. For example, DOTMA (Roche), Superfect^{™} (QIAGEN #301305), DOTAP, DOPE, and DOSPER (Roche #1811169) may be cited. In order to prevent degradation in the endosome, chloroquine may also be added (Calos, M. P., 1983, Proc. Natl. Acad. Sci. USA 80: 3015). In cells transduced with RNP, viral gene expression from RNP and RNP replication progress, and the virus is amplified. By diluting the viral solution (culture supernatant) thus obtained (for example, 10⁶-fold), and then repeating the amplification, the vaccinia virus vTF7-3 can be completely eliminated. Amplification is repeated, for example, three or more times. Vectors thus obtained can be stored at -80°C. In order to reconstitute a nontransmissible virus lacking a gene encoding an envelope protein, LLC-MK2 cells expressing the envelope protein may be used for transfection, or a plasmid expressing the envelope protein may be cotransfected. Alternatively, an envelope-gene defective type virus can be amplified by culturing the transfected cells overlaid with LLK-MK2 cells expressing the envelope protein (see WO00/70055 and WO00/70070).

Titers of collected virus vectors can be determined, for example, by measuring CIU (Cell Infectious Unit) or Hemagglutination Activity (HA) (WO 00/70070; Kato, A. et al., Genes Cells 1: 569-579,1996; Yonemitsu, Y. & Kaneda, Y., Hemagglutinating virus of Japan-liposome-mediated gene delivery to vascular cells. Ed. by Baker AH. Molecular Biology of Vascular Diseases. Method in Molecular Medicine: Humana Press: pp. 295-306, 1999). Alternatively, titers of vectors carrying marker genes such as GFP (green fluorescent protein) can be quantified by directly counting infected cells, using the marker as an index (for example, as GFP-CIU). Titers thus determined can be treated in the same way as CIU (WO 00/70070).

The collected virus vectors can be purified to be substantially pure. Purification can be achieved using known purification/separation methods including filtration, centrifugation, column purification, and such, or any combination thereof. "Substantially pure" means that the viral vector is a component that accounts for the major proportion of a sample containing the vector. Typically, a viral vector can be confirmed to be substantially pure when the percentage of the viral vector-derived proteins relative to the total proteins (excluding proteins added as carriers, stabilizers, and such) in a sample is 10% or more, preferably 20% or more, more preferably 50% or more, preferably 70% or more, more preferably 80% or more, and even more preferably 90% or more. Specific methods for purifying paramyxoviruses include, for example, methods using cellulose sulfate esters or cross-linked polysaccharide sulfate esters (Japanese Patent Application Kokoku Publication No. (JP-B) S62-30752 (examined, approved Japanese patent application published for opposition), JP-B S62-33879, and JP-B S62-30753), and methods of adsorbing viruses to fucose sulfate-containing polysaccharides and/or degradation products thereof (WO97/32010).

Specifically, a method for construction and preparation of Sendai virus vectors defective in F gene is described in detail below for reference (see WO00/70055 and WO00/70070).

### <1> Construction of a genomic cDNA of an F-gene deficient Sendai virus, and a plasmid expressing F gene:

A full-length genomic cDNA of Sendai virus (SeV), the cDNA of pSeV18⁺ b (+) (Hasan, M. K. et al., J. General Virology 78: 2813-2820, 1997) ("pSeV18⁺ b (+)" is also referred to as "'pSeV18⁺"), is digested with *Sph*I/*Kpn*I to recover a fragment (14673 bp), which is cloned into pUC18 to prepare plasmid pUC18/KS. Construction of an F gene-deficient site is performed on this pUC18/KS. An F gene deficiency is created by a combination of PCR-ligation methods, and, as a result, the F gene ORF (ATG-TGA = 1698 bp) is removed. Then, for example, 'atgcatgccggcagatga (SEQ ID NO: 12)' is ligated to construct an F gene-deficient type SeV genomic cDNA (pSeV18⁺/ΔF). A PCR product formed in PCR by using the pair of primers [forward: 5'-gttgagtactgcaagagc/SEQ ID NO: 13, reverse:
5'-tttgccggcatgcatgtttcccaaggggagagttttgcaacc/SEQ ID NO: 14] is connected upstream of F, and a PCR product formed using the pair of primers [forward: 5'-atgcatgccggcagatga/SEQ ID NO: 15, reverse: 5'-tgggtgaatgagagaatcagc/SEQ ID NO: 16] is connected downstream of the F gene with *Eco*T22I. The plasmid thus obtained is digested with *Sac*I and *Sal*I to recover a 4931 bp fragment of the region including the F gene-deficient site, which is cloned into pUC18 to form pUC18/dFSS. This pUC18/dFSS is digested with *Dra*III*,* the fragment is recovered, replaced with the *Dra*III fragment of the region comprising the F gene of pSeV18⁺, and ligated to obtain the plasmid pSeV18⁺/ΔF. A foreign gene is inserted, for example, into the *Nsi* I and *Ngo* MIV restriction enzyme sites in the F gene-deficient site of pUC18/dFSS. For this, a foreign gene fragment may be, for example, amplified using an *Nsi* I-tailed primer and an *Ngo* MIV-tailed primer.

### <2> Preparation of helper cells that induce SeV-F protein expression:

To construct an expression plasmid of the Cre/loxP induction type that expresses the Sendai virus F gene (SeV-F), the SeV-F gene is amplified by PCR, and inserted to the unique *Swa* I site of the plasmid pCALNdlw (Arai, T. et al., J. Virology 72, p.1115-1121, 1998), which is designed to enable the inducible expression of a gene product by Cre DNA recombinase, thus constructing the plasmid pCALNdLw/F. To recover infectious virions from the F gene-deficient genome, a helper cell line expressing SeV-F protein is established. The monkey kidney-derived LLC-MK2 cell line, which is commonly used for SeV proliferation, can be used as the cells, for example. LLC-MK2 cells are cultured in MEM supplemented with 10% heat-inactivated fetal bovine serum (FBS), penicillin G sodium (50 units/ml), and streptomycin (50 µg/ml) at 37°C in 5% CO₂. Since the SeV-F gene product is cytotoxic, the above-described plasmid pCALNdLw/F, which was designed to enable inducible expression of the F gene product with Cre DNA recombinase, is transfected to LLC-MK2 cells by the calcium phosphate method (using a mammalian transfection kit (Stratagene)), according to protocols well known in the art. The plasmid pCALNdLw/F (10 µg) is transduced into LLC-MK2 cells grown to 40% confluency using a 10-cm plate, and the cells are then cultured in MEM (10 ml) containing 10% FBS, in a 5% CO₂ incubator at 37°C for 24 hours. After 24 hours, the cells are detached and suspended in the medium (10 ml). The suspension is then seeded into five 10-cm dishes, 5 ml into one dish, 2 ml each into two dishes, and 0.2 ml each into two dishes, and cultured in MEM (10 ml) containing G418 (GIBCO-BRL) (1200 µg/ml) and 10% FBS. The cells are cultured for 14 days, exchanging the medium every two days, to select cell lines stably transduced with the gene. The cells grown in the above medium that show G418 resistance are recovered using cloning rings. Culture of each clone thus recovered is continued in 10-cm plates until confluent. After the cells have grown to confluency in a 6-cm dish, F protein expression can be induced by infecting the cells with adenovirus AxCANCre, for example, at MOI = 3, according to the method of Saito, *et al.* (Saito et al., Nucl. Acids Res. 23: 3816-3821,1995; Arai, T. et al., J. Virol 72, 1115-1121 1998).

### <3> Reconstitution and amplification ofF gene-deficient SeV virus:

The above-described plasmid pSeV18⁺/ΔF inserted with the foreign gene is transfected into LLC-MK2 cells by the procedure described below. LLC-MK2 cells are seeded on 100-mm dishes at 5 x 10⁶ cells/dish. To transcribe the genomic RNA using T7 RNA polymerase, the cells are cultured for 24 hours, and then recombinant vaccinia virus, which expresses T7 RNA polymerase (PLWUV-VacT7: Fuerst, T.R. et al., Proc. Natl. Acad. Sci. USA 83, 8122-8126 (1986)) and is treated with psoralen and long-wavelength ultraviolet light (365 nm) for 20 minutes, is inoculated to the cells at an MOI of about 2 at room temperature for one hour. The ultraviolet light irradiation to the vaccinia virus can be achieved, for example, by using UV Stratalinker 2400 with five 15-watt bulbs (catalog No. 400676 (100V); Stratagene, La Jolla, CA, USA). After the cells are washed with serum-free MEM, plasmid expressing the genomic RNA and expression plasmids each expressing N, P, L, F, or HN protein of the negative-strand RNA virus are transfected into the cells using an appropriate lipofection reagent. The plasmid ratio is preferably, but is not limited to, 6:2:1:2:2:2 in this order. For example, the expression plasmid for the genomic RNA, and the expression plasmids each of which expresses N, P, or L protein, or F and HN proteins (pGEM/NP, pGEM/P, pGEM/L, and pGEM/F-HN; WO00/70070, Kato, A. et al., Genes Cells 1, 569-579, 1996) are transfected at amounts of 12, 4, 2, 4, and 4 µg/dish, respectively. After a few hours of culture, the cells are washed twice with serum-free MEM, and then cultured in MEM supplemented with 40 µg/ml cytosine β-D-arabinofuranoside (AraC: Sigma, St. Louis, MO) and 7.5 µg/ml trypsin (Gibco-BRL, Rockville, MD). The cells are recovered, and the resulting pellet is suspended in OptiMEM (10⁷ cells/ml). The suspension is subjected to three freeze-thaw cycles, and mixed with lipofection reagent DOSPER (Roche #1811169) (10⁶ cells/25 µl DOSPER). After the mixture is allowed to stand at room temperature for 15 minutes, it is transfected to F-expressing helper cells (10⁶ cells/well in 12-well-plate) cloned as described above. The cells are cultured in serum-free MEM (containing 40 µg/ml AraC and 7.5 µg/ml trypsin), and the supernatant is collected. Viruses deficient in a gene other than F, for example, HN or M gene, can be prepared by a similar method as described above.

### (5) Viral vector compositions and cells

The vectors of the present invention can be prepared as compositions depending on the purpose. In the production of compositions containing the viral vectors of the present invention, the vectors may be combined with desired pharmaceutically acceptable carriers or media according to needs. The "pharmaceutically acceptable carriers or medina" refers to materials that can be administered together with the vectors and that do not significantly inhibit the gene transfer via the vectors. Such carriers and media include, for example, sterile water, sodium chloride solution, dextrose solution, Ringer's solution containing dextrose, sodium chloride, and lactate, culture medium, serum, and phosphate buffered saline (PBS). They may be appropriately combined with the vectors to formulate a composition. The compositions of the present invention may include carriers or media such as deionized water and aqueous dextrose solution. The compositions may also include membrane stabilizers for liposome (for example, sterols such as cholesterol). The compositions may also include antioxidants (for example, tocopherol or vitamin E). In addition, the compositions may also include vegetable oils, suspending agents, detergents, stabilizers, biocidal agents, and such. Furthermore, preservatives and other additives may also be added.

The formula of the present composition may be aqueous solution, capsule, suspension, syrup, or such. The compositions of the present invention may also be in a form of solution, freeze-dried product, or aerosol. When it is a freeze-dried product, it may include sorbitol, sucrose, amino acids, various proteins, and such as a stabilizer.

In an embodiment, the present invention includes anti-tumor agents containing a negative-strand RNA viral vector that encodes a cytokine. In another embodiment, the present invention includes cells into which a negative-strand RNA viral vector that encodes a cytokine of the present invention has been introduced, and anti-tumor agents containing such cells. Compositions containing the vectors of the present invention and cells introduced with the vectors are useful as anti-tumor pharmaceuticals. Furthermore, the vector compositions of the present invention and the cells introduced with the vectors are also useful as anti-tumor vaccines. The vector compositions and the cells may include immunostimulants such as cytokine, cholera toxin, and Salmonella toxin to improve immunogenicity. Furthermore, the vaccines may be combined with adjuvants, such as alum, incomplete Freund's adjuvant, MF59 (oil emulsion), MTP-PE (muramyl tripeptide derived from cell wall of mycobacteria), and QS-21 (derived from soapbark tree *Quilaja saponaria*).

When administering the compositions or cells, they may be combined with cytokines that improve the adjuvant effect. Such cytokines include, for example, IL-12 (Proc. Natl. Acad. Sci. USA 96 (15): 8591-8596, 1999), interferon-γ (U.S. Patent No. 5,798,100), and IL-4 (J. Neurosurgery 90 (6), 1115-1124 (1999)).

### (6) Gene therapy

The above-described viral vectors of the present invention, and compositions or cells comprising the vectors can induce *in vivo* immune responses by cytokine production in the cells into which the vectors have been introduced. Thus, the vectors are preferably used, for example, in gene therapy against tumors for which cytokines are effective. For example, the viral vectors of the present invention encoding GM-CSF are expressed in various cancer cells, and have an *in vivo* anti-tumor activity, as described in the Examples in this specification. Herein, "anti-tumor activity" refers to an activity of suppressing tumor development and/or growth.

The viral vectors of the present invention, or compositions or cells comprising the vectors can suppress tumor formation (including recurrence) or growth (including metastasis) by induction of anti-tumor immune response at the administration site, when they are locally administered to a tumor tissue, a risky site of tumor development (for example, a site from which tumor has been removed), or such. The vector introduction can be achieved *in vivo* or *ex vivo.* For *in vivo* introduction, the vectors are injected directly into tumor sites. For *ex-vivo* introduction, the vectors are introduced into cells outside the body and the cells are injected into tumor sites. A tumor site refers to a tumor itself, a tumor excision site, or a region adjacent thereto. Herein, the adjacent region refers to a region from which cytokines secreted from cells introduced with the vectors can reach the tumor or tumor excision site. The region is preferably within 5 mm, for example, within 3 mm, 2 mm, or 1 mm of a tumor or tumor excision site. The vector introduction may be conducted by dissolving or suspending a vector or cells introduced with the vector in a desired carrier (desired physiological aqueous solution, for example, culture medium, physiological saline, blood, plasma, serum, body fluid, etc.), and then directly injecting them into a tumor or a region adjacent thereto. The method of the present invention allows effective treatment and prevention of tumors.

When the *ex vivo* method is carried out, MOI (multiplicity of infection, the number of infecting viruses per cell) for cell infection is preferably in the range of 1 to 500, more preferably 2 to 300, even more preferably 3 to 200, still more preferably 5 to 100, yet more preferably 7 to 70. Contact between the vector and target cells needs only a short period of time, which may be, for example, 1 minute or longer, preferably 3 minutes or longer, 5 minutes or longer, 10 minutes or longer, or 20 minutes or longer, about 1 to about 60 minutes, and more specifically about 5 to 30 minutes. Also, the contact time may be longer, for example, several days or longer. Cells derived from a patient to be administered may be used, for example, primary cultured fibroblast cells derived from a patient can be preferably used. Alternatively, xenogeneic cells and allogeneic cells can be used (Iwadate, Y. et al., Cancer Res., 61: 8769-8774, 2001). Xenogeneic or allogeneic cells are expected to be eliminated through host immune response after *ex-vivo* injection. The cells may be treated by UV, X-ray or gamma-ray irradiation, or such to make their dividing ability defective, and introduced *ex vivo* into tumors subsequently.

The vectors of the present invention were demonstrated to have the ability to express genes in various cancer cells, as described in the Examples herein. Thus, the vectors can be used to treat various tumors. For example, metastatic renal cell cancer (kidney cancer) is a disease with very poor prognosis; and the five-year survival rate after treatment is only about 10% even when the disease is treated with conventional therapy such as surgical therapy, radiotherapy, chemotherapy, or such. Thus, there is a demand for new therapeutic methods for metastatic renal cell cancer. The vectors of the present invention are also expected to be effective against metastatic cancers including metastatic kidney cancer, since they are delivered to tumor tissues via blood stream and expected to have fewer adverse effects as compared to anticancer agents used in conventional chemotherapy. In particular, in a clinical study of immunogene therapy using autologous kidney cancer cells into which the human GM-CSF gene was introduced as vaccine cells, tumor-specific immunity was demonstrated to be induced in three out of four cases. In addition, administration of a constant dose of IL-2 after inoculation of the vaccine cells resulted in the survival of two patients out of four for five years or longer (one patient has survived for seven years and eight months). This indicates that the vectors of the present invention, which encode the cytokines, GM-CSF and/or IL-2, are effective against metastatic cancers including metastatic kidney cancer.

The dosage of the vectors of the present invention can be appropriately determined by those skilled in the art, although it varies depending on the disease, patient's weight, age, sex, symptom, objective of administration, form of composition administered, administration method, type of gene to be introduced, and such. The route of administration can be appropriately selected, and includes, for example, percutaneous, intranasal, transbronchial, intramuscular, intraperitoneal, intravenous, intraarticular, intraspinal, and subcutaneous administrations, but is not limited thereto. Intranasal administration is a preferred administration route. The administration may be local and/or systemic. It is preferred to administer the vectors at a dosage within the range of preferably about 10⁵ CIU/ml to about 10¹¹ CIU/ml, more preferably about 10⁷ CIU/ml to about 10⁹ CIU/ml, and still more preferably about 1 x 10⁸ CIU/ml to about 5 x 10⁸ CIU/ml, together with a pharmaceutically acceptable carrier. A single dose for human is preferably 2 x 10⁵ CIU to 2 x 10¹⁰ CIU. The frequency of administration can be once or more, and within the range of clinically acceptable side effects. The same applies to the daily administration frequency. For protein preparations produced using vectors of the present invention, the protein dosage may be, for example, within the range of 10 ng/kg to 100 µg/kg, preferably 100 ng/kg to 50 µg/kg, and more preferably 1 µg/kg to 5 µg/kg. For non-human animals, for example, the dosage to be administered can be converted from the above-described dosage based on the body weight ratio or volume ratio (*e.g.,* average value) of the target site for administration between the animal of interest and human.

The subjects to which the vectors of the present invention are administered are not particularly limited, and include humans, and all nonhuman mammals such as monkeys, mice, rats, rabbits, sheep, bovines, and dogs.

The administration of the vectors of the present invention may be achieved in combination with immunization using a tumor antigen or a vector that expresses the antigen. The treatment in combination with tumor antigen immunization is expected to produce an anti-tumor effect that is significantly stronger than that of the administration of the vectors alone. The antigens to be used for tumor antigen immunization include, for example, tumor cells that have lost their growth ability, and tumor cell lysates. It is preferable to eliminate the growth ability of tumor cells by heating, radiation, mitomycin C treatment, or such. For example, when X-ray irradiation is used, a total radiation dose of 700 to 3300 Rad can be irradiated. The mitomycin C treatment can be carried out, for example, by adding 25 to 50 µg/ml mitomycin C to cells and incubating the cells at 37°C for 30 to 60 minutes. The heat treatment of cells can be achieved, for example, by heating at 50 to 65°C for 20 minutes. Alternatively, instead of tumor cells, tumor antigens expressed in target tumor cells may be used. Such tumor antigens may be naturally occurring or recombinant polypeptides. Alternatively, a tumor antigen-expressing vector can be administered. The tumor antigen-expressing vector is not particularly limited; for example, desired expression vectors that have the ability to express a tumor antigen in administered subjects, such as plasmid, viral vector, and naked DNA, are used. Such vectors may be those containing a nucleic acid in which a nucleic acid encoding a tumor antigen is linked downstream of an appropriate promoter (for example, SV40 promoter, CAG promoter, CMV promoter, EFI promoter, or LTR promoter). When viral vectors are used, a tumor antigen-encoding nucleic acid is linked so that its expression is controlled under an expression regulatory sequence suitable for each viral vector.

If desired, the cytokine-encoding negative-strand RNA virus vectors of the present invention may be provided in the form of a kit, in combination with other substances required for anti-tumor treatment (for example, solvents required for vector administration, tumor antigens, cytokines, and adjuvants to be co-administered with the vectors, etc.), apparatuses, and such.

All the prior art documents cited herein are incorporated by reference into the present specification.

### Examples

Hereinbelow, the present invention is specifically described with reference to the Examples; however, it should not be construed as being limited thereto.

### Materials and methods used in the experiments

### (1) Mouse

Six to eight week-old female BALB/c and C57BL/6 mice were purchased from CLEA Japan, Inc (Tokyo, Japan) and housed in the Animal Maintenance Facility at Kyushu University under specific pathogen-free conditions. All animal experiments were approved by the Committee of the Ethics on Animal Experiments in the Faculty of Medicine, Kyushu University, and performed following the Guidelines for Animal Experiments in the Faculty of Medicine, Kyushu University, Fukuoka, Japan and the Law and Notification of the Government. Mouse experiments were performed at least twice to confirm the results.

### (2-1) Vector construction (Sendai virus vectors)

### (i) Construction of a NotI fragment for each gene (addition of the transcription signal of Sendai virus)

PCR was carried out using a mouse GM-CSF gene cDNA (mGM-CSF gene cDNA) (SEQ ID NO: 17) as template, and the following two primers: pmGMCSF-NNotI (SEQ ID NO: 18) and pmGMCSF-CNotI (SEQ ID NO: 19). The resulting PCR product was digested with *Nat*I*,* and then subcloned into pBluescript^{™} II KS (Stratagene) to construct a GM-CSF gene *Not*I fragment to which the transcriptional signal of Sendai virus (italic) was added (SEQ ID NO: 20).

### (ii) Construction of an F gene-deficient SeV cDNA carrying the GM-CSF gene

AcDNA (pSeV 18+NotI/ΔF) of an F gene-deficient SeV vector (WO 00/70070) was digested with *Not*I*.* The GM-CSF gene *Nat*I fragment constructed as described in (i) above was inserted into the *Not*I site to construct an F gene-deficient SeV cDNA carrying the mGM-CSF gene (pSeV18+mGM-CSF/ΔF).

### (iii) Reconstitution and amplification of Sendai virus vectors

Virus reconstitution and amplification were carried out according to the report of Li *et al.* (Li, H.-O. et al., J. Virology 74: 6564-6569, 2000; WO 00/70070) and a modified method thereof (WO 2005/071092; Inoue M, et al. J. virology. 2003; 77: 3238-46). Production of the vector used helper cells for the F protein, in which the F protein expression can be induced by the Cre/loxP expression induction system. This system uses the pCALNdLw plasmid (Arai, T. et al., J. Virol. 72: 1115-1121 (1988)), which was designed so that the expression of gene products is induced by Cre DNA recombinase. To express the inserted genes, a transformant having the plasmid was infected with a recombinant adenovirus that expresses Cre DNA recombinase (AxCANCre) by the method of Saito *et al.* (Saito, I. et al., Nucl. Acid. Res. 23, 3816-3821 (1995); Arai, T. et al., J. Virol, 72, 1115-1121 (1998)).

An F gene-deficient SeV vector carrying the mGM-CSF gene (abbreviated as SeV18+mGM-CSF/ΔF) was prepared by the methods described above (Fig. 1b). SeV18+mGM-CSF/ΔF was used as a sample in the experiments described in Example 10.

In addition, an F gene-deficient SeV vector carrying mouse TARC (PUBMED Accession No. NM_011332) (abbreviated as SeV18+mTARC/ΔF) and an F gene-deficient SeV vector carrying mouse RANTES (PUBMED Accession No. X70675) (abbreviated as SeV18+mRANTES/ΔF) were prepared by the same methods as described above (PUBMED URL: http://www.ncbi.nlm.nih.gov/sites/entrez).

Furthermore, an F gene-deficient Sendai virus vector expressing mGM-CSF that carries the following mutations (hereinafter abbreviated as SeV18+mGM-CSF/TSΔF) was constructed: the G69E, T116A, and A183S temperature-sensitive mutations in the M protein, the A262T, G264, and K461 G temperature-sensitive mutations in the HN protein, the L511F mutation in the P protein, and the N1197S and K179SE mutations in the L protein (Fig. la, the full-length sequence is shown in SEQ ID NO: 21).

### (2-2) Vector construction (Adenovirus vectors)

The replication-defective recombinant Ad5 adenovirus vectors that lack the E1A, E1B, and E3 genes, and carry the GFP or mouse GM-CSF gene (AdV/GFP and AdV/GM, respectively) were constructed as previously described (Abe J, et al. Journal of cancer research and clinical oncology. 1995; 121: 587-92), and kindly provided by the RIKEN BioResource Center (BRC). The recombinant virus vectors were propagated in 293 cells (ATCC), and the titer was determined by a plaque-forming assay in 293 cells (the TCID₅₀ method). The adenovirus solutions were stored at -80°C until use. The recombinant adenovirus vectors were used as a control to compare their antitumor activity with that of recombinant SeV vectors in this study (Fig. 1c).

### (3) Tumor cell lines and culture media

WEHI-3B cells, a mouse myelomonocytic leukemia cell line, were kindly provided by Dr. D. Metcalf (University of Melbourne), and RENCA cells, a mouse renal cell cancer cell line, were kindly provided by Dr. M. Azuma (Tokyo Medical and Dental University). LLC (mouse Lewis lung carcinoma) and EL4 (mouse lymphoma) cell lines were purchased from the American Type Culture Collection (Manassas, VA). The human non-small cell carcinoma cell lines, PC9, H1299, H460, and LK87, were kindly provided by Dr. K. Takayama (Kyushu University). The human RCC (renal cell cancer) cell lines, Caki-1, Caki-2, and A498 were purchased from the American Type Culture Collection (ATCC) (Manassas, VA). OSRC-2 and VMRC-RCW cells were purchased from the RIKEN BioResource Center (BRC) (Ibaraki, Japan) and the Japanese Collection of Research Bioresources (JCRB) (Osaka, Japan), respectively. All the cell lines were cultured at 37°C in a humidified atmosphere containing 5% CO₂. All the tumor cell lines, except for LLC cells which were maintained in DMEM (GIBCO BRL, NY), were cultured in tissue flasks or petri dishes containing RPMI 1640 (GIBCO BRL) supplemented with 10% heat-inactivated FBS and penicillin (100 units/ml), streptomycin (0.1 mg/ml), and 2 mM blutamine (complete medium: CM).

### (4) Preparation of vaccine cells

Vaccine cells used in the experiments (RENCA/SeV18+mGM-CSF/TSΔF, RENCA/AdV+mGM-CSF, RENCA/SeV18+GFP/TSΔF, and RENCA/AdV+GFP) were prepared by introducing each of the following into RENCA cells: SeV18+mGM-CSF/TSΔF, and AdV+mGM-CSF, an F gene-deficient SeV vector carrying GFP (SeV18+GFP/TSΔF), and AdV carrying GFP (AdV+GFP) as controls. The amount of GM-CSF produced by 1.0 x 10⁶ vaccine cells in a culture medium for 24 hours was determined by ELISA (BD Pharmingen, NJ). The level of GM-CSF production was 147.5 and 1241 ng/10⁶ cells/48 hours in RENCA transformed with SeV18+mGM-CSF/TSΔF (MOI=100) and AdV+mGM-CSF (MOI=300) (RENCA/SeV18+mGM-CSF/TSΔF and RENCA/AdV+mGM-CSF), respectively.

### (5) Viability of tumor cells after non-transmissible SeV infection

Cell viability was determined by trypan blue exclusion. Two million RENCA or LLC cells were seeded onto 100-mm petri dishes. The cells were infected with SeV18+GFP/TSΔF (MOI=100) or SeV18+mGM-CSF/TSΔF (MOI=100) for 90 minutes in serum-free RPMI or DMEM, and cultured in CM for 48 hours. Then, the cells were treated with trypsin, diluted, and stained with 0.4 % (w/v) trypan blue (GIBCO BRL). The number of trypan blue-positive and -negative cells was counted under a light microscope. The percentage of cells that excluded trypan blue was obtained as an index of cell viability. Cell morphology was also visualized under the light microscope.

### (6) in vitro proliferation assay

For the proliferation assay, RENCA and LLC cells were separately cultured in 96-well microplates at a concentration of 1 x 10⁴ cells/well. After incubation for four to five hours with CM to promote cell adhesion, the tumor cells were washed with PBS, infected with SeV18+GFP/TSΔF (MOI=1, 10, or 100) or SeV18+mGM-CSF/TSΔF (MOI=1, 10, or 100) for 90 minutes in serum-free RPMI or DMEM, and incubated for one to four days. At each time point (day zero, one, two, and four post SeV infection), the number of viable cells was estimated spectrophotometrically by incorporation of tetrazolium dye using Cell Count Reagent SF (Nacalai Tesque, Inc., Kyoto, Japan). The reagent was added to the cells, and this was incubated for an additional hour. Then, the OD value at 450 nm was determined using a microplate reader. For each sample, all the assays from three independent experiments were performed in triplicate.

### (7) Statistical analysis

For statistical analysis, a two-tailed Student's t test was used. The p values were obtained from two-tailed tests of statistical significance. Survival was plotted using Kaplan-Meier curves, and statistical relevance was determined by a log-rank comparison using the Statview software. A probability value was considered to be significant when p<0.05.

### [Example 1] Gene transfer efficiency and expression efficiency of SeV vectors in colon cancer cell lines

### (i) Expression experiment

Seven types of colon cancer cell lines (SW620, SW837, Colo205, Colo320, WiDr, HCT116, HCT116, and LS 174) were infected with a vector carrying LacZ. The Sendai virus vector carrying the LacZ gene (SeV18+LacZ) was prepared by the method described in the document (Shiotani A, Fukumura M, Maeda M, Hou X, Inoue M, Kanamori T, Komaba S, Washizawa K, Fujikawa S, Yamamoto T, Kadono C, Watabe K, Fukuda H, Saito K, Sakai Y, Nagai Y, Kanzaki J, Hasegawa M., Skeletal muscle regeneration after insulin-like growth factor I gene transfer by recombinant Sendai virus vector. Gene Ther 8 (14): 1043-50, 2001). The adenovirus vector carrying the LacZ gene (AdV+LacZ) was used as a control (http://clontech.takara-bio.co.jp/product/catalog/004003003.shtml). AdV infection was carried out at an Moi of 5 or 50, and SeV infection was carried out at an Moi of 1 or 5. Forty eight hours after infection, the expression efficiency of each vector was determined based on the LacZ activity by measuring the fluorescence intensity of cells into which each vector was introduced, using a flow cytometer and the fluorescent substrate, fluorescein di-β-D-galactopyranoside (FDG). The result showed that the LacZ expression at an MOI of 5 was greater in the SeV of the present invention than in AdV. This demonstrates that the SeV of the present invention is suitable for gene introduction into cancer cells (Fig. 2).

In addition, the following experiment was carried out, as also shown in Fig. 3.

Nine human and five mouse tumor cell lines propagated *in vitro* were collected, transduced with SeV/ΔF/GFP, and examined for gene transduction efficiency. Flow cytometric analysis showed dose-dependent GFP expression, and optimal expression was obtained at an MOI of 10 to 100 (Figs. 3a and b).

Furthermore, the following cells were examined for GFP expression. The cell transduction efficiency of SeV was determined based on the presence/absence of fluorescence emission.
(1) The cell lines in which 100% GFP fluorescence was observed in most cells at moi=10:
   Colon cancer (SW620, SW837, SW480, Colo205, Colo320, LS174, WiDr, HCT116, HT29)
   Esophageal cancer (T, Tn, YES)
   Glioma (U87, A172, U251, mutant p53)
   Pancreatic cancer (pancreatic adenocarcinoma) (Pane I, BXPC-3, MIAPACA-2, ASPA-1)
   Breast cancer (MSF-7, MDA-MB-231, MDA-MB-435s, MDA-MB-157, MDA-MB-468)
   Ovarian cancer (OVACAR 3, OVACAR4, OVACAR5, OVACAR8, SKOV3)
   Lung cancer (A549 (non-small cell lung cancer cell line), PC14 (non-small cell lung cancer cell line), RERF-LC-A1, ANIP-973 (lung adenocarcinoma, meta hirono stock))
   Head and neck cancer
   Oral squamous cell carcinoma (HO-1-u-1 (TKG0455), HSC-2 (TKG0487))
   Oral cancer (KB (TKG0401))
   Laryngeal cancer (HEp-2 (TKG0403))
   Gingival cancer (Ca9-22 (TKG0485))
   Prostate cancer (PC-3, DU145)
(2) The cell lines in which 70 to 80% GFP fluorescence was observed in most cells at moi=100:
   Mouse melanoma (B 16)
   Mouse squamous cell carcinoma (SCCVII)
   Lingual cancer (SAS (TLG0470), HSC-3 (TKG0484), HSC-4 (TKG0489), TF)
   Human prostate cancer (LNCAP)

### [Example 2] GM-CSF expression in tumor cells into which SeV18+mGM-CSF/TSΔF or SeV18+hGM-CSF/TSΔF was introduced

Next, the level of GM-CSF expression was quantified in tumor cells into which SeV18+mGM-CSF/TSΔF or SeV18+hGM-CSF/TSΔF was introduced. As shown in Fig. 4a, the level of mouse GM-CSF in four histologically different mouse tumor cell lines transduced with SeV18+mGM-CSF/TSΔF was maximized to more than 300 ng/10⁶ cells/24 hours, at an MOI of more than 50. Likewise, two NSCLC (human) and two RCC (human) cell lines that were transduced with SeV18+hGM-CSF/TSΔF produced GM-CSF at a high level in an MOI-dependent manner for at least seven days after transduction (Fig. 4b). Thus, it was demonstrated that SeV18+mGM-CSF/TSΔF and SeV18+hGM-CSF/TSΔF have high gene transduction ability in various tumor cell lines.

### [Example 3] Growth and viability of tumor cells into which SeV18+mGM-CSF/TSΔF was introduced

The following experiment was performed to exclude the possibility that the survival and growth of tumor cells are affected by Sendai virus vector-mediated exogenous gene transduction itself or Sendai virus component genes themselves. RENCA and LLC tumor cells transduced with either SeV18+GFP/TSΔF or SeV18+mGM-CSF/TSΔF were cultured *in vitro* under the same conditions, and their cell viability and proliferation were evaluated.

### Experimental method

Cell viability was evaluated by trypan blue exclusion. Two million RENCA or LLC cells were seeded onto 100-mm petri dishes. These cells were infected with SeV18+GFP/TSΔF (MOI=100) or SeV18+mGM-CSF/TSΔF (MOI=100) for 90 minutes in serum-free RPMI or DMEM, and cultured for 48 hours in CM. Then, the cells were treated with trypsin, diluted, and stained with 0.4 % (w/v) trypan blue (GIBCO BRL). The number of trypan blue-positive and -negative cells was counted under a light microscope. The percentage of cells that excluded trypan blue was obtained as an index of cell viability. Cell morphology was also visualized under the light microscope.

### Experimental Result

No remarkable difference in cell viablilty was observed between SeV18+GFP/TSΔF-transduced and SeV18+mGM-CSF/TSΔF-transduced LLC cells. However, in RENCA cells, significantly higher cell viability was observed in SeV18+mGM-CSF/TSΔF transduction than in SeV18+GFP/TSΔF transduction (p<0.05) (Figs. 3c and d). Furthermore, after SeV18+GFP/TSΔF or SeV18+mGM-CSF/TSΔF transduction at various MOIs, both RENCA and LLC cells showed a proliferation rate similar to that of non-transduced cells (control) over four consecutive days (p<0.05) (Figs. 3e and f).

Thus, one can rule out the possibility that Sendai virus vector-mediated exogenous gene transduction itself, or Sendai virus component genes themselves affect the survival and growth of tumor cells.

### [Example 4] The anti-tumor effect of SeV18+mGM-CSF/TSΔF (Part 1

The anti-tumor activity against kidney cancer was compared between IrRENCA/SeV18+mGM-CSF/TSΔF and IrRENCA/AdV+mGM-CSF, based on the tumor size and mouse survival rate.

The following experiment was performed to determine the effect of irradiation on the znGM-CSF expression in SeV18-mGM-CSF/TSΔF-transduced tumor cells. SeV18+mGM-CSF/TSΔF-transduced RENCA cells (mouse-derived cancer cells), and SeV18+hGM-CSF/TSΔF-transduced H1299 cells (A549, human-derived cancer cells) were irradiated at 50 Gy and 100 Gy, respectively, and the level of GM-CSF production in these cells was determined.

The irradiated RENCA and H1299 cells continuously produced abundant GM-CSF until day five post transduction with SeV18+mGM-CSF/TSΔF and such (Figs. 5a and b)

### Administration to mice

Cryopreserved RENCA cells (murine renal cell carcinoma) were thawed, cultured, and grown *in vitro* for one to two weeks. Then, the cells were treated with trypsin, washed twice in HBSS, and inoculated subcutaneously in the right flank of BALB/c mice (day zero, 1 x 10⁶ cells/mouse, n=9). The tumor volume was monitored two or three times per week. The renal cell cancer model mice were thereby prepared. Seven days after cells were inoculated into mice (the tumor diameter was 3 to 5 mm), 1.0 x 10⁶ vaccine cells (IrRENCA/SeV18+mGM-CSF/TSΔF (MOI=100) and IrRENCA/AdV+mGM-CSF (MOI=300), each irradiated at 50 Gy) were subcutaneously injected into the left flank region of the mice. Vaccine administration was carried out at the same site (subcutaneously into the left flank region) once every other week for three times. IrRENCA/SeV18+GFP/TSΔF (MOI=100) irradiated at 50 Gy, IrRENCA/AdV+GFP (MOI=300) irradiated at 50 Gy, RC irradiated at 50 Gy, and Hanks' balanced solution salt (HBSS) were administered as control. The tumor size (mm³) (Fig. 6a) and survival rate (%) of the above-mentioned model mice (Fig. 6b) were determined.

This experimental result showed that IrRENCA/SeV18+mGM-CSF/TSΔF has an anti-tumor activity comparable to or greater than that of IrRENCA/AdV+mGM-CSF. Specifically, the tumor suppression effect of IrRENCA/SeV18+mGM-CSF/TSΔF (MOI=100) administration ((7) in the figure) was comparable to or greater than that of IrRENCA/SeV18+GFP/TSΔF (MOI=300) administration ((6) in the figure). Furthermore, the survival rate of the above-described model mice was demonstrated to be higher in the IrRENCAlSeV18+mGM-CSF/TSΔF (MOI=100) administration group as compared to the other administration groups. This experimental result suggests that SeV18+mGM-CSF/ΔF has the activity of suppressing tumor growth.

### [Example 5] CTL assay and cytokine analysis

Next, it was tested whether the tumor shrinkage in the above-mentioned kidney cancer model mice could be induced by immune response upon administration of IrRENCA/SeV18+mGM-CSF/TSΔF to the mice. Specifically, the CTL activity against RENCA cells was assayed (⁵¹Cr CTL assay).

MHC class I expression in tumor cells is required for CTL recognition in cancer immunotherapy (Vertuani S, De Geer A, Levitsky V, Kogner P, Kiessling R, Levitskaya J. Retinoids act as multistep modulators of the major histocompatibility class I presentation pathway and sensitize neuroblastomas to cytotoxic lymphocytes. Cancer research. 2003; 63: 8006-13). Therefore, before CTL assay, the present inventors assessed RENCA and WEHI-3B tumor cells for the expression of MHC class I (H-2K^{d}) on their cell surface by flow cytometry. RENCA and WEHI-3B cells showed high and moderate MHC class I expression, respectively (data not shown).

The method for assaying the above-mentioned CTL activity is schematically illustrated in Fig. 7. Specifically, kidney cancer model mice were prepared by subcutaneously inoculating 1.0 x 10⁶ mouse RENCA cells into the right flank region. From day seven, the below-mentioned vaccines and such were administered to the above mice every week for three times in total by subcutaneous injection into the left flank region. The vaccines and such are as follows:
(1) HBSS
(2) IrRENCA/AdV+GFP (MOI=300) irradiated at 50 Gy
(3) IrRENCA/SeV18+GFP/TSΔF (M=100) irradiated at 50 Gy
(4) IrRENCA/AdV+mGM-CSF (MOI=300) irradiated at 50 Gy
(5) IrRENCAlSeV18+mGM-CSF/TSΔF (M=100) irradiated at 50 Gy

Regarding the results shown in Figs. 8(a) and (b), the dosage of the above-mentioned vaccines ((4) and (5) above) administered to the mice in the experiment was 3.0 x 10⁶ cells. As for the results shown in Figs. 8(c) and (d), the dosage of the above-mentioned vaccines ((2) to (5) above) administered to the mice in the experiment was 1.0 x 10⁶ cells.

*In vitro* CTL activity (⁵¹Cr release) assay was carried out using spleen cells from the mice seven days after immunization by the injection described above (Fig. 7) (Shibata S, Okano S, Yonemitsu Y, *et al.* Induction of efficient antitumor immunity using dendritic cells activated by recombinant Sendai virus and its modulation by exogenous IFN-beta gene. J Immunol. 2006; 177: 3564-76).

Furthermore, spleen cells from immunized mice were assessed for their *in vitro* cytokine productivity (cytokine analysis). Specifically, supernatant cytokines (IL-2, IL-4, IL-5, IL-6, TNF-α, and IFN-γ) were assayed using spleen cells from mice immunized with HBSS, RENCA/AdV+GFP, RENCA/SeV18+GFP/TSΔF, RENCA/AdV+mGM-CSF, or RENCA/SeV18+mGM-CSF/TSΔF (six days after the second vaccination). The supernatant was prepared by culturing spleen cells *in vitro* for 20 hours alone or together with RENCA cells re-stimulated with 50-Gy irradiation.

### Results of CTL assay and such

In both of the CTL analysis (Fig. 8) and cytokine analysis in the vaccination model, the tumor-specific response and significantly high production of IL-2, IL-4, and IFN-γ were observed in the mice treated with IrRENCA/AdV+mGM-CSF and IrRENCA/SeV18+mGM-CSF/TSΔF, which suppressed tumor growth, as compared to the respective control. In particular, the mice treated with RENCA/SeV18+mGM-CSF/TSΔF produced a significantly higher level of IFN-γ as compared to the mice treated with IrRENCA/AdV+mGM-CSF (p<0.05) (data not shown). As seen from the CTL analysis result shown in Fig. 8, the tumor-specific response against RENCA cells was observed as compared to the negative control.

The experimental result shown in Fig. 8 indicates that
RENCA/SeV18+mGM-CSF/TSΔF has an *in vivo* anti-tumor activity comparable to or greater than that of IrRENCA/AdV+mGM-CSF. This suggests that IrRENCA/SeV18+mGM-CSF/TSΔ, which is a vector of the present invention, has a greater application for the production of autologous GM-CSF vaccines.

### [Example 6] Tumor-specific cytokine production in splenocytes after inoculation of tumor vaccines transduced with GM-CSF

To determine the immunomodulatory effect of GM-CSF-transduced RENCA vaccination, the present inventors evaluated the level of cytokine production in splenocytes in the presence or absence of irradiated RENCA cells, using RENCA vaccine cell-immunized immunocompetent mice.

First, to quantitate the number of IL-4- or IFN-γ-producing splenocytes upon re-stimulation *in vitro,* splenocytes harvested from mice either untreated or treated with IrRENCA/AdV+GFP, IrRENCA/SeV18+GFP/TSΔF, IrRENCA/AdV+mGM-CSF or IrRENCA/SeV18+mGM-CSF/TSΔF were subjected to *in vitro* ELISPOT assay for IFN-γ and IL-4. When splenocytes from RENCA-bearing mice treated with either IrRENCA/AdV+mGM-CSF or IrRENCA/SeV18+mGM-CSF/TSΔF were co-cultured in the presence of irradiated RENCA cells, the number of splenocytes that secreted both IFN-γ and IL-4 was significantly higher than that from the respective control group (IrRENCA/AdV+GFP or IrRENCA/SeV18+GFP/TSΔF) (p<0.05). In addition, the number of splenocytes from mice treated with irRC/SeV/GM cells that secreted IFN-γ was significantly higher than that from mice treated with irRC/AdV/GM cells (p<0.05) (Fig. 9a). These enhanced IFN-γ-producing cells are tumor antigen-specific, since the number of IFN-γ- and IL-4-producing cells in the presence of an irrelevant antigen, irradiated WEHI-3B cells, is as low as that in the absence of antigen (Fig. 9b).

Next, the present inventors measured the various *in vitro* inflammatory cytokines produced by splenocytes from mice either left untreated (HBSS only) or treated with IrRENCA/AdV+GFP, IrRENCA/SeV18+GFP/TSΔF, IrRENCA/AdV+mGM-CSF or IrRENCA/SeV18+mGM-CSF/TSΔF. After a 20-hour co-culture with or without irradiated RENCA cells for re-stimulation, the supernatant was collected, and the following inflammatory cytokines were measured: IL-2, IL-4, IL-5, IL-6, IFN-γ and TNF-α. Similar to the ELISPOT assay, the level of IFN-γ, IL-2, and IL-4 produced in splenocytes from mice treated with IrRENCA/AdV+mGM-CSF or IrRENCA/SeV18+mGM-CSF/TSΔF was significantly higher than that in splenocytes treated with the respective GFP control (p<0.05) (Figs. 9d, e, and f). In particular, similar to the ELISPOT assay, only IFN-γ production in splenocytes of mice treated with IrRENCA/SeV18+mGM-CSF/TSΔF was greater than that of mice treated with IrRENCA/AdV+mGM-CSF (p<0.05) (Fig. 9d). IL-2 produced from splenocytes of mice treated with IrRENCA/AdV+GFP was significantly higher than that in mice treated with IrRENCA/SeV18+GFP/TSΔF (p<0.05) (Fig. 9e). Although the level of TNF-α and IL-6 production in each vaccine group, except for the HBSS-treated group, was elevated in the presence of stimulator cells, there was no significant difference between each GFP-treated group and GM-CSF-treated group (Figs. 9c and h). Intriguingly, the level of IL-5 produced by splenocytes from mice treated with IrRENCA/AdV+mGM-CSF or IrRENCA/SeV18+mGM-CSF/TSΔF in the presence of stimulator cells was elevated compared with the respective GFP control (IrRENCA/SeV18+mGM-CSF/TSΔF versus IrRENCA/SeV18+GFP/TSΔF; p<0.05) (Fig. 9g). This result suggests that the vaccination systemically activated eosinophils, which are putatively involved in GM-CSF-induced antitumor responses (Soiffer R, Lynch T, Mihm M, et al. Vaccination with irradiated autologous melanoma cells engineered to secrete human granulocyte-macrophage colony-stimulating factor generates potent antitumor immunity in patients with metastatic melanoma. Proc Natl Acad Sci USA. 1998; 95: 13141-6.; Tani K, Azuma M, Nakazaki Y, et al. Phase I study of autologous tumor vaccines transduced with the GM-CSF gene in four patients with stage IV renal cell cancer in Japan: clinical and immunological findings. Mol Ther. 2004; 10: 799-816.; Chu Y, Xia M, Lin Y, et al. Th2-dominated antitumor immunity induced by DNA immunization with the genes coding for a basal core peptide PDTRP and GM-CSF. Cancer gene therapy. 2006; 13: 510-9.; Ellyard JI, Simson L, Parish CR. Th2-mediated anti-tumour immunity: friend or foe? Tissue antigens. 2007; 70: 1-11.)

The experimental method of evaluating the above-mentioned cytokine production level according to this Example is more specifically described below.

### ELISPOT assay

On day six after the second inoculation of tumor vaccine cells into mice, they were sacrificed, and their splenocytes were tested for mouse IFN-γ and IL-4 secretion using an ELISPOT assay kit (Cytokine ELISPOT Set, BD Pharmingen, NJ). ImmunoSpot^{™} ELISPOT 96-well plates were coated with 5 µg/ml of purified anti-mouse IFN-γ or anti-mouse IL-4 monoclonal antibody, and incubated overnight at 4°C. The wells were washed with PBS containing 0.05% Tween 20, and incubated for two hours with a blocking buffer (RPMI 1640 containing 10% FBS) at room temperature. RBC-depleted splenocytes (1 x 10⁵) were incubated for 20 hours at 37°C in 5 % CO₂ in the presence of irradiated RENCA or WEHI-3B cells at the above-mentioned splenocyte : irradiated tumor cell ratio (100:1 or 50:1) in a total volume of 200 µl. As a positive control, PMA (20 ng/ml of phorbol 12-myristate 13-acetate; Sigma, St.Louis, MO), which is known as a mitogen for T cell stimulation, was added to the above-mentioned wells. After washing the plates, the wells were incubated with 2 µg/ml of biotinylated anti-mouse IFN-γ or anti-mouse IL-4 monoclonal antibody for two hours at room temperature. Next, the plates were washed extensively, incubated with a streptavidin-HRP solution for an hour at room temperature, washed twice, incubated with a final substrate solution (AEC substrate mixed with AEC chromogen, BD Pharmingen, NJ, US), and then monitored for spot development for five minutes at room temperature. After drying the plates, spots indicating IFN-γ- or IL-4-secreting cells were enumerated manually under a Zeiss Axiovert dissecting microscope (Jena, Germany), and the spot number was expressed as the mean + SD based on quadruplicate determinations per sample.

### CBA and ELISA assays

Similar to the ELISPOT analysis, RBC-depleted mouse splenocytes (5 x 10⁶) harvested on day six after the second tumor vaccination were incubated in the presence or absence of irradiated RENCA cells at a 10:1 ratio in a total volume of 1.0 ml at 37°C for 20 hours. The cell supernatant was collected, and the concentration of mouse IL-2, IL-4, IL-5, TNF-α, and IFN-γ was measured using the BD Mouse Th1/Th2 Cytokine CBA (Cytometric Bead Array) Kit (BD Pharmingen) according to the manufacturer's protocol. The concentration of IL-6 was measured using a mouse IL-6 immunoassay kit (R&D Systems, MN) according to the manufacturer's procedure.

### [Example 7 Immunohistochemical characterization of tumor infiltrating leukocytes stimulated by irradiated GM-CSF-transduced RENCA vaccine cells

To identify the key immune cells that are involved in the antitumor effect of irradiated GM-CSF-transduced RENCA vaccine cells, the distribution profile of tumor infiltrating leukocytes (TILs) in RENCA-bearing mice either untreated or treated with the above-mentioned vaccines (IrRENCA/AdV+GFP, IrRENCA/SeV18+GFP/TSΔF, IrRENCA/AdV+mGM-CSF, or IrRENCA/SeV18+mGM-CSF/TSΔF) was assessed by immunohistochemistry (IHC).

### Experimental method

On day six after the second inoculation of tumor vaccine cells into the above-mentioned therapeutic RENCA model, established RENCA nodules were snap-frozen by overlaying with OCT compound (Sakura Fine Technical Co., Tokyo, Japan). All samples were stored at -80°C until analysis. Serial cryostat (8-10 µm) frozen sections were adhered to Superfrost slide glasses (Matsunami, Osaka, Japan), fixed in acetone at room temperature for ten minutes, air-dried, and rinsed in distilled water to remove the embedding medium. Staining was conducted according to standard procedures. Briefly, sections were sequentially incubated overnight at 4°C with appropriately diluted anti-mouse CD4 (GK1.5), CD8 (53-6.7), CD11c (N418), and FoxP3 (FJK-16s) primary antibodies (all from eBioscience, San Diego, CA) following the manufacturer's instructions, and then incubated for an hour with biotinylated anti-rat or anti-hamster secondary antibody (eBioscience). After incubation for 30 minutes with streptavidin-peroxidase (Dako Japan Co. Ltd., Kyoto, Japan), the antigen-antibody reaction was detected using 3, 3'-diaminobenzidine (Nakalai tesque, Kyoto, Japan) substrate. Slides were washed three times with PBS between each incubation step, and counterstained with Mayer's hematoxylin, and dehydrated in sequentially degraded alcohol and xylene prior to mounting. All the incubation steps were conducted in a humid chamber. Photographs were taken with a Zeiss Axiovert microscope. The stained cells were visualized in a series of high-power fields (HPFs), and counted microscopically at 200X magnification in 30 to 70 HPFs. The percentage of positive cells was calculated, and the result was expressed as the mean ± SD.

### Experimental result

IHC analysis revealed more infiltrating CD8⁺ T cells in tumor masses from mice treated with IrRENCA/AdV+mGM-CSF or IrRENCA/SeV18+mGM-CSF/TSΔF than those treated with the respective control (IrRENCA/AdV+GFP or IrRENCA/SeV18+GFP/TSΔF) (p<0.05) (Fig. 10). There was no significant difference in CD11c (dendritic cells) and FoxP3 (regulatory T cells) staining among the tumor vaccination groups.

### [Example 8] The anti-tumor effect of SeV18+mGM-CSF/TSΔF (Part 2)

The tumor suppressing effect of SeV18+mGM-CSF/TSΔF was assessed using lung cancer model mice.

Lung cancer model mice were prepared by subcutaneously inoculating LLC cells into the right flank of BALB/c mice (day 0, 2 x 10⁵ cells/mouse, n = 6). From day three post inoculation of LLC cells (when tumors became palpable) (Kojima T, Yamazaki K, Tamura Y, et al. Granulocyte-macrophage colony-stimulating factor gene-transduced tumor cells combined with tumor-derived gp96 inhibit tumor growth in mice. Human gene therapy. 2003; 14: 715-28), the below-mentioned vaccine cells and such were subcutaneously inoculated into the left flank of the above-described model mice every four days for three times (days three, six, and nine). The vaccine cells and such are the following:
(1) HBSS alone
(2) 1.0 x 10⁶ IrLLC cells irradiated at 50 Gy
(3) 1.0 x 10⁶ IrLLC/SeV18+GFP/TSΔF (MOI=100) cells irradiated at 50 Gy
(4) 1.0 x 10⁶ IrLLC/SeV18+mGM-CSF/TSΔF (MOI=100) cells irradiated at 50 Gy

Tumor suppression was observed in mice of the IrLLC/SeV18+mGM-CSF/TSΔF (MOI=100) administration group ((4) above) as compared to the other administration groups ((1) to (3) above) (Fig. 11(a)).

Furthermore, the mouse survival rate was higher in the IrLLC/SeV18+mGM-CSF/TSΔF (MOI=100) administration group ((4) above) as compared to the other administration groups ((1) to (3) above).

The experimental result described above suggests that SeV18+mGM-CSF/ΔF has an activity of suppressing tumor growth.

### [Example 9] The anti-tumor effect of SeV18+mGM-CSF/ΔF, SeV18-mTARC/ΔF, and SeV18+mRANTES/ΔF

*In vivo* tumor formation in mice was observed (Fig. 12) by subcutaneously inoculating the cells listed below into the right flank region of Balb/c mice (n = 6). The cells are as follows:
(1) 5.0 x 10⁵ LLC cells,
(2) 5.0 x 10⁵ LLC cells transduced with SeV18+GFP/ΔF (MOI=10),
(3) 5.0 x 10⁵ LLC cells transduced with SeV18+GFP/ΔF (MOI=100),
(4) 5.0 x 10⁵ LLC cells transduced with SeV18+mGM-CSF/ΔF (MOI=10),
(5) 5.0 x 10⁵ LLC cells transduced with SeV18+mGM-CSF/ΔF (MOI=100),
(6) 5.0 x 10⁵ LLC cells transduced with SeV18+mTARC/ΔF (MOI=10) (LLC/SeV18+mTARC/ΔF (MOI=10)),
(7) 2.5 x 10⁵ LLC/SeV18+mTARC/ΔF (MOI=10) cells and 2.5 x 10⁵ LLC cells,
(8) 5.0 x 10⁴ LLC/SeV18+mTARC/ΔF (MOI=10) cells and 4.5 x 10⁵ LLC cells,
(9) 5.0 x 10⁵ LLC cells transduced with SeV18+mRANTES/ΔF (MOI=10) (LLC/SeV18+mRANTES/ΔF (MOI=10)),
(10) 2.5 x 10⁵ LLC/SeV18+mRANTES/ΔF (MOI=10) cells and 2.5 x 10⁵ LLC cells,
(11) 5.0 x 10⁴ LLC/SeV18+mRANTES/ΔF (MOI=10) cells and 4.5 x 10⁵ LLC cells.

This experimental result suggests that SeV18+mGM-CSF/ΔF, SeV18+mTARC/ΔF, and SeV18+mRANTES/ΔF have an activity of suppressing tumor formation.

### [Example 10] The anti-tumor effect of SeV18+mGM-CSF/TSΔF(Part 3)

Lung cancer model mice were prepared by subcutaneously inoculating 2.0 x 10⁵ mouse LLC tumor cells into the right flank region of C57BL/6 mice (n = 4). Three days after inoculation (when the tumor diameter was 3 to 5 mm), 5.0 x 10⁵ DC/SeV18+mGM-CSF/TSΔF cells were subcutaneously inoculated into the left flank region of the above-described mice. The experimental procedure is specifically shown in Figs. 13(a) and (b).

As seen in Figs. 13(c) to (e), tumor growth suppression in the above-mentioned mice was observed in the DC/SeV18+mGM-CSF/TSΔF administration group as compared to the other administration groups (Fig. 13(a)). Furthermore, the mouse survival rate was higher in the DC/SeV18+mGM-CSF/TSΔF administration group as compared to the other administration groups.

This experimental result suggests that SeV18+mGM-CSF/ΔF has an activity of suppressing tumor growth.

### Industrial Applicability

The present inventors demonstrated that an anti-tumor effect was produced when a cytokine having heparin-binding activity, such as granulocyte macrophage colony stimulating factor (GM-CSF), was expressed *in vivo* using viral vectors based on a negative-strand RNA virus. The present inventors also demonstrated that the vectors of the present invention produced a superior protective effect as compared to conventional adenovirus vectors. The negative-strand RNA virus vectors of the present invention carrying a cytokine gene effectively suppress tumor growth by a simple technique. Thus, the vectors of the present invention are expected to be widely applicable to cancer gene therapy. The viral vectors of the present invention can be applied to treatment of various cancers. In particular, the vectors are suitable for treatment of metastatic cancers.

In the Examples described above, specific vectors such as SeV18+GM-CSF/TSΔF and SeV18+GM-CSF/ΔF were used. However, the vectors of the present invention are not limited to these embodiments described in the Examples. For example, various types of Sendai virus vectors can be used, as described above in detail in "Mode for Carrying Out the Invention." For example, it is also expected that an effect similar to that described in the above Examples can be obtained when a heparin-binding cytokine such as GM-CSF is introduced into a non-F gene-deficient Sendai virus vector, temperature-sensitive Sendai virus vector, or such.

## Claims

1. A negative-strand RNA viral vector that carries a cytokine gene.

2. The vector of claim 1, which is a paramyxovirus vector.

3. The vector of claim 2, which is a Sendai virus vector.

4. The vector of any one of claims 1 to 3, wherein the cytokine gene encodes a cytokine having heparin-binding activity.

5. The vector of claim 4, wherein the cytokine having heparin-binding activity is granulocyte macrophage colony stimulating factor (GM-CSF).

6. The vector of any one of claims 1 to 3, wherein the cytokine gene encodes a chemokine.

7. The viral vector of claim 6, wherein the chemokine is TARC or RANTES.

8. The vector of any one of claims 1 to 7, which is intended for treatment of cancer.

9. The vector of claim 8, wherein the cancer is metastatic cancer.

10. The vector of claim 9, wherein the cancer is kidney cancer.

11. The vector of claim 9, wherein the cancer is lung cancer.

12. An anti-tumor composition that comprises the vector of any one of claims 1 to 11.

13. The anti-tumor composition of claim 12, which comprises a dendritic cell.

14. The anti-tumor composition of claim 13 or 14, which comprises two types of vectors, wherein one of the vectors is a vector comprising a GM-CSF-encoding gene, and the other is a vector comprising a chemokine-encoding gene.


SEQUENCE LISTING

<110> KYUSHU UNIVERSITY, NATIONAL UNIVERSITY CORPORATION
DNAVEC CORPORATION

<120> Viral vector for gene therapy

<130> D4-A0703P

<140> PCT/JP2008/058132
<141> 2008-04-25

<150> JP 2007-119130
<151> 2007-04-27

<160> 23

<170> PatentIn version 3.4

<210> 1
<211> 10
<212> RNA
<213> Sendai virus

<400> 1
cmuuvmcccu 10

<210> 2
<211> 10
<212> RNA
<213> Sendai virus

<400> 2
cuuugacccu 10

<210> 3
<211> 10
<212> RNA
<213> Sendai virus

<400> 3
cauucacccu 10

<210> 4
<211> 10
<212> RNA
<213> Sendai virus

<400> 4
cuuucacccu 10

<210> 5
<211> 10
<212> DNA
<213> Sendai virus

<400> 5
agggtcaaag 10

<210> 6
<211> 10
<212> DNA
<213> Sendai virus

<400> 6
agggtgaatg 10

<210> 7
<211> 10
<212> DNA
<213> Sendai virus

<400> 7
agggtgaaag 10

<210> 8
<211> 9
<212> RNA
<213> Sendai virus

<400> 8
uuuuucuua 9

<210> 9
<211> 9
<212> DNA
<213> Sendai virus

<400> 9
taagaaaaa 9

<210> 10
<211> 10
<212> DNA
<213> Sendai virus

<400> 10
ctttcaccct 10

<210> 11
<211> 15
<212> DNA
<213> Sendai virus

<400> 11
tttttcttac tacgg 15

<210> 12
<211> 18
<212> DNA
<213> Artificial

<220>
<223> An artificially synthesized nucleotide sequence

<400> 12
atgcatgccg gcagatga 18

<210> 13
<211> 18
<212> DNA
<213> Artificial

<220>
<223> An artificially synthesized primer sequence

<400> 13
gttgagtact gcaagagc 18

<210> 14
<211> 42

<212> DNA
<213> Artificial

<220>
<223> An artificially synthesized primer sequence

<400> 14
tttgccggca tgcatgtttc ccaaggggag agttttgcaa cc 42

<210> 15
<211> 18
<212> DNA
<213> Artificial

<220>
<223> An artificially synthesized primer sequence

<400> 15
atgcatgccg gcagatga 18

<210> 16
<211> 21
<212> DNA
<213> Artificial

<220>
<223> An artificially synthesized primer sequence

<400> 16
tgggtgaatg agagaatcag c 21

<210> 17
<211> 426

<212>  DNA
<213>  Sendai virus

<400>  17
atgtggctgc agaatttact tttcctgggc attgtggtct acagcctctc agcacccacc      60

cgctcaccca tcactgtcac ccggccttgg aagcatgtag aggccatcaa agaagccctg     120

aacctcctgg atgacatgcc tgtcacattg aatgaagagg tagaagtcgt ctctaacgag     180

ttctccttca agaagctaac atgtgtgcag acccgcctga agatattcga gcagggtcta     240

cggggcaatt tcaccaaact caagggcgcc ttgaacatga cagccagcta ctaccagaca     300

tactgccccc caactccgga aacggactgt gaaacacaag ttaccaccta tgcggatttc     360

atagacagcc ttaaaacctt tctgactgat atcccctttg aatgcaaaaa accagtccaa     420

aaatga                                                                426


<210>  18
<211>  48
<212>  DNA
<213>  Artificial

<220>
<223>  An artificially synthesized primer sequence

<400>  18
attgcggccg ccaaggttca cttatgtggc tgcagaattt acttttcc                  48


<210>  19
<211>  78
<212>  DNA

<213>  Artificial

<220>
<223>  An artificially synthesized primer sequence

<400>  19
attgcggccg cgatgaactt tcaccctaag tttttcttac tacggtcatt tttggactgg      60

tttttgcat tcaaaggg                                                    78

<210>  20
<211>  485
<212>  DNA
<213>  Artificial

<220>
<223>  An artificially synthesized nucleotide sequence

<220>
<221>  misc_feature
<222>  (1)..(17)
<223>  Transcription signal of Sendai Virus

<220>
<221>  misc_feature
<222>  (444)..(485)
<223>  Transcription signal of Sendai Virus

<400>  20
gcggccgcca aagttcaatg tggctgcaga atttactttt cctgggcatt gtggtctaca      60

gcctctcagc acccacccgc tcacccatca ctgtcacccg gccttggaag catgtagagg     120

ccatcaaaga agccctgaac ctcctggatg acatgcctgt cacattgaat gaagaggtag     180

aagtcgtctc taacgagttc tccttcaaga agctaacatg tgtgcagacc cgcctgaaga 240

tattcgagca gggtctacgg ggcaatttca ccaaactcaa gggcgccttg aacatgacag 300

ccagctacta ccagacatac tgccccccaa ctccggaaac ggactgtgaa acacaagtta 360

ccacctatgc ggatttcata gacagcctta aaacctttct gactgatatc ccctttgaat 420

gcaaaaaacc agtccaaaaa tgaccgtagt aagaaaaact tagggtgaaa gttcatcgcg 480

gccgc 485

<210> 21
<211> 16842
<212> DNA
<213> Artificial

<220>
<223> An artificially synthesized vector sequence

<400> 21
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gctcgcgtcg tacgggccct 420

aatacgactc actataacca aacaagagaa aaaacatgta tgggatatgt aatgaagtta 480

tacaggattt tagggtcaaa gtatccaccc tgaggagcag gttccagacc ctttgctttg 540

ctgccaaagt tcacgcggcc gccaaggttc acttatgtgg ctgcagaatt tacttttcct 600

gggcattgtg gtctacagcc tctcagcacc cacccgctca cccatcactg tcacccggcc 660

ttggaagcat gtagaggcca tcaaagaagc cctgaacctc ctggatgaca tgcctgtcac 720

attgaatgaa gaggtagaag tcgtctctaa cgagttctcc ttcaagaagc taacatgtgt 780

gcagacccgc ctgaagatat tcgagcaggg tctacggggc aatttcacca aactcaaggg 840

cgccttgaac atgacagcca gctactacca gacatactgc cccccaactc cggaaacgga 900

ctgtgaaaca caagttacca cctatgcgga tttcatagac agccttaaaa cctttctgac 960

tgatatcccc tttgaatgca aaaaaccagt ccaaaaatga ccgtagtaag aaaaacttag 1020

ggtgaaagtt catcgcggcc gcagatcttc acgatggccg ggttgttgag caccttcgat 1080

acatttagct ctaggaggag cgaaagtatt aataagtcgg gaggaggtgc tgttatcccc 1140

ggccagagga gcacagtctc agtgttcgta ctaggcccaa gtgtgactga tgatgcagac 1200

aagttattca ttgcaactac cttcctagct cactcattgg acacagataa gcagcactct 1260

cagagagggg ggttcctcgt ctctctgctt gccatggctt acagtagtcc agaattgtac 1320

ttgacaacaa acggagtaaa cgccgatgtc aaatatgtga tctacaacat agagaaagac 1380

cctaagagga cgaagacaga cggattcatt gtgaagacga gagatatgga atatgagagg 1440

accacagaat ggctgtttgg acctatggtc aacaagagcc cactcttcca gggtcaacgg 1500

gatgctgcag accctgacac actccttcaa atctatgggt atcctgcatg cctaggagca 1560

ataattgtcc aagtctggat tgtgctggtg aaggccatca caagcagcgc cggcttaagg 1620

aaagggttct tcaacaggtt agaggcgttc agacaagacg gcaccgtgaa aggtgcctta 1680

gttttcactg gggagacagt tgaggggata ggctcggtta tgagatctca gcaaagcctt 1740

gtatctctca tggttgagac ccttgtgact atgaatactg caagatctga tctcaccaca 1800

ttagagaaga acatccagat cgttgggaac tacatccgag atgcagggct ggcttccttc 1860

atgaacacta ttaaatatgg ggtggaaaca aagatggcag ctctaacgtt gtcaaacctg 1920

aggcccgata ttaataagct tagaagcctc atagacacct acctgtcaaa aggccccaga 1980

gctcccttta tctgtatcct caaggaccct gttcatggtg aatttgctcc aggcaattat 2040

cctgcactat ggagttacgc catgggagtc gccgtcgtac agaacaaggc aatgcagcag 2100

tacgtcacag ggaggacata ccttgatatg gaaatgttct tactaggaca agccgtggca 2160

aaggatgctg aatcgaagat cagcagtgcc ttggaagatg agttaggagt gacggataca 2220

gccaagggga ggctcagaca tcatctggca aacttgtccg gtggggatgg tgcttaccac 2280

aaaccaacag gcggtggtgc aattgaggta gctctagaca atgccgacat cgacctagaa 2340

acaaaagccc atgcggacca ggacgctagg ggttggggtg gagatagtgg tgaaagatgg 2400

gcacgtcagg tgagtggtgg ccactttgtc acactacatg gggctgaacg gttagaggag 2460

gaaaccaatg atgaggatgt atcagacata gagagaagaa tagccatgag actcgcagag 2520

agacggcaag aggattctgc aacccatgga gatgaaggcc gcaataacgg tgtcgatcat 2580

gacgaagatg acgatgccgc agcagtagct gggataggag gaatctagga tcatacgagg 2640

cttcaaggta cttgatccgt agtaagaaaa acttagggtg aaagttcatc caccgatcgg 2700

ctcaggcaag gccacaccca accccaccga ccacacccag cagtcgagac agccacggct 2760

tcggctacac ttaccgcatg gatcaagatg ccttcattct taaagaagat tctgaagttg 2820

agagggaggc gccaggagga cgagagtcgc tctcggatgt tatcggattc ctcgatgctg 2880

tcctgtcgag tgaaccaact gacatcggag gggacagaag ctggctccac aacaccatca 2940

acactcccca aggaccaggc tctgctcata gagccaaaag tgagggcgaa ggagaagtct 3000

caacaccgtc gacccaagat aatcgatcag gtgaggagag tagagtctct gggagaacaa 3060

gcaagccaga ggcagaagca catgctggaa accttgataa acaaaatata caccgggcct 3120

ttgggggaag aactggtaca aactctgtat ctcaggatct gggcgatgga ggagactccg 3180

gaatccttga aaatcctcca aatgagagag gatatccgag atcaggtatt gaagatgaaa 3240

acagagagat ggctgcgcac cctgataaga ggggagaaga ccaagctgaa ggacttccag 3300

aagaggtacg aggaagtaca tccctacctg atgaaggaga aggtggagca agtaataatg 3360

gaagaagcat ggagcctggc agctcacata gtgcaagagt aactggggtc ctggtgattc 3420

ctagccccga acttgaagag gctgtgctac ggaggaacaa aagaagacct accaacagtg 3480

ggtccaaacc tcttactcca gcaaccgtgc ctggcacccg gtccccaccg ctgaatcgtt 3540

acaacagcac agggtcacca ccaggaaaac ccccatctac acaggatgag cacatcaact 3600

ctggggacac ccccgccgtc agggtcaaag accggaaacc accaataggg acccgctctg 3660

tctcagattg tccagccaac ggccgcccaa tccacccggg tctagagacc gactcaacaa 3720

aaaagggcat aggagagaac acatcatcta tgaaagagat ggctacattg ttgacgagtc 3780

ttggtgtaat ccagtctgct caagaattcg aatcatcccg agacgcgagt tatgtgtttg 3840

caagacgtgc cctaaagtct gcaaactatg cagagatgac attcaatgta tgcggcctga 3900

tcctttctgc cgagaaatct tccgctcgta aggtagatga gaacaaacaa ctgctcaaac 3960

agatccaaga gagcgtggaa tcattccggg atatttacaa gagattctct gagtatcaga 4020

aagaacagaa ctcattgctg atgtccaacc tatctacact tcatatcatc acagatagag 4080

gtggcaagac tgacaacaca gactccctta caaggtcccc ctccgttttt gcaaaatcaa 4140

aagagaacaa gactaaggct accaggtttg acccatctat ggagacccta gaagatatga 4200

agtacaaacc ggacctaatc cgagaggatg aatttagaga tgagatccgc aacccggtgt 4260

accaagagag ggacacagaa cccagggcct caaacgcatc acgtctcttt ccctccaaag 4320

agaagcccac aatgcactct ctcaggctcg tcatagagag cagtccccta agcagagctg 4380

agaaagtagc atatgtgaaa tcattatcca agtgcaagac agaccaagag gttaaggcag 4440

tcatggaact cgtagaagag gacatagagt cactgaccaa ctagatcccg ggtgaggcat 4500

cctaccatcc tcagtcatag agagatccaa tctaccatca gcatcagcca gtaaagatta 4560

agaaaaactt agggtgaaag aaatttcacc taacacggcg caatggcaga tatctataga 4620

ttccctaagt tctcatatga ggataacggt actgtggagc ccctgcctct gagaactggt 4680

ccggataaga aagccatccc ccacatcagg attgtcaagg taggagaccc tcctaaacat 4740

ggagtgagat acctagattt attgctcttg ggtttctttg agacaccgaa acaaacaacc 4800

aatctagaga gcgtatctga cttgacagag ccgaccagct actcaatatg cggctccggg 4860

tcgttaccca taggtgtggc caaatactac gggactgatc aggaactctt aaaggcctgc 4920

accgatctca gaattacggt gaggagggct gttcgagcag gagagatgat cgtatacatg 4980

gtggattcga ttggtgctcc actcctacca tggtcaggca ggctgagaca gggaatgata 5040

tttaatgcaa acaaggtcgc actagctccc caatgcctcc ctgtggacaa ggacataaga 5100

ctcagagtgg tgtttgtcaa tgggacatct ctaggggcaa tcaccatatc caagatccca 5160

aagacccttg cagaccttgc attgcccaac tctatatctg ttaatttact ggtgacactc 5220

aagaccggga tctccacaga acaaaagggg gtactcccag tacttgatga tcaaggggag 5280

aaaaagctca attttatggt gcacctcggg ttgatcagga gaaaggtcgg gaagatatac 5340

tctgttgagt actgcaagag caagattgag agaatgcggc tgattttctc acttgggtta 5400

atcggcggta taagcttcca tgttcaggtt aatgggacac tatctaagac attcatgagt 5460

cagctcgcat ggaagagggc agtctgcttc ccattaatgg atgtgaatcc ccatatgaac 5520

atggtgattt gggcggcatc tgtagaaatc acaggcgtcg atgcggtgtt ccaaccggcc 5580

atccctcgtg atttccgcta ctaccctaat gttgtggcta agaacatcgg aaggatcaga 5640

aagctgtaaa tgtgcaccca tcagagacct gcgacaatgc cccaagcaga caccacctgg 5700

cagtcggagc caccgggtca ctccttgtct taaataagaa aaacttaggg ataaagtccc 5760

ttgtgagtgc ttggttaatt aagctttcac ctcaaacaag cacagatcat ggatggtgat 5820

aggggcaaac gtgactcgta ctggtctact tctcctagtg gtagcaccac aaaaccagca 5880

tcaggttggg agaggtcaag taaagccgac acatggttgc tgattctctc attcacccag 5940

tgggctttgt caattgccac agtgatcatc tgtatcataa tttctgctag acaagggtat 6000

agtatgaaag agtactcaat gactgtagag gcattgaaca tgagcagcag ggaggtgaaa 6060

gagtcactta ccagtctaat aaggcaagag gttatagcaa gggctgtcaa cattcagagc 6120

tctgtgcaaa ccggaatccc agtcttgttg aacaaaaaca gcagggatgt catccagatg 6180

attgataagt cgtgcagcag acaagagctc actcagcact gtgagagtac gatcgcagtc 6240

caccatgccg atggaattgc cccacttgag ccacatagtt tctggagatg ccctgtcgga 6300

gaaccgtatc ttagctcaga tcctgaaatc tcattgctgc ctggtccgag cttgttatct 6360

ggttctacaa cgatctctgg atgtgttagg ctcccttcac tctcaattgg cgaggcaatc 6420

tatgcctatt catcaaatct cattacacaa ggttgtgctg acatagggaa atcatatcag 6480

gtcctgcagc tagggtacat atcactcaat tcagatatgt tccctgatct taaccccgta 6540

gtgtcccaca cttatgacat caacgacaat cggaaatcat gctctgtggt gacaacccgg 6600

actaggggtt atcagctttg ctccatgccg actgtagacg aaagaaccga ctactctagt 6660

gatggtattg aggatctggt ccttgatgtc ctggatctca aagggagaac taagtctcac 6720

cggtatcgca acagcgaggt agatcttgat cacccgttct ctgcactata ccccagtgta 6780

ggcaacggca ttgcaacaga aggctcattg atatttcttg ggtatggtgg actaaccacc 6840

cctctgcagg gtgatacaaa atgtaggacc caaggatgcc aacaggtgtc gcaagacaca 6900

tgcaatgagg ctctgaaaat tacatggcta ggagggaaac aggtggtcag cgtgatcatc 6960

caggtcaatg actatctctc agagaggcca aagataagag tcacaaccat tccaatcact 7020

caaaactatc tcggggcgga aggtagatta ttaaaattgg gtgatcgggt gtacatctat 7080

acaagatcat caggctggca ctctcaactg cagataggag tacttgatgt cagccaccct 7140

ttgactatca actggacacc tcatgaagcc ttgtctagac caggaaatga agagtgcaat 7200

tggtacaata agtgtccgaa ggaatgcata tcaggcgtat acactgatgc ttatccattg 7260

tcccctgatg cagctaacgt cgctaccgtc acgctatatg ccaatacatc gcgtgtcaac 7320

ccaacaatca tgtattctaa cactactaac attataaata tgttaaggat aaaggatgtt 7380

caattagagg ctgcatatac cacgacatcg tgtatcacgc attttggtaa aggctactgc 7440

tttcacatca tcgagatcaa tcagaagagc ctgaatacct tacagccgat gctctttaag 7500

actagcatcc ctaaattatg caaggccgag tcttaaattt aactgactag caggcttgtc 7560

ggccttgctg acactagagt catctccgaa catccacaat atctctcagt ctcttacgtc 7620

tctcacagta ttaagaaaaa cccagggtga atgggaagct tgccataggt catggatggg 7680

caggagtcct cccaaaaccc ttctgacata ctctatccag aatgccacct gaactctccc 7740

atagtcaggg ggaagatagc acagttgcac gtcttgttag atgtgaacca gccctacaga 7800

ctgaaggacg acagcataat aaatattaca aagcacaaaa ttaggaacgg aggattgtcc 7860

ccccgtcaaa ttaagatcag gtctctgggt aaggctcttc aacgcacaat aaaggattta 7920

gaccgataca cgtttgaacc gtacccaacc tactctcagg aattacttag gcttgatata 7980

ccagagatat gtgacaaaat ccgatccgtc ttcgcggtct cggatcggct gaccagggag 8040

ttatctagtg ggttccagga tctttggttg aatatcttca agcaactagg caatatagaa 8100

ggaagagagg ggtacgatcc gttgcaggat atcggcacca tcccggagat aactgataag 8160

tacagcagga atagatggta taggccattc ctaacttggt tcagcatcaa atatgacatg 8220

cggtggatgc agaagaccag accgggggga cccctcgata cctctaattc acataacctc 8280

ctagaatgca aatcatacac tctagtaaca tacggagatc ttgtcatgat actgaacaag 8340

ttgacattga cagggtatat cctaacccct gagctggtct tgatgtattg tgatgttgta 8400

gaaggaaggt ggaatatgtc tgctgcaggg catctagata agaagtccat tgggataaca 8460

agcaaaggtg aggaattatg ggaactagtg gattccctct tctcaagtct tggagaggaa 8520

atatacaatg tcatcgcact attggagccc ctatcacttg ctctcataca actaaatgat 8580

cctgttatac ctctacgtgg ggcatttatg aggcatgtgt tgacagagct acagactgtt 8640

ttaacaagta gagacgtgta cacagatgct gaagcagaca ctattgtgga gtcgttactc 8700

gccattttcc atggaacctc tattgatgag aaagcagaga tcttttcctt ctttaggaca 8760

tttggccacc ccagcttaga ggctgtcact gccgccgaca aggtaagggc ccatatgtat 8820

gcacaaaagg caataaagct taagacccta tacgagtgtc atgcagtttt ttgcactatc 8880

atcataaatg ggtatagaga gaggcatggc ggacagtggc ccccctgtga cttccctgat 8940

cacgtgtgtc tagaactaag gaacgctcaa gggtccaata cggcaatctc ttatgaatgt 9000

gctgtagaca actatacaag tttcataggc ttcaagtttc ggaagtttat agaaccacaa 9060

ctagatgaag atctcacaat atatatgaaa gacaaagcac tatcccccag gaaggaggca 9120

tgggactctg tatacccgga tagtaatctg tactataaag ccccagagtc tgaagagacc 9180

cggcggctta ttgaagtgtt cataaatgat gagaatttca acccagaaga aattatcaat 9240

tatgtggagt caggagattg gttgaaagac gaggagttca acatctcgta cagtctcaaa 9300

gagaaagaga tcaagcaaga gggtcgtcta ttcgcaaaaa tgacttataa gatgcgagcc 9360

gtacaggtgc tggcagagac actactggct aaaggaatag gagagctatt cagcgaaaat 9420

gggatggtta aaggagagat agacctactt aaaagattga ctactctttc tgtctcaggc 9480

gtccccagga ctgattcagt gtacaataac tctaaatcat cagagaagag aaacgaaggc 9540

atggaaaata agaactctgg ggggtactgg gacgaaaaga agaggtccag acatgaattc 9600

aaggcaacag attcatcaac agacggctat gaaacgttaa gttgcttcct cacaacagac 9660

ctcaagaaat actgcttaaa ctggagattt gagagtactg cattgtttgg tcagagatgc 9720

aacgagatat ttggcttcaa gaccttcttt aactggatgc atccagtcct tgaaaggtgt 9780

acaatatatg ttggagatcc ttactgtcca gtcgccgacc ggatgcatcg acaactccag 9840

gatcatgcag actctggcat tttcatacat aatcctaggg gggcatagaa aggttactgc 9900

cagaagctgt ggaccttaat ctcaatcagt gcaatccacc tagcagctgt gagagtgggt 9960

gtcagggtct ctgcaatggt tcagggtgac aatcaagcta tagccgtgac atcaagagta 10020

cctgtagctc agacttacaa gcagaagaaa aatcatgtct atgaggagat caccaaatat 10080

ttcggtgctc taagacacgt catgtttgat gtagggcacg agctaaaatt gaacgagacc 10140

atcattagta gcaagatgtt tgtctatagt aaaaggatat actatgatgg gaagatttta 10200

ccacagtgcc tgaaagcctt gaccaagtgt gtattctggt ccgagacact ggtagatgaa 10260

aacagatctg cttgttcgaa catctcaaca tccatagcaa aagctatcga aaatgggtat 10320

tctcctatac taggctactg cattgcgttg tataagacct gtcagcaggt gtgcatatca 10380

ctagggatga ctataaatcc aactatcagc ccgaccgtaa gagatcaata ct[?]taagggt 10440

aagaattggc tgagatgtgc agtgttgatt ccagcaaatg ttggaggatt caactacatg 10500

tctacatcta gatgctttgt tagaaatatt ggagaccccg cagtagcagc cctagctgat 10560

ctcaaaagat tcatcagagc ggatctgtta gacaagcagg tattatacag ggtcatgaat 10620

caagaacccg gtgactctag ttttctagat tgggcttcag acccttattc gtgtaacctc 10680

ccgcattctc agagtataac tacgattata aagaatatca ctgctagatc tgtgctgcag 10740

gaatccccga atcctctact gtctggtctc ttcaccgaga ctagtggaga agaggatctc 10800

aacctggcct cgttccttat ggaccggaaa gtcatcctgc cgagagtggc tcatgagatc 10860

ctgggtaatt ccttaactgg agttagggag gcgattgcag ggatgcttga tacgaccaag 10920

tctctagtga gagccagcgt taggaaagga ggattatcat atgggatatt gaggaggctt 10980

gtcaattatg atctattgca gtacgagaca ctgactagaa ctctcaggaa accggtgaaa 11040

gacaacatcg aatatgagta tatgtgttca gttgagctag ctgtcggtct aaggcagaaa 11100

atgtggatcc acctgactta cgggagaccc atacatgggc tagaaacacc agacccttta 11160

gagctcttga ggggaatatt tatcgaaggt tcagaggtgt gcaagctttg caggtctgaa 11220

ggagcagacc ccatctatac atggttctat cttcctgact ctatagacct ggacacgctt 11280

acaaacggat gtccggctat aagaatcccc tattttggat cagccactga tgaaaggtcg 11340

gaagcccaac tcgggtatgt aagaaatcta agcaaacccg caaaggcggc catccggata 11400

gctatggtgt atacgtgggc ctacgggact gatgagatat cgtggatgga agccgctctt 11460

atagcccaaa caagagctaa tctgagctta gagaatctaa agctgctgac tcctgtttca 11520

acctccacta atctatctca taggttgaaa gatacggcaa cccagatgaa gttctctagt 11580

gcaacactag tccgtgcaag tcggttcata acaatatcaa atgataacat ggcactcaaa 11640

gaagcagggg agtcgaagga tactaatctc gtgtatcagc agattatgct aactgggcta 11700

agcttgttcg agttcaatat gagatataag aaaggttcct tagggaagcc actgatattg 11760

cacttacatc ttaataacgg gtgctgtata atggagtccc cacaggaggc gaatatcccc 11820

ccaaggtcca cattagattt agagattaca caagagaaca ataaattgat ctatgatcct 11880

gatccactca aggatgtgga ccttgagcta tttagcaagg tcagagatgt tgtacacaca 11940

gttgacatga cttattggtc agatgatgaa gttatcagag caaccagtat ctgtactgca 12000

atgacgatag ctgatacaat gtctcaatta gatagagaca acttaaaaga gatgatcgca 12060

ctagtaaatg acgatgatgt caacagcttg attactgagt ttatggtgat tgatgttcct 12120

ttattttgct caacgttcgg gggtattcta gtcaatcagt ttgcatactc actctacggc 12180

ttaaacatca gaggaaggga agaaatatgg ggacatgtag tccggattct taaagatacc 12240

tcccacgcag ttttaaaagt cttatctaat gctctatctc atcccaaaat cttcaaacga 12300

ttctggaatg caggtgtcgt ggaacctgtg tatgggccta acctctcaaa tcaggataag 12360

atactcttgg ccctctctgt ctgtgaatat tctgtggatc tattcatgca cgattggcaa 12420

gggggtgtac cgcttgagat ctttatctgt gacaatgacc cagatgtggc cgacatgagg 12480

aggtcctctt tcttggcaag acatcttgca tacctatgca gcttggcaga gatatctagg 12540

gatgggccaa gattagaatc aatgaactct ctagagaggc tcgagtcact aaagagttac 12600

ctggaactca catttcttga tgacccggta ctgaggtaca gtcagttgac tggcctagtc 12660

atcaaagtat tcccatctac tttgacctat atccggaagt catctataaa agtgttaagg 12720

acaagaggta taggagtccc tgaagtctta gaagattggg atcccgaggc agataatgca 12780

ctgttagatg gtatcgcggc agaaatacaa cagaatattc ctttgggaca tcagactaga 12840

gccccttttt gggggttgag agtatccaag tcacaggtac tgcgtctccg ggggtacaag 12900

gagatcacaa gaggtgagat aggcagatca ggtgttggtc tgacgttacc attcgatgga 12960

agatatctat ctcaccagct gaggctcttt ggcatcaaca gtactagctg cttgaaagca 13020

cttgaactta cctacctatt gagcccctta gttgacgaag ataaagatag gctatattta 13080

ggggaaggag ctggggccat gctttcctgt tatgacgcta ctcttggccc atgcatcaac 13140

tattataact caggggtata ctcttgtgat gtcaatgggc agagagagtt aaatatatat 13200

cctgctgagg tggcactagt gggaaagaaa ttaaacaatg ttactagtct gggtcaaaga 13260

gttaaagtgt tattcaacgg gaatcctggc tcgacatgga ttgggaatga tgagtgtgag 13320

gctttgattt ggaatgaatt acagaatagc tcgataggcc tagtccactg tgacatggag 13380

ggaggagatc ataaggatga tcaagttgta ctgcatgagc attacagtgt aatccggatc 13440

gcgtatctgg tgggggatcg agacgttgtg cttataagca agattgctcc caggctgggc 13500

acggattgga ccaggcagct cagcctatat ctgagatact gggacgaggt taacctaata 13560

gtgcttaaaa catctaaccc tgcttccaca gagatgtatc tcctatcgag gcaccccaaa 13620

tctgacatta tagaggacag caagacagtg ttagctagtc tcctcccttt gtcaaaagaa 13680

gatagcatca agatagaaaa gtggatctta atagagaagg caaaggctca cgaatgggtt 13740

actcgggaat tgagagaagg aagctcttca tcagggatgc ttagacctta ccatcaagca 13800

ctgcagacgt ttggctttga accaaacttg tataaattga gcagagattt cttgtccacc 13860

atgaacatag ctgatacaca caactgcatg atagctttca acagggtttt gaaggataca 13920

atcttcgaat gggctagaat aactgagtca gataaaaggc ttaaactaac tggtaagtat 13980

gacctgtatc ctgtgagaga ttcaggcaag ttgaagacaa tttctagaag acttgtgcta 14040

tcttggatat ctttatctat gtccacaaga ttggtaactg ggtcattccc tgaccagaag 14100

tttgaagcaa gacttcaatt gggaatagtt tcattatcat cccgtgaaat caggaacctg 14160

agggttatca caaaaacttt attagacagg tttgaggata ttatacatag tataacgtat 14220

agattcctca ccaaagaaat aaagattttg atgaagattt taggggcagt caagatgttc 14280

ggggccaggc aaaatgaata cacgaccgtg attgatgatg gatcactagg tgatatcgag 14340

ccatatgaca gctcgtaata attagtccct atcgtgcaga acgatcgaag ctccgcggta 14400

cctggaagtc ttggacttgt ccatatgaca atagtaagaa aaacttacaa gaagacaaga 14460

aaatttaaaa ggatacatat ctcttaaact cttgtctggt gggtcggcat ggcatctcca 14520

cctcctcgcg gtccgacctg ggcatccgaa ggaggacgca cgtccactcg gatggctaag 14580

ggagagctac gcgtggatcc ccgggaattc cgtaatcatg gtcatagctg tttcctgtgt 14640

gaaattgtta tccgctcaca attccacaca acatacgagc cggaagcata aagtgtaaag 14700

cctggggtgc ctaatgagtg agctaactca cattaattgc gttgcgctca ctgcccgctt 14760

tccagtcggg aaacctgtcg tgccagctgc attaatgaat cggccaacgc gcggggagag 14820

gcggtttgcg tattgggcgc tcttccgctt cctcgctcac tgactcgctg cgctcggtcg 14880

ttcggctgcg gcgagcggta tcagctcact caaaggcggt aatacggtta tccacagaat 14940

caggggataa cgcaggaaag aacatgtgag caaaaggcca gcaaaaggcc aggaaccgta 15000

aaaaggccgc gttgctggcg tttttccata ggctccgccc ccctgacgag catcacaaaa 15060

atcgacgctc aagtcagagg tggcgaaacc cgacaggact ataaagatac caggcgtttc 15120

cccctggaag ctccctcgtg cgctctcctg ttccgaccct gccgcttacc ggatacctgt 15180

ccgcctttct cccttcggga agcgtggcgc tttctcatag ctcacgctgt aggtatctca 15240

gttcggtgta ggtcgttcgc tccaagctgg gctgtgtgca cgaacccccc gttcagcccg 15300

accgctgcgc cttatccggt aactatcgtc ttgagtccaa cccggtaaga cacgacttat 15360

cgccactggc agcagccact ggtaacagga ttagcagagc gaggtatgta ggcggtgcta 15420

cagagttctt gaagtggtgg cctaactacg gctacactag aagaacagta tttggtatct 15480

gcgctctgct gaagccagtt accttcggaa aaagagttgg tagctcttga tccggcaaac 15540

aaaccaccgc tggtagcggt ggtttttttg tttgcaagca gcagattacg cgcagaaaaa 15600

aaggatctca agaagatcct ttgatctttt ctacggggtc tgacgctcag tggaacgaaa 15660

actcacgtta agggattttg gtcatgagat tatcaaaaag gatcttcacc tagatccttt 15720

taaattaaaa atgaagtttt aaatcaatct aaagtatata tgagtaaact tggtctgaca 15780

gttaccaatg cttaatcagt gaggcaccta tctcagcgat ctgtctattt cgttcatcca 15840

tagttgcctg actccccgtc gtgtagataa ctacgatacg ggagggctta ccatctggcc 15900

ccagtgctgc aatgataccg cgagacccac gctcaccggc tccagattta tcagcaataa 15960

accagccagc cggaagggcc gagcgcagaa gtggtcctgc aactttatcc gcctccatcc 16020

agtctattaa ttgttgccgg gaagctagag taagtagttc gccagttaat agtttgcgca 16080

acgttgttgc cattgctaca ggcatcgtgg tgtcacgctc gtcgtttggt atggcttcat 16140

tcagctccgg ttcccaacga tcaaggcgag ttacatgatc ccccatgttg tgcaaaaaag 16200

cggttagctc cttcggtcct ccgatcgttg tcagaagtaa gttggccgca gtgttatcac 16260

tcatggttat ggcagcactg cataattctc ttactgtcat gccatccgta agatgctttt 16320

ctgtgactgg tgagtactca accaagtcat tctgagaata gtgtatgcgg cgaccgagtt 16380

gctcttgccc ggcgtcaata cgggataata ccgcgccaca tagcagaact ttaaaagtgc 16440

tcatcattgg aaaacgttct tcggggcgaa aactctcaag gatcttaccg ctgttgagat 16500

ccagttcgat gtaacccact cgtgcaccca actgatcttc agcatctttt actttcacca 16560

gcgtttctgg gtgagcaaaa acaggaaggc aaaatgccgc aaaaaaggga ataagggcga 16620

cacggaaatg ttgaatactc atactcttcc tttttcaata ttattgaagc atttatcagg 16680

gttattgtct catgagcgga tacatatttg aatgtattta gaaaaataaa caaatagggg 16740

ttccgcgcac atttccccga aaagtgccac ctgacgtcta agaaaccatt attatcatga 16800

cattaaccta taaaaatagg cgtatcacga ggccctttcg tc 16842

<210> 22
<211> 282
<212> DNA
<213> Mus musculus

<400> 22
atgaggtcac ttcagatgct gctcctggct gctctgcttc tggggacttt tctgcagcat 60

gccagagctg ctcgagccac caatgtaggc cgagagtgct gcctggatta cttcaaaggg 120

gccattccta tcaggaagtt ggtgagctgg tataagacct cagtggagtg ttccagggat 180

gccatcgtgt ttctgactgt ccagggcaag ctcatctgtg cagaccccaa agacaaacat 240

gtgaagaagg ccatcagatt ggtgaaaaac ccaaggccat ga 282

<210> 23
<211> 276
<212> DNA
<213> Mus musculus

<400> 23
atgaagatct ctgcagctgc cctcaccatc atcctcactg cagccgccct ctgcaccccc 60

gcacctgcct caccatatgg ctcggacacc actccctgct gctttgccta cctctccctc 120

gcgctgcctc gtgcccacgt caaggagtat ttctacacca gcagcaagtg ctccaatctt 180
gcagtcgtgt ttgtcactcg aaggaaccgc caagtgtgtg ccaacccaga gaagaagtgg 240
gttcaagaat acatcaacta tttggagatg agctag 276

SEQUENCE LISTING

<110> KYUSHU UNIVERSITY, NATIONAL UNIVERSITY CORPORATION
DNAVEC CORPORATION

<120> Viral vector for gene therapy

<130> D4-A0703P

<150> JP 2007-119130
<151> 2007-04-27

<160> 23

<170> PatentIn version 3.4

<210> 1
<211> 10
<212> RNA
<213> Sendai virus

<400> 1
cmuuvmcccu 10

<210> 2
<211> 10
<212> RNA
<213> Sendai virus

<400> 2
cuuugacccu 10

<210> 3
<211> 10
<212> RNA
<213> Sendai virus

<400> 3
cauucacccu 10

<210> 4
<211> 10
<212> RNA
<213> Sendai virus

<400> 4
cuuucacccu 10

<210> 5
<211> 10
<212> DNA
<213> Sendai virus

<400> 5
agggtcaaag 10

<210> 6
<211> 10
<212> DNA
<213> Sendai virus

<400> 6
agggtgaatg 10

<210> 7
<211> 10
<212> DNA
<213> Sendai virus

<400> 7
agggtgaaag 10

<210> 8
<211> 9
<212> RNA
<213> Sendai virus

<400> 8
uuuuucuua 9

<210> 9
<211> 9
<212> DNA
<213> Sendai virus

<400> 9
taagaaaaa 9

<210> 10
<211> 10
<212> DNA
<213> Sendai virus

<400> 10
ctttcaccct 10

<210> 11
<211> 15
<212> DNA
<213> Sendai virus

<400> 11
tttttcttac tacgg 15

<210> 12
<211> 18
<212> DNA
<213> Artificial

<220>
<223> An artificially synthesized nucleotide sequence

<400> 12
atgcatgccg gcagatga 18

<210> 13
<211> 18
<212> DNA
<213> Artificial

<220>
<223> An artificially synthesized primer sequence

<400> 13
gttgagtact gcaagagc 18

<210> 14
<211> 42
<212> DNA
<213> Artificial

<220>
<223> An artificially synthesized primer sequence

<400> 14
tttgccggca tgcatgtttc ccaaggggag agttttgcaa cc 42


<210> 15
<211> 18
<212> DNA
<213> Artificial

<220>
<223> An artificially synthesized primer sequence

<400> 15
atgcatgccg gcagatga 18


<210> 16
<211> 21
<212> DNA
<213> Artificial

<220>
<223> An artificially synthesized primer sequence

<400> 16
tgggtgaatg agagaatcag c 21


<210> 17
<211> 426
<212> DNA
<213> Sendai virus

<400> 17
atgtggctgc agaatttact tttcctgggc attgtggtct acagcctctc agcacccacc 60

cgctcaccca tcactgtcac ccggccttgg aagcatgtag aggccatcaa agaagccctg 120

aacctcctgg atgacatgcc tgtcacattg aatgaagagg tagaagtcgt ctctaacgag 180

ttctccttca agaagctaac atgtgtgcag acccgcctga agatattcga gcagggtcta 240

cggggcaatt tcaccaaact caagggcgcc ttgaacatga cagccagcta ctaccagaca 300

tactgccccc caactccgga aacggactgt gaaacacaag ttaccaccta tgcggatttc 360

atagacagcc ttaaaacctt tctgactgat atcccctttg aatgcaaaaa accagtccaa 420

aaatga 426


<210> 18
<211> 48
<212> DNA
<213> Artificial

<220>
<223> An artificially synthesized primer sequence

<400> 18

attgcggccg ccaaggttca cttatgtggc tgcagaattt acttttcc 48

<210> 19
<211> 78
<212> DNA
<213> Artificial

<220>
<223> An artificially synthesized primer sequence

<400> 19
attgcggccg cgatgaactt tcaccctaag tttttcttac tacggtcatt tttggactgg 60

tttttgcat tcaaaggg 78

<210> 20
<211> 485
<212> DNA
<213> Artificial

<220>
<223> An artificially synthesized nucleotide sequence

<220>
<221> misc_feature
<222> (1)..(17)
<223> Transcription signal of Sendai Virus

<220>
<221> misc_feature
<222> (444)..(485)
<223> Transcription signal of Sendai Virus

<400> 20
gcggccgcca aagttcaatg tggctgcaga atttactttt cctgggcatt gtggtctaca 60

gcctctcagc acccacccgc tcacccatca ctgtcacccg gccttggaag catgtagagg 120

ccatcaaaga agccctgaac ctcctggatg acatgcctgt cacattgaat gaagaggtag 180

aagtcgtctc taacgagttc tccttcaaga agctaacatg tgtgcagacc cgcctgaaga 240

tattcgagca gggtctacgg ggcaatttca ccaaactcaa gggcgccttg aacatgacag 300

ccagctacta ccagacatac tgccccccaa ctccggaaac ggactgtgaa acacaagtta 360

ccacctatgc ggatttcata gacagcctta aaacctttct gactgatatc ccctttgaat 420

gcaaaaaacc agtccaaaaa tgaccgtagt aagaaaaact tagggtgaaa gttcatcgcg 480

gccgc 485

<210> 21
<211> 16842
<212> DNA
<213> Artificial

<220>
<223> An artificially synthesized vector sequence

<400> 21
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180
accatatgcg gtgtgaaata ccgcacagat gcgtaaggag aaaataccgc atcaggcgcc 240
attcgccatt caggctgcgc aactgttggg aagggcgatc ggtgcgggcc tcttcgctat 300
tacgccagct ggcgaaaggg ggatgtgctg caaggcgatt aagttgggta acgccagggt 360
tttcccagtc acgacgttgt aaaacgacgg ccagtgccaa gctcgcgtcg tacgggccct 420
aatacgactc actataacca aacaagagaa aaacatgtat gggatatgt aatgaagtta 480
tacaggattt tagggtcaaa gtatccaccc tgaggagcag gttccagacc ctttgctttg 540
ctgccaaagt tcacgcggcc gccaaggttc acttatgtgg ctgcagaatt tacttttcct 600
gggcattgtg gtctacagcc tctcagcacc cacccgctca cccatcactg tcacccggcc 660
ttggaagcat gtagaggcca tcaaagaagc cctgaacctc ctggatgaca tgcctgtcac 720
attgaatgaa gaggtagaag tcgtctctaa cgagttctcc ttcaagaagc taacatgtgt 780
gcagacccgc ctgaagatat tcgagcaggg tctacggggc aatttcacca aactcaaggg 840
cgccttgaac atgacagcca gctactacca gacatactgc cccccaactc cggaaacgga 900
ctgtgaaaca caagttacca cctatgcgga tttcatagac agccttaaaa cctttctgac 960
tgatatcccc tttgaatgca aaaaaccagt ccaaaaatga ccgtagtaag aaaaacttag 1020
ggtgaaagtt catcgcggcc gcagatcttc acgatggccg ggttgttgag caccttcgat 1080
acatttagct ctaggaggag cgaaagtatt aataagtcgg gaggaggtgc tgttatcccc 1140
ggccagagga gcacagtctc agtgttcgta ctaggcccaa gtgtgactga tgatgcagac 1200
aagttattca ttgcaactac cttcctagct cactcattgg acacagataa gcagcactct 1260
cagagagggg ggttcctcgt ctctctgctt gccatggctt acagtagtcc agaattgtac 1320
ttgacaacaa acggagtaaa cgccgatgtc aaatatgtga tctacaacat agagaaagac 1380
cctaagagga cgaagacaga cggattcatt gtgaagacga gagatatgga atatgagagg 1440
accacagaat ggctgtttgg acctatggtc aacaagagcc cactcttcca gggtcaacgg 1500
gatgctgcag accctgacac actccttcaa atctatgggt atcctgcatg cctaggagca 1560
ataattgtcc aagtctggat tgtgctggtg aaggccatca caagcagcgc cggcttaagg 1620
aaagggttct tcaacaggtt agaggcgttc agacaagacg gcaccgtgaa aggtgcctta 1680
gttttcactg gggagacagt tgaggggata ggctcggtta tgagatctca gcaaagcctt 1740
gtatctctca tggttgagac ccttgtgact atgaatactg caagatctga tctcaccaca 1800
ttagagaaga acatccagat cgttgggaac tacatccgag atgcagggct ggcttccttc 1860
atgaacacta ttaaatatgg ggtggaaaca aagatggcag ctctaacgtt gtcaaacctg 1920
aggcccgata ttaataagct tagaagcctc atagacacct acctgtcaaa ggccccagag 1980
gctcccttta tctgtatcct caaggaccct gttcatggtg aatttgctcc aggcaattat 2040
cctgcactat ggagttacgc catgggagtc gccgtcgtac agaacaaggc aatgcagcag 2100
tacgtcacag ggaggacata ccttgatatg gaaatgttct tactaggaca agccgtggca 2160
aaggatgctg aatcgaagat cagcagtgcc ttggaagatg agttaggagt gacggataca 2220

gccaagggga ggctcagaca tcatctggca aacttgtccg gtggggatgg tgcttaccac 2280
aaaccaacag gcggtggtgc aattgaggta gctctagaca atgccgacat cgacctagaa 2340
acaaaagccc atgcggacca ggacgctagg ggttggggtg gagatagtgg tgaaagatgg 2400
gcacgtcagg tgagtggtgg ccactttgtc acactacatg gggctgaacg gttagaggag 2460
gaaaccaatg atgaggatgt atcagacata gagagaagaa tagccatgag actcgcagag 2520
agacggcaag aggattctgc aacccatgga gatgaaggcc gcaataacgg tgtcgatcat 2580
gacgaagatg acgatgccgc agcagtagct gggataggag gaatctagga tcatacgagg 2640
cttcaaggta cttgatccgt agtaagaaaa acttagggtg aaagttcatc caccgatcgg 2700
ctcaggcaag gccacaccca accccaccga ccacacccag cagtcgagac agccacggct 2760
tcggctacac ttaccgcatg gatcaagatg ccttcattct taaagaagat tctgaagttg 2820
agagggaggc gccaggagga cgagagtcgc tctcggatgt tatcggattc ctcgatgctg 2880
tcctgtcgag tgaaccaact gacatcggag gggacagaag ctggctccac aacaccatca 2940
acactcccca aggaccaggc tctgctcata gagccaaaag tgagggcgaa ggagaagtct 3000
caacaccgtc gacccaagat aatcgatcag gtgaggagag tagagtctct gggagaacaa 3060
gcaagccaga ggcagaagca catgctggaa accttgataa acaaaatata caccgggcct 3120
ttgggggaag aactggtaca aactctgtat ctcaggatct gggcgatgga ggagactccg 3180
gaatccttga aaatcctcca aatgagagag gatatccgag atcaggtatt gaagatgaaa 3240
acagagagat ggctgcgcac cctgataaga ggggagaaga ccaagctgaa ggacttccag 3300
aagaggtacg aggaagtaca tccctacctg atgaaggaga aggtggagca agtaataatg 3360
gaagaagcat ggagcctggc agctcacata gtgcaagagt aactggggtc ctggtgattc 3420
ctagccccga acttgaagag gctgtgctac ggaggaacaa aagaagacct accaacagtg 3480
ggtccaaacc tcttactcca gcaaccgtgc ctggcacccg gtccccaccg ctgaatcgtt 3540
acaacagcac agggtcacca ccaggaaaac ccccatctac acaggatgag cacatcaact 3600
ctggggacac ccccgccgtc agggtcaaag accggaaacc accaataggg acccgctctg 3660
tctcagattg tccagccaac ggccgcccaa tccacccggg tctagagacc gactcaacaa 3720
aaaagggcat aggagagaac acatcatcta tgaaagagat ggctacattg ttgacgagtc 3780
ttggtgtaat ccagtctgct caagaattcg aatcatcccg agacgcgagt tatgtgtttg 3840
caagacgtgc cctaaagtct gcaaactatg cagagatgac attcaatgta tgcggcctga 3900
tcctttctgc cgagaaatct tccgctcgta aggtagatga gaacaaacaa ctgctcaaac 3960
agatccaaga gagcgtggaa tcattccggg atatttacaa gagattctct gagtatcaga 4020
aagaacagaa ctcattgctg atgtccaacc tatctacact tcatatcatc acagatagag 4080
gtggcaagac tgacaacaca gactccctta caaggtcccc ctccgttttt gcaaaatcaa 4140
aagagaacaa gactaaggct accaggtttg acccatctat ggagacccta gaagatatga 4200
agtacaaacc ggacctaatc cgagaggatg aatttagaga tgagatccgc aacccggtgt 4260

accaagagag ggacacagaa cccagggcct caaacgcatc acgtctcttt ccctccaaag 4320
agaagcccac aatgcactct ctcaggctcg tcatagagag cagtccccta agcagagctg 4380
agaaagtagc atatgtgaaa tcattatcca agtgcaagac agaccaagag gttaaggcag 4440
tcatggaact cgtagaagag gacatagagt cactgaccaa ctagatcccg ggtgaggcat 4500
cctaccatcc tcagtcatag agagatccaa tctaccatca gcatcagcca gtaaagatta 4560
agaaaaactt agggtgaaag aaatttcacc taacacggcg caatggcaga tatctataga 4620
ttccctaagt tctcatatga ggataacggt actgtggagc ccctgcctct gagaactggt 4680
ccggataaga aagccatccc ccacatcagg attgtcaagg taggagaccc tcctaaacat 4740
ggagtgagat acctagattt attgctcttg ggtttctttg agacaccgaa acaaacaacc 4800
aatctagaga gcgtatctga cttgacagag ccgaccagct actcaatatg cggctccggg 4860
tcgttaccca taggtgtggc caaatactac gggactgatc aggaactctt aaaggcctgc 4920
accgatctca gaattacggt gaggagggct gttcgagcag gagagatgat cgtatacatg 4980
gtggattcga ttggtgctcc actcctacca tggtcaggca ggctgagaca gggaatgata 5040
tttaatgcaa acaaggtcgc actagctccc caatgcctcc ctgtggacaa ggacataaga 5100
ctcagagtgg tgtttgtcaa tgggacatct ctaggggcaa tcaccatatc caagatccca 5160
aagacccttg cagaccttgc attgcccaac tctatatctg ttaatttact ggtgacactc 5220
aagaccggga tctccacaga acaaaagggg gtactcccag tacttgatga tcaaggggag 5280
aaaaagctca attttatggt gcacctcggg ttgatcagga gaaaggtcgg gaagatatac 5340
tctgttgagt actgcaagag caagattgag agaatgcggc tgattttctc acttgggtta 5400
atcggcggta taagcttcca tgttcaggtt aatgggacac tatctaagac attcatgagt 5460
cagctcgcat ggaagagggc agtctgcttc ccattaatgg atgtgaatcc ccatatgaac 5520
atggtgattt gggcggcatc tgtagaaatc acaggcgtcg atgcggtgtt ccaaccggcc 5580
atccctcgtg atttccgcta ctaccctaat gttgtggcta agaacatcgg aaggatcaga 5640
aagctgtaaa tgtgcaccca tcagagacct gcgacaatgc cccaagcaga caccacctgg 5700
cagtcggagc caccgggtca ctccttgtct taaataagaa aaacttaggg ataaagtccc 5760
ttgtgagtgc ttggttaatt aagctttcac ctcaaacaag cacagatcat ggatggtgat 5820
aggggcaaac gtgactcgta ctggtctact tctcctagtg gtagcaccac aaaaccagca 5880
tcaggttggg agaggtcaag taaagccgac acatggttgc tgattctctc attcacccag 5940
tgggctttgt caattgccac agtgatcatc tgtatcataa tttctgctag acaagggtat 6000
agtatgaaag agtactcaat gactgtagag gcattgaaca tgagcagcag ggaggtgaaa 6060
gagtcactta ccagtctaat aaggcaagag gttatagcaa gggctgtcaa cattcagagc 6120
tctgtgcaaa ccggaatccc agtcttgttg aacaaaaaca gcagggatgt catccagatg 6180
attgataagt cgtgcagcag acaagagctc actcagcact gtgagagtac gatcgcagtc 6240
caccatgccg atggaattgc cccacttgag ccacatagtt tctggagatg ccctgtcgga 6300
gaaccgtatc ttagctcaga tcctgaaatc tcattgctgc ctggtccgag cttgttatct 6360

ggttctacaa cgatctctgg atgtgttagg ctcccttcac tctcaattgg cgaggcaatc 6420
tatgcctatt catcaaatct cattacacaa ggttgtgctg acatagggaa atcatatcag 6480
gtcctgcagc tagggtacat atcactcaat tcagatatgt tccctgatct taaccccgta 6540
gtgtcccaca cttatgacat caacgacaat cggaaatcat gctctgtggt gacaacccgg 6600
actaggggtt atcagctttg ctccatgccg actgtagacg aaagaaccga ctactctagt 6660
gatggtattg aggatctggt ccttgatgtc ctggatctca aagggagaac taagtctcac 6720
cggtatcgca acagcgaggt agatcttgat cacccgttct ctgcactata ccccagtgta 6780
ggcaacggca ttgcaacaga aggctcattg atatttcttg ggtatggtgg actaaccacc 6840
cctctgcagg gtgatacaaa atgtaggacc caaggatgcc aacaggtgtc gcaagacaca 6900
tgcaatgagg ctctgaaaat tacatggcta ggagggaaac aggtggtcag cgtgatcatc 6960
caggtcaatg actatctctc agagaggcca aagataagag tcacaaccat tccaatcact 7020
caaaactatc tcggggcgga aggtagatta ttaaaattgg gtgatcgggt gtacatctat 7080
acaagatcat caggctggca ctctcaactg cagataggag tacttgatgt cagccaccct 7140
ttgactatca actggacacc tcatgaagcc ttgtctagac caggaaatga agagtgcaat 7200
tggtacaata agtgtccgaa ggaatgcata tcaggcgtat acactgatgc ttatccattg 7260
tcccctgatg cagctaacgt cgctaccgtc acgctatatg ccaatacatc gcgtgtcaac 7320
ccaacaatca tgtattctaa cactactaac attataaata tgttaaggat aaaggatgtt 7380
caattagagg ctgcatatac cacgacatcg tgtatcacgc attttggtaa aggctactgc 7440
tttcacatca tcgagatcaa tcagaagagc ctgaatacct tacagccgat gctctttaag 7500
actagcatcc ctaaattatg caaggccgag tcttaaattt aactgactag caggcttgtc 7560
ggccttgctg acactagagt catctccgaa catccacaat atctctcagt ctcttacgtc 7620
tctcacagta ttaagaaaaa cccagggtga atgggaagct tgccataggt catggatggg 7680
caggagtcct cccaaaaccc ttctgacata ctctatccag aatgccacct gaactctccc 7740
atagtcaggg ggaagatagc acagttgcac gtcttgttag atgtgaacca gccctacaga 7800
ctgaaggacg acagcataat aaatattaca aagcacaaaa ttaggaacgg aggattgtcc 7860
ccccgtcaaa ttaagatcag gtctctgggt aaggctcttc aacgcacaat aaaggattta 7920
gaccgataca cgtttgaacc gtacccaacc tactctcagg aattacttag gcttgatata 7980
ccagagatat gtgacaaaat ccgatccgtc ttcgcggtct cggatcggct gaccagggag 8040
ttatctagtg ggttccagga tctttggttg aatatcttca agcaactagg caatatagaa 8100
ggaagagagg ggtacgatcc gttgcaggat atcggcacca tcccggagat aactgataag 8160
tacagcagga atagatggta taggccattc ctaacttggt tcagcatcaa atatgacatg 8220
cggtggatgc agaagaccag accgggggga cccctcgata cctctaattc acataacctc 8280
ctagaatgca aatcatacac tctagtaaca tacggagatc ttgtcatgat actgaacaag 8340
ttgacattga cagggtatat cctaacccct gagctggtct tgatgtattg tgatgttgta 8400

gaaggaaggt ggaatatgtc tgctgcaggg catctagata agaagtccat tgggataaca 8460
agcaaaggtg aggaattatg ggaactagtg gattccctct tctcaagtct tggagaggaa 8520
atatacaatg tcatcgcact attggagccc ctatcacttg ctctcataca actaaatgat 8580
cctgttatac ctctacgtgg ggcatttatg aggcatgtgt tgacagagct acagactgtt 8640
ttaacaagta gagacgtgta cacagatgct gaagcagaca ctattgtgga gtcgttactc 8700
gccattttcc atggaacctc tattgatgag aaagcagaga tcttttcctt ctttaggaca 8760
tttggccacc ccagcttaga ggctgtcact gccgccgaca aggtaagggc ccatatgtat 8820
gcacaaaagg caataaagct taagacccta tacgagtgtc atgcagtttt ttgcactatc 8880
atcataaatg ggtatagaga gaggcatggc ggacagtggc ccccctgtga cttccctgat 8940
cacgtgtgtc tagaactaag gaacgctcaa gggtccaata cggcaatctc ttatgaatgt 9000
gctgtagaca actatacaag tttcataggc ttcaagtttc ggaagtttat agaaccacaa 9060
ctagatgaag atctcacaat atatatgaaa gacaaagcac tatcccccag gaaggaggca 9120
tgggactctg tatacccgga tagtaatctg tactataaag ccccagagtc tgaagagacc 9180
cggcggctta ttgaagtgtt cataaatgat gagaatttca acccagaaga aattatcaat 9240
tatgtggagt caggagattg gttgaaagac gaggagttca acatctcgta cagtctcaaa 9300
gagaaagaga tcaagcaaga gggtcgtcta ttcgcaaaaa tgacttataa gatgcgagcc 9360
gtacaggtgc tggcagagac actactggct aaaggaatag gagagctatt cagcgaaaat 9420
gggatggtta aaggagagat agacctactt aaaagattga ctactctttc tgtctcaggc 9480
gtccccagga ctgattcagt gtacaataac tctaaatcat cagagaagag aaacgaaggc 9540
atggaaaata agaactctgg ggggtactgg gacgaaaaga agaggtccag acatgaattc 9600
aaggcaacag attcatcaac agacggctat gaaacgttaa gttgcttcct cacaacagac 9660
ctcaagaaat actgcttaaa ctggagattt gagagtactg cattgtttgg tcagagatgc 9720
aacgagatat ttggcttcaa gaccttcttt aactggatgc atccagtcct tgaaaggtgt 9780
acaatatatg ttggagatcc ttactgtcca gtcgccgacc ggatgcatcg acaactccag 9840
gatcatgcag actctggcat tttcatacat aatcctaggg gggcatagaa aggttactgc 9900
cagaagctgt ggaccttaat ctcaatcagt gcaatccacc tagcagctgt gagagtgggt 9960
gtcagggtct ctgcaatggt tcagggtgac aatcaagcta tagccgtgac atcaagagta 10020
cctgtagctc agacttacaa gcagaagaaa aatcatgtct atgaggagat caccaaatat 10080
ttcggtgctc taagacacgt catgtttgat gtagggcacg agctaaaatt gaacgagacc 10140
atcattagta gcaagatgtt tgtctatagt aaaaggatat actatgatgg gaagatttta 10200
ccacagtgcc tgaaagcctt gaccaagtgt gtattctggt ccgagacact ggtagatgaa 10260
aacagatctg cttgttcgaa catctcaaca tccatagcaa aagctatcga aaatgggtat 10320
tctcctatac taggctactg cattgcgttg tataagacct gtcagcaggt gtgcatatca 10380
ctagggatga ctataaatcc aactatcagc ccgaccgtaa gagatcaata ctttaagggt 10440
aagaattggc tgagatgtgc agtgttgatt ccagcaaatg ttggaggatt caactacatg 10500

tctacatcta gatgctttgt tagaaatatt ggagaccccg cagtagcagc cctagctgat 10560
ctcaaaagat tcatcagagc ggatctgtta gacaagcagg tattatacag ggtcatgaat 10620
caagaacccg gtgactctag ttttctagat tgggcttcag acccttattc gtgtaacctc 10680
ccgcattctc agagtataac tacgattata aagaatatca ctgctagatc tgtgctgcag 10740
gaatccccga atcctctact gtctggtctc ttcaccgaga ctagtggaga agaggatctc 10800
aacctggcct cgttccttat ggaccggaaa gtcatcctgc cgagagtggc tcatgagatc 10860
ctgggtaatt ccttaactgg agttagggag gcgattgcag ggatgcttga tacgaccaag 10920
tctctagtga gagccagcgt taggaaagga ggattatcat atgggatatt gaggaggctt 10980
gtcaattatg atctattgca gtacgagaca ctgactagaa ctctcaggaa accggtgaaa 11040
gacaacatcg aatatgagta tatgtgttca gttgagctag ctgtcggtct aaggcagaaa 11100
atgtggatcc acctgactta cgggagaccc atacatgggc tagaaacacc agacccttta 11160
gagctcttga ggggaatatt tatcgaaggt tcagaggtgt gcaagctttg caggtctgaa 11220
ggagcagacc ccatctatac atggttctat cttcctgact ctatagacct ggacacgctt 11280
acaaacggat gtccggctat aagaatcccc tattttggat cagccactga tgaaaggtcg 11340
gaagcccaac tcgggtatgt aagaaatcta agcaaacccg caaaggcggc catccggata 11400
gctatggtgt atacgtgggc ctacgggact gatgagatat cgtggatgga agccgctctt 11460
atagcccaaa caagagctaa tctgagctta gagaatctaa agctgctgac tcctgtttca 11520
acctccacta atctatctca taggttgaaa gatacggcaa cccagatgaa gttctctagt 11580
gcaacactag tccgtgcaag tcggttcata acaatatcaa atgataacat ggcactcaaa 11640
gaagcagggg agtcgaagga tactaatctc gtgtatcagc agattatgct aactgggcta 11700
agcttgttcg agttcaatat gagatataag aaaggttcct tagggaagcc actgatattg 11760
cacttacatc ttaataacgg gtgctgtata atggagtccc cacaggaggc gaatatcccc 11820
ccaaggtcca cattagattt agagattaca caagagaaca ataaattgat ctatgatcct 11880
gatccactca aggatgtgga ccttgagcta tttagcaagg tcagagatgt tgtacacaca 11940
gttgacatga cttattggtc agatgatgaa gttatcagag caaccagtat ctgtactgca 12000
atgacgatag ctgatacaat gtctcaatta gatagagaca acttaaaaga gatgatcgca 12060
ctagtaaatg acgatgatgt caacagcttg attactgagt ttatggtgat tgatgttcct 12120
ttattttgct caacgttcgg gggtattcta gtcaatcagt ttgcatactc actctacggc 12180
ttaaacatca gaggaaggga agaaatatgg ggacatgtag tccggattct taaagatacc 12240
tcccacgcag ttttaaaagt cttatctaat gctctatctc atcccaaaat cttcaaacga 12300
ttctggaatg caggtgtcgt ggaacctgtg tatgggccta acctctcaaa tcaggataag 12360
atactcttgg ccctctctgt ctgtgaatat tctgtggatc tattcatgca cgattggcaa 12420
gggggtgtac cgcttgagat ctttatctgt gacaatgacc cagatgtggc cgacatgagg 12480
aggtcctctt tcttggcaag acatcttgca tacctatgca gcttggcaga gatatctagg 12540

gatgggccaa gattagaatc aatgaactct ctagagaggc tcgagtcact aaagagttac 12600
ctggaactca catttcttga tgacccggta ctgaggtaca gtcagttgac tggcctagtc 12660
atcaaagtat tcccatctac tttgacctat atccggaagt catctataaa agtgttaagg 12720
acaagaggta taggagtccc tgaagtctta gaagattggg atcccgaggc agataatgca 12780
ctgttagatg gtatcgcggc agaaatacaa cagaatattc ctttgggaca tcagactaga 12840
gccccttttt gggggttgag agtatccaag tcacaggtac tgcgtctccg ggggtacaag 12900
gagatcacaa gaggtgagat aggcagatca ggtgttggtc tgacgttacc attcgatgga 12960
agatatctat ctcaccagct gaggctcttt ggcatcaaca gtactagctg cttgaaagca 13020
cttgaactta cctacctatt gagcccctta gttgacgaag ataaagatag gctatattta 13080
ggggaaggag ctggggccat gctttcctgt tatgacgcta ctcttggccc atgcatcaac 13140
tattataact caggggtata ctcttgtgat gtcaatgggc agagagagtt aaatatatat 13200
cctgctgagg tggcactagt gggaaagaaa ttaaacaatg ttactagtct gggtcaaaga 13260
gttaaagtgt tattcaacgg gaatcctggc tcgacatgga ttgggaatga tgagtgtgag 13320
gctttgattt ggaatgaatt acagaatagc tcgataggcc tagtccactg tgacatggag 13380
ggaggagatc ataaggatga tcaagttgta ctgcatgagc attacagtgt aatccggatc 13440
gcgtatctgg tgggggatcg agacgttgtg cttataagca agattgctcc caggctgggc 13500
acggattgga ccaggcagct cagcctatat ctgagatact gggacgaggt taacctaata 13560
gtgcttaaaa catctaaccc tgcttccaca gagatgtatc tcctatcgag gcaccccaaa 13620
tctgacatta tagaggacag caagacagtg ttagctagtc tcctcccttt gtcaaaagaa 13680
gatagcatca agatagaaaa gtggatctta atagagaagg caaaggctca cgaatgggtt 13740
actcgggaat tgagagaagg aagctcttca tcagggatgc ttagacctta ccatcaagca 13800
ctgcagacgt ttggctttga accaaacttg tataaattga gcagagattt cttgtccacc 13860
atgaacatag ctgatacaca caactgcatg atagctttca acagggtttt gaaggataca 13920
atcttcgaat gggctagaat aactgagtca gataaaaggc ttaaactaac tggtaagtat 13980
gacctgtatc ctgtgagaga ttcaggcaag ttgaagacaa tttctagaag acttgtgcta 14040
tcttggatat ctttatctat gtccacaaga ttggtaactg ggtcattccc tgaccagaag 14100
tttgaagcaa gacttcaatt gggaatagtt tcattatcat cccgtgaaat caggaacctg 14160
agggttatca caaaaacttt attagacagg tttgaggata ttatacatag tataacgtat 14220
agattcctca ccaaagaaat aaagattttg atgaagattt taggggcagt caagatgttc 14280
ggggccaggc aaaatgaata cacgaccgtg attgatgatg gatcactagg tgatatcgag 14340
ccatatgaca gctcgtaata attagtccct atcgtgcaga acgatcgaag ctccgcggta 14400
cctggaagtc ttggacttgt ccatatgaca atagtaagaa aaacttacaa gaagacaaga 14460
aaatttaaaa ggatacatat ctcttaaact cttgtctggt gggtcggcat ggcatctcca 14520
cctcctcgcg gtccgacctg ggcatccgaa ggaggacgca cgtccactcg gatggctaag 14580
ggagagctac gcgtggatcc ccgggaattc cgtaatcatg gtcatagctg tttcctgtgt 14640

gaaattgtta tccgctcaca attccacaca acatacgagc cggaagcata aagtgtaaag 14700
cctggggtgc ctaatgagtg agctaactca cattaattgc gttgcgctca ctgcccgctt 14760
tccagtcggg aaacctgtcg tgccagctgc attaatgaat cggccaacgc gcggggagag 14820
gcggtttgcg tattgggcgc tcttccgctt cctcgctcac tgactcgctg cgctcggtcg 14880
ttcggctgcg gcgagcggta tcagctcact caaaggcggt aatacggtta tccacagaat 14940
caggggataa cgcaggaaag aacatgtgag caaaaggcca gcaaaaggcc aggaaccgta 15000
aaaaggccgc gttgctggcg tttttccata ggctccgccc ccctgacgag catcacaaaa 15060
atcgacgctc aagtcagagg tggcgaaacc cgacaggact ataaagatac caggcgtttc 15120
cccctggaag ctccctcgtg cgctctcctg ttccgaccct gccgcttacc ggatacctgt 15180
ccgcctttct cccttcggga agcgtggcgc tttctcatag ctcacgctgt aggtatctca 15240
gttcggtgta ggtcgttcgc tccaagctgg gctgtgtgca cgaacccccc gttcagcccg 15300
accgctgcgc cttatccggt aactatcgtc ttgagtccaa cccggtaaga cacgacttat 15360
cgccactggc agcagccact ggtaacagga ttagcagagc gaggtatgta ggcggtgcta 15420
cagagttctt gaagtggtgg cctaactacg gctacactag aagaacagta tttggtatct 15480
gcgctctgct gaagccagtt accttcggaa aaagagttgg tagctcttga tccggcaaac 15540
aaaccaccgc tggtagcggt ggtttttttg tttgcaagca gcagattacg cgcagaaaaa 15600
aaggatctca agaagatcct ttgatctttt ctacggggtc tgacgctcag tggaacgaaa 15660
actcacgtta agggattttg gtcatgagat tatcaaaaag gatcttcacc tagatccttt 15720
taaattaaaa atgaagtttt aaatcaatct aaagtatata tgagtaaact tggtctgaca 15780
gttaccaatg cttaatcagt gaggcaccta tctcagcgat ctgtctattt cgttcatcca 15840
tagttgcctg actccccgtc gtgtagataa ctacgatacg ggagggctta ccatctggcc 15900
ccagtgctgc aatgataccg cgagacccac gctcaccggc tccagattta tcagcaataa 15960
accagccagc cggaagggcc gagcgcagaa gtggtcctgc aactttatcc gcctccatcc 16020
agtctattaa ttgttgccgg gaagctagag taagtagttc gccagttaat agtttgcgca 16080
acgttgttgc cattgctaca ggcatcgtgg tgtcacgctc gtcgtttggt atggcttcat 16140
tcagctccgg ttcccaacga tcaaggcgag ttacatgatc ccccatgttg tgcaaaaaag 16200
cggttagctc cttcggtcct ccgatcgttg tcagaagtaa gttggccgca gtgttatcac 16260
tcatggttat ggcagcactg cataattctc ttactgtcat gccatccgta agatgctttt 16320
ctgtgactgg tgagtactca accaagtcat tctgagaata gtgtatgcgg cgaccgagtt 16380
gctcttgccc ggcgtcaata cgggataata ccgcgccaca tagcagaact ttaaaagtgc 16440
tcatcattgg aaaacgttct tcggggcgaa aactctcaag gatcttaccg ctgttgagat 16500
ccagttcgat gtaacccact cgtgcaccca actgatcttc agcatctttt actttcacca 16560
gcgtttctgg gtgagcaaaa acaggaaggc aaaatgccgc aaaaaaggga ataagggcga 16620
cacggaaatg ttgaatactc atactcttcc tttttcaata ttattgaagc atttatcagg 16680

gttattgtct catgagcgga tacatatttg aatgtattta gaaaaataaa caaatagggg 16740

ttccgcgcac atttccccga aaagtgccac ctgacgtcta agaaaccatt attatcatga 16800

cattaaccta taaaaatagg cgtatcacga ggccctttcg tc 16842

<210> 22
<211> 282
<212> DNA
<213> Mus musculus

<400> 22
atgaggtcac ttcagatgct gctcctggct gctctgcttc tggggacttt tctgcagcat 60

gccagagctg ctcgagccac caatgtaggc cgagagtgct gcctggatta cttcaaaggg 120

gccattccta tcaggaagtt ggtgagctgg tataagacct cagtggagtg ttccagggat 180

gccatcgtgt ttctgactgt ccagggcaag ctcatctgtg cagaccccaa agacaaacat 240

gtgaagaagg ccatcagatt ggtgaaaaac ccaaggccat ga 282

<210> 23
<211> 276
<212> DNA
<213> Mus musculus

<400> 23
atgaagatct ctgcagctgc cctcaccatc atcctcactg cagccgccct ctgcaccccc 60

gcacctgcct caccatatgg ctcggacacc actccctgct gctttgccta cctctccctc 120

gcgctgcctc gtgcccacgt caaggagtat ttctacacca gcagcaagtg ctccaatctt 180

gcagtcgtgt ttgtcactcg aaggaaccgc caagtgtgtg ccaacccaga gaagaagtgg 240

gttcaagaat acatcaacta tttggagatg agctag 276